# EUROPEAN PATENT APPLICATION

(11) **EP 3 868 399 A2**
(43) Date of publication of application: **25.08.2021**
(21) Application number: 19866309.8
(22) Date of filing: 12.09.2019
(51) Int. Cl.: A61K 39/25, A61P 31/22, C07K 19/00, C12N 15/70, C12N 15/62

(54) **IMMUNE COMPOSITION, PREPARATION METHOD THEREFOR, AND APPLICATION THEREOF**

(30) Priority: 27.09.2018 CN 201811131501; 28.06.2019 CN 201910571839
(71) Applicant: Bravovax Co., Ltd., Wuhan, Hubei 430075 (CN)
(72) Inventor: MU, Ting, Wuhan, Hubei 430075 (CN); ZHAO, Ping, Wuhan, Hubei 430075 (CN); XU, Long, Wuhan, Hubei 430075 (CN); XIAO, Yang, Wuhan, Hubei 430075 (CN); JULIEN, Jean-philippe, Wuhan, Hubei 430075 (CN); WU, Yue, Wuhan, Hubei 430075 (CN); XIE, Liang, Wuhan, Hubei 430075 (CN); CHEN, Xueting, Wuhan, Hubei 430075 (CN); LIDU, Qi, Wuhan, Hubei 430075 (CN); SIA, Charles Dwo Yuan, Wuhan, Hubei 430075 (CN); CHONG, Pele Choi Sing, Wuhan, Hubei 430075 (CN); KLEIN, Michel, Wuhan, Hubei 430075 (CN); DU, Linsen, Wuhan, Hubei 430075 (CN); WU, Ke, Wuhan, Hubei 430075 (CN)
(74) Representative: Gille Hrabal Partnerschaftsgesellschaft mbB Patentanwälte
(86) International application number: PCT/CN2019/105716
(87) International publication number: WO 2020/063370

(57) **Abstract**

Provided are an immune composition, a preparation method therefor, and a use thereof. In the present invention, a prokaryotic expression system or a recombinant adenovirus system is used to highly efficiently express VZV envelope gE glycoprotein and the flagellin fusion protein thereof. The produced recombinant gE protein, gE flagellin fusion protein and recombinant adenovirus vector, or composition thereof is used to immunize a mouse so as to promote the body to generate gE and VZV-specific antibody titer, as well as gE-specific and VZV-specific cell immunity.

SEQUENCE LISTING

<110>  BRAVOVAX CO., LTD

<120>  IMMUNE COMPOSITION, PREPARATION METHOD THEREFOR AND USE THEREOF

<130>  FPUS20111744

<150>  2019105718393
<151>  2019-06-28

<150>  2018111315018
<151>  2019-09-27

<160>  50

<170>  PatentIn version 3.3

<210>  1
<211>  507
<212>  PRT
<213>  Adenovirus

<220>
<221>  UNSURE
<222>  (1)..(507)

<400>  1

Ser Val Leu Arg Tyr Asp Asp Phe His Ile Asp Glu Asp Lys Leu Asp
1               5                   10                  15

Thr Asn Ser Val Tyr Glu Pro Tyr Tyr His Ser Asp His Ala Glu Ser
            20                  25                  30

Ser Trp Val Asn Arg Gly Glu Ser Ser Arg Lys Ala Tyr Asp His Asn
        35                  40                  45

Ser Pro Tyr Ile Trp Pro Arg Asn Asp Tyr Asp Gly Phe Leu Glu Asn
    50                  55                  60

Ala His Glu His His Gly Val Tyr Asn Gln Gly Arg Gly Ile Asp Ser
65                  70                  75                  80

Gly Glu Arg Leu Met Gln Pro Thr Gln Met Ser Ala Gln Glu Asp Leu
                85                  90                  95

Gly Asp Asp Thr Gly Ile His Val Ile Pro Thr Leu Asn Gly Asp Asp
            100                 105                 110

Arg His Lys Ile Val Asn Val Asp Gln Arg Gln Tyr Gly Asp Val Phe
        115                 120                 125

Lys Gly Asp Leu Asn Pro Lys Pro Gln Gly Gln Arg Leu Ile Glu Val
    130                 135                 140

Ser Val Glu Glu Asn His Pro Phe Thr Leu Arg Ala Pro Ile Gln Arg
145                 150                 155                 160

Ile Tyr Gly Val Arg Tyr Thr Glu Thr Trp Ser Phe Leu Pro Ser Leu
                165                 170                 175

Thr Cys Thr Gly Asp Ala Ala Pro Ala Ile Gln His Ile Cys Leu Lys
            180                 185                 190

His Thr Thr Cys Phe Gln Asp Val Val Val Asp Val Asp Cys Ala Glu
        195                 200                 205

Asn Thr Lys Glu Asp Gln Leu Ala Glu Ile Ser Tyr Arg Phe Gln Gly
    210                 215                 220

Lys Lys Glu Ala Asp Gln Pro Trp Ile Val Val Asn Thr Ser Thr Leu
225                 230                 235                 240

Phe Asp Glu Leu Glu Leu Asp Pro Pro Glu Ile Glu Pro Gly Val Leu
                245                 250                 255

Lys Val Leu Arg Thr Glu Lys Gln Tyr Leu Gly Val Tyr Ile Trp Asn
            260                 265                 270

Met Arg Gly Ser Asp Gly Thr Ser Thr Tyr Ala Thr Phe Leu Val Thr
        275                 280                 285

Trp Lys Gly Asp Glu Lys Thr Arg Asn Pro Thr Pro Ala Val Thr Pro
    290                 295                 300

Gln Pro Arg Gly Ala Glu Phe His Met Trp Asn Tyr His Ser His Val
305                 310                 315                 320

Phe Ser Val Gly Asp Thr Phe Ser Leu Ala Met His Leu Gln Tyr Lys
                325                 330                 335

Ile His Glu Ala Pro Phe Asp Leu Leu Leu Glu Trp Leu Tyr Val Pro
            340                 345                 350

Ile Asp Pro Thr Cys Gln Pro Met Arg Leu Tyr Ser Thr Cys Leu Tyr
        355                 360                 365

His Pro Asn Ala Pro Gln Cys Leu Ser His Met Asn Ser Gly Cys Thr
    370                 375                 380

Phe Thr Ser Pro His Leu Ala Gln Arg Val Ala Ser Thr Val Tyr Gln

385                 390                 395                 400

Asn Cys Glu His Ala Asp Asn Tyr Thr Ala Tyr Cys Leu Gly Ile Ser
                405                 410                 415

His Met Glu Pro Ser Phe Gly Leu Ile Leu His Asp Gly Gly Thr Thr
            420                 425                 430

Leu Lys Phe Val Asp Thr Pro Glu Ser Leu Ser Gly Leu Tyr Val Phe
        435                 440                 445

Val Val Tyr Phe Asn Gly His Val Glu Ala Val Ala Tyr Thr Val Val
    450                 455                 460

Ser Thr Val Asp His Phe Val Asn Ala Ile Glu Glu Arg Gly Phe Pro
465                 470                 475                 480

Pro Thr Ala Gly Gln Pro Pro Ala Thr Thr Lys Pro Lys Glu Ile Thr
                485                 490                 495

Pro Val Asn Pro Gly Thr Ser Pro Leu Ile Arg
            500                 505

<210>  2
<211>  1518
<212>  DNA
<213>  Adenovirus

<220>
<221>  unsure
<222>  (1)..(1518)

<400>  2
agcgtgtgag atatgatgac ttccacattg atgaggataa actagacact aatagcgtgt      60

atgagcctta ctaccatagt gaccatgctg aatctagctg ggtgaatagg ggtgaatcta     120

gccgtaaggc atagatcata actcccctta tatctggcct aggaatgact atgacggctt     180

tctggagaat gcccacgagc atcacggcgt gtacaatcag gggcggggca tcgacagcgg     240

cgagaggctg atgcagccaa ccagatgagc gcccaggagg acctgggcga cgacaccggc     300

atccacgtga tccccaccct gaacggcgac gacaggcaca agatcgtgaa cgtggaccag     360

aggcagtacg gcgatgtgtt caagggcacc tgaaccccaa accccagggc cagaggctga     420

tcgaggtgtc cgtggaggag aaccaccctt tcaccctgag agcccccatc cagcggatct     480

acggcgtgag atacaccgag acctggagct tcttcccagc ctgacctgca cgggcgatgc     540

cgcccccgcc atccagcaca tctgcctgaa gcacaccacc tgtttccagg atgtggtggt     600

tgatgtggac tgtgccgaaa atactaagga ggaccagctg gcgagatctc ctaccgcttc     660

cagggcaaaa aggaggccga ccagccctgg atcgtggtga acacctccac cctgttcgat     720

gagttagagc tggacccccc tgagatcgag cctggcgtgc tgaaagtgtg cgcaccgaga 780

agcagtacct gggcgtgtac atctggaaca tgaggggcag cgacggcacc agcacctacg 840

ccaccttcct ggtgacctgg aagggcgacg agaagaccag aaatcctacc cccgcgtgac 900

cccccagcct aggggcgccg agtttcacat gtggaactac cacagccacg tgttcagcgt 960

gggcgacacc ttcagcctgg ccatgcacct gcagtacaag atccacgagg cccccttcga 1020

ccgctgctgg agtggctgta cgtgcccatc gaccccacct gccagcccat gagactgtac 1080

agcacttgcc tgtaccaccc caacgccccc cagtgcctga gccacatgaa tagcggctgc 1140

accttcaccg cccccacctg gctcagaggg tggccagcac cgtgtaccag aactgcgagc 1200

acgccgacaa ttacaccgcc tactgcctgg gcatcagcca catggagccc agcttcggcc 1260

tgatcctgca cgacggggca ccaccctgaa gtttgtggac acccccgagt ccctgtctgg 1320

actgtatgtc tttgtggtgt attttaatgg acacgttgaa gccgtggcct atacagtggt 1380

gtctacagtg gaccactttg tgaatgccat tgaagaaaga ggctttcctc ctaccgccgg 1440

ccagccccca gccacaccaa gcctaaagaa atcacaccag tgaatccagg aactagcccc 1500

ctgatcagat aataataa 1518

<210> 3
<211> 495
<212> PRT
<213> Salmonella typhimurium

<220>
<221> UNSURE
<222> (1)..(495)

<400> 3

<210> 4
<211> 16
<212> PRT
<213> Artificial Sequence

<220>
<221> UNSURE
<222> (1)..(16)
<223> unknown

<400> 4

Ser Pro Gly Ile Ser Gly Gly Gly Gly Gly Ile Leu Asp Ser Met Gly
1               5                   10                  15

<210> 5
<211> 129
<212> PRT
<213> Salmonella typhimurium

<220>
<221> UNSURE
<222> (1)..(129)

<400>  5

Met Ala Gln Val Ile Asn Thr Asn Ser Leu Ser Leu Leu Thr Gln Asn
1               5                   10                  15

Asn Leu Asn Lys Ser Gln Ser Ala Leu Gly Thr Ala Ile Glu Arg Leu
            20                  25                  30

Ser Ser Gly Leu Arg Ile Asn Ser Ala Lys Asp Asp Ala Ala Gly Gln
        35                  40                  45

Ala Ile Ala Asn Arg Phe Thr Ala Asn Ile Lys Gly Leu Thr Gln Ala
    50                  55                  60

Ser Arg Asn Ala Asn Asp Gly Ile Ser Ile Ala Gln Thr Thr Glu Gly
65                  70                  75                  80

Ala Leu Asn Glu Ile Asn Asn Asn Leu Gln Arg Val Arg Glu Leu Ala
                85                  90                  95

Val Gln Ser Ala Asn Ser Thr Asn Ser Gln Ser Asp Leu Asp Ser Ile
            100                 105                 110

Gln Ala Glu Ile Thr Gln Arg Leu Asn Glu Ile Asp Arg Val Ser Gly
        115                 120                 125

Gln

<210>  6
<211>  104
<212>  PRT
<213>  Salmonella typhimurium

<220>
<221>  UNSURE
<222>  (1)..(104)

<400>  6

Lys Ala Gln Pro Asp Leu Ala Glu Ala Ala Ala Thr Thr Thr Glu Asn
1               5                   10                  15

Pro Leu Gln Lys Ile Asp Ala Ala Leu Ala Gln Val Asp Thr Leu Arg
            20                  25                  30

Ser Asp Leu Gly Ala Val Gln Asn Arg Phe Asn Ser Ala Ile Thr Asn
        35                  40                  45

Leu Gly Asn Thr Val Asn Asn Leu Thr Ser Ala Arg Ser Arg Ile Glu
    50                  55                  60

Asp Ser Asp Tyr Ala Thr Glu Val Ser Asn Met Ser Arg Ala Gln Ile
65                  70                  75                  80

Leu Gln Gln Ala Gly Thr Ser Val Leu Ala Gln Ala Asn Gln Val Pro
                85                  90                  95

Gln Asn Val Leu Ser Leu Leu Arg
            100

<210>  7
<211>  15
<212>  PRT
<213>  Artificial Sequence

<220>
<221>  UNSURE
<222>  (1)..(15)
<223>  unknown

<400>  7

Gly Gly Gly Gly Ser Gly Gly Gly Gly Ser Gly Gly Gly Gly Ser
1               5                   10                  15

<210>  8
<211>  817
<212>  PRT
<213>  Artificial Sequence

<220>
<221>  UNSURE
<222>  (1)..(817)
<223>  unknown

<400>  8
   Ala Gln Val Ile Asn Thr Asn Ser Leu Ser Leu Leu Thr Gln Asn Asn
   1               5                   10                  15
   Leu Asn Lys Ser Gln Ser Ala Leu Gly Thr Ala Ile Glu Arg Leu Ser
               20                  25                  30
   Ser Gly Leu Arg Ile Asn Ser Ala Lys Asp Asp Ala Ala Gly Gln Ala
           35                  40                  45
   Ile Ala Asn Arg Phe Thr Ala Asn Ile Lys Gly Leu Thr Gln Ala Ser
       50                  55                  60
   Arg Asn Ala Asn Asp Gly Ile Ser Ile Ala Gln Thr Thr Glu Gly Ala
   65                  70                  75                  80
   Leu Asn Glu Ile Asn Asn Asn Leu Gln Arg Val Arg Glu Leu Ala Val
   85                  90                  95
   Gln Ser Ala Asn Ser Thr Asn Ser Gln Ser Asp Leu Asp Ser Ile Gln
               100                 105                 110
   Ala Glu Ile Thr Gln Arg Leu Asn Glu Ile Asp Arg Val Ser Gly Gln
           115                 120                 125
   Thr Gln Phe Asn Gly Val Lys Val Leu Ala Gln Asp Asn Thr Leu Thr
       130                 135                 140
   Ile Gln Val Gly Ala Asn Asp Gly Glu Thr Ile Asp Ile Asp Leu Lys
   145                 150                 155                 160
   Gln Ile Asn Ser Gln Thr Leu Gly Leu Asp Thr Leu Asn Val Gln Ser
                   165                 170                 175
   Pro Gly Ile Ser Gly Gly Gly Gly Gly Ile Leu Asp Ser Met Gly Lys
               180                 185                 190
   Ala Gln Pro Asp Leu Ala Glu Ala Ala Ala Thr Thr Thr Glu Asn Pro
           195                 200                 205
   Leu Gln Lys Ile Asp Ala Ala Leu Ala Gln Val Asp Thr Leu Arg Ser
       210                 215                 220
   Asp Leu Gly Ala Val Gln Asn Arg Phe Asn Ser Ala Ile Thr Asn Leu
   225                 230                 235                 240
   Gly Asn Thr Val Asn Asn Leu Thr Ser Ala Arg Ser Arg Ile Glu Asp
                   245                 250                 255
   Ser Asp Tyr Ala Thr Glu Val Ser Asn Met Ser Arg Ala Gln Ile Leu
               260                 265                 270
   Gln Gln Ala Gly Thr Ser Val Leu Ala Gln Ala Asn Gln Val Pro Gln
           275                 280                 285
   Asn Val Leu Ser Leu Leu Arg Gly Gly Gly Gly Ser Gly Gly Gly Gly
       290                 295                 300
   Ser Gly Gly Gly Gly Ser Ser Val Leu Arg Tyr Asp Asp Phe His Ile
   305                 310                 315                 320
   Asp Glu Asp Lys Leu Asp Thr Asn Ser Val Tyr Glu Pro Tyr Tyr His
                   325                 330                 335
   Ser Asp His Ala Glu Ser Ser Trp Val Asn Arg Gly Glu Ser Ser Arg
               340                 345                 350
   Lys Ala Tyr Asp His Asn Ser Pro Tyr Ile Trp Pro Arg Asn Asp Tyr
           355                 360                 365
   Asp Gly Phe Leu Glu Asn Ala His Glu His His Gly Val Tyr Asn Gln
       370                 375                 380
   Gly Arg Gly Ile Asp Ser Gly Glu Arg Leu Met Gln Pro Thr Gln Met
   385                 390                 395                 400
   Ser Ala Gln Glu Asp Leu Gly Asp Asp Thr Gly Ile His Val Ile Pro
                   405                 410                 415
   Thr Leu Asn Gly Asp Asp Arg His Lys Ile Val Asn Val Asp Gln Arg
               420                 425                 430
   Gln Tyr Gly Asp Val Phe Lys Gly Asp Leu Asn Pro Lys Pro Gln Gly
           435                 440                 445
   Gln Arg Leu Ile Glu Val Ser Val Glu Glu Asn His Pro Phe Thr Leu
       450                 455                 460
   Arg Ala Pro Ile Gln Arg Ile Tyr Gly Val Arg Tyr Thr Glu Thr Trp
   465                 470                 475                 480
   Ser Phe Leu Pro Ser Leu Thr Cys Thr Gly Asp Ala Ala Pro Ala Ile
                   485                 490                 495
   Gln His Ile Cys Leu Lys His Thr Thr Cys Phe Gln Asp Val Val Val
               500                 505                 510
   Asp Val Asp Cys Ala Glu Asn Thr Lys Glu Asp Gln Leu Ala Glu Ile
           515                 520                 525
   Ser Tyr Arg Phe Gln Gly Lys Lys Glu Ala Asp Gln Pro Trp Ile Val
       530                 535                 540
   Val Asn Thr Ser Thr Leu Phe Asp Glu Leu Glu Leu Asp Pro Pro Glu
   545                 550                 555                 560
   Ile Glu Pro Gly Val Leu Lys Val Leu Arg Thr Glu Lys Gln Tyr Leu
                   565                 570                 575
   Gly Val Tyr Ile Trp Asn Met Arg Gly Ser Asp Gly Thr Ser Thr Tyr
               580                 585                 590
   Ala Thr Phe Leu Val Thr Trp Lys Gly Asp Glu Lys Thr Arg Asn Pro
   595 600 605
   Thr Pro Ala Val Thr Pro Gln Pro Arg Gly Ala Glu Phe His Met Trp
   610 615 620
   Asn Tyr His Ser His Val Phe Ser Val Gly Asp Thr Phe Ser Leu Ala
   625 630 635 640
   Met His Leu Gln Tyr Lys Ile His Glu Ala Pro Phe Asp Leu Leu Leu
   645 650 655
   Glu Trp Leu Tyr Val Pro Ile Asp Pro Thr Cys Gln Pro Met Arg Leu
   660 665 670
   Tyr Ser Thr Cys Leu Tyr His Pro Asn Ala Pro Gln Cys Leu Ser His
   675 680 685
   Met Asn Ser Gly Cys Thr Phe Thr Ser Pro His Leu Ala Gln Arg Val
   690 695 700
   Ala Ser Thr Val Tyr Gln Asn Cys Glu His Ala Asp Asn Tyr Thr Ala
   705 710 715 720
   Tyr Cys Leu Gly Ile Ser His Met Glu Pro Ser Phe Gly Leu Ile Leu
   725 730 735
   His Asp Gly Gly Thr Thr Leu Lys Phe Val Asp Thr Pro Glu Ser Leu
   740 745 750
   Ser Gly Leu Tyr Val Phe Val Val Tyr Phe Asn Gly His Val Glu Ala
   755 760 765
   Val Ala Tyr Thr Val Val Ser Thr Val Asp His Phe Val Asn Ala Ile
   770 775 780
   Glu Glu Arg Gly Phe Pro Pro Thr Ala Gly Gln Pro Pro Ala Thr Thr
   785 790 795 800
   Lys Pro Lys Glu Ile Thr Pro Val Asn Pro Gly Thr Ser Pro Leu Ile
   805 810 815
   Arg

<210>  9
<211>  817
<212>  PRT

<213>  Artificial Sequence

<220>
<221>  UNSURE
<222>  (1)..(817)
<223>  unknown

<400>  9

Ser Val Leu Arg Tyr Asp Asp Phe His Ile Asp Glu Asp Lys Leu Asp
1               5                   10                  15

Thr Asn Ser Val Tyr Glu Pro Tyr Tyr His Ser Asp His Ala Glu Ser
            20                  25                  30

Ser Trp Val Asn Arg Gly Glu Ser Ser Arg Lys Ala Tyr Asp His Asn
        35                  40                  45

Ser Pro Tyr Ile Trp Pro Arg Asn Asp Tyr Asp Gly Phe Leu Glu Asn
    50                  55                  60

Ala His Glu His His Gly Val Tyr Asn Gln Gly Arg Gly Ile Asp Ser
65                  70                  75                  80

Gly Glu Arg Leu Met Gln Pro Thr Gln Met Ser Ala Gln Glu Asp Leu
                85                  90                  95

Gly Asp Asp Thr Gly Ile His Val Ile Pro Thr Leu Asn Gly Asp Asp
            100                 105                 110

Arg His Lys Ile Val Asn Val Asp Gln Arg Gln Tyr Gly Asp Val Phe
        115                 120                 125

Lys Gly Asp Leu Asn Pro Lys Pro Gln Gly Gln Arg Leu Ile Glu Val
    130                 135                 140

Ser Val Glu Glu Asn His Pro Phe Thr Leu Arg Ala Pro Ile Gln Arg
145                 150                 155                 160

Ile Tyr Gly Val Arg Tyr Thr Glu Thr Trp Ser Phe Leu Pro Ser Leu
                165                 170                 175

Thr Cys Thr Gly Asp Ala Ala Pro Ala Ile Gln His Ile Cys Leu Lys
            180                 185                 190

His Thr Thr Cys Phe Gln Asp Val Val Val Asp Val Asp Cys Ala Glu
        195                 200                 205

Asn Thr Lys Glu Asp Gln Leu Ala Glu Ile Ser Tyr Arg Phe Gln Gly
    210                 215                 220

Lys Lys Glu Ala Asp Gln Pro Trp Ile Val Val Asn Thr Ser Thr Leu
225                 230                 235                 240

Phe Asp Glu Leu Glu Leu Asp Pro Pro Glu Ile Glu Pro Gly Val Leu
                245                 250                 255

Lys Val Leu Arg Thr Glu Lys Gln Tyr Leu Gly Val Tyr Ile Trp Asn
            260                 265                 270

Met Arg Gly Ser Asp Gly Thr Ser Thr Tyr Ala Thr Phe Leu Val Thr
        275                 280                 285

Trp Lys Gly Asp Glu Lys Thr Arg Asn Pro Thr Pro Ala Val Thr Pro
    290                 295                 300

Gln Pro Arg Gly Ala Glu Phe His Met Trp Asn Tyr His Ser His Val
305                 310                 315                 320

Phe Ser Val Gly Asp Thr Phe Ser Leu Ala Met His Leu Gln Tyr Lys
                325                 330                 335

Ile His Glu Ala Pro Phe Asp Leu Leu Leu Glu Trp Leu Tyr Val Pro
            340                 345                 350

Ile Asp Pro Thr Cys Gln Pro Met Arg Leu Tyr Ser Thr Cys Leu Tyr
        355                 360                 365

His Pro Asn Ala Pro Gln Cys Leu Ser His Met Asn Ser Gly Cys Thr
    370                 375                 380

Phe Thr Ser Pro His Leu Ala Gln Arg Val Ala Ser Thr Val Tyr Gln
385                 390                 395                 400

Asn Cys Glu His Ala Asp Asn Tyr Thr Ala Tyr Cys Leu Gly Ile Ser
                405                 410                 415

His Met Glu Pro Ser Phe Gly Leu Ile Leu His Asp Gly Gly Thr Thr
            420                 425                 430

Leu Lys Phe Val Asp Thr Pro Glu Ser Leu Ser Gly Leu Tyr Val Phe
        435                 440                 445

Val Val Tyr Phe Asn Gly His Val Glu Ala Val Ala Tyr Thr Val Val
    450                 455                 460

Ser Thr Val Asp His Phe Val Asn Ala Ile Glu Glu Arg Gly Phe Pro

465                 470                 475                 480

Pro Thr Ala Gly Gln Pro Pro Ala Thr Thr Lys Pro Lys Glu Ile Thr
                485                 490                 495

Pro Val Asn Pro Gly Thr Ser Pro Leu Ile Arg Gly Gly Gly Gly Ser
            500                 505                 510

Gly Gly Gly Gly Ser Gly Gly Gly Gly Ser Ala Gln Val Ile Asn Thr
        515                 520                 525

Asn Ser Leu Ser Leu Leu Thr Gln Asn Asn Leu Asn Lys Ser Gln Ser
    530                 535                 540

Ala Leu Gly Thr Ala Ile Glu Arg Leu Ser Ser Gly Leu Arg Ile Asn
545                 550                 555                 560

Ser Ala Lys Asp Asp Ala Ala Gly Gln Ala Ile Ala Asn Arg Phe Thr
                565                 570                 575

Ala Asn Ile Lys Gly Leu Thr Gln Ala Ser Arg Asn Ala Asn Asp Gly
            580                 585                 590

Ile Ser Ile Ala Gln Thr Thr Glu Gly Ala Leu Asn Glu Ile Asn Asn
        595                 600                 605

Asn Leu Gln Arg Val Arg Glu Leu Ala Val Gln Ser Ala Asn Ser Thr
    610                 615                 620

Asn Ser Gln Ser Asp Leu Asp Ser Ile Gln Ala Glu Ile Thr Gln Arg
625                 630                 635                 640

Leu Asn Glu Ile Asp Arg Val Ser Gly Gln Thr Gln Phe Asn Gly Val
                645                 650                 655

Lys Val Leu Ala Gln Asp Asn Thr Leu Thr Ile Gln Val Gly Ala Asn
            660                 665                 670

Asp Gly Glu Thr Ile Asp Ile Asp Leu Lys Gln Ile Asn Ser Gln Thr
        675                 680                 685

Leu Gly Leu Asp Thr Leu Asn Val Gln Ser Pro Gly Ile Ser Gly Gly
    690                 695                 700

Gly Gly Gly Ile Leu Asp Ser Met Gly Lys Ala Gln Pro Asp Leu Ala
705                 710                 715                 720

Glu Ala Ala Ala Thr Thr Thr Glu Asn Pro Leu Gln Lys Ile Asp Ala
                725                 730                 735

Ala Leu Ala Gln Val Asp Thr Leu Arg Ser Asp Leu Gly Ala Val Gln
            740                 745                 750

Asn Arg Phe Asn Ser Ala Ile Thr Asn Leu Gly Asn Thr Val Asn Asn
        755                 760                 765

Leu Thr Ser Ala Arg Ser Arg Ile Glu Asp Ser Asp Tyr Ala Thr Glu
    770                 775                 780

Val Ser Asn Met Ser Arg Ala Gln Ile Leu Gln Gln Ala Gly Thr Ser
785                 790                 795                 800

Val Leu Ala Gln Ala Asn Gln Val Pro Gln Asn Val Leu Ser Leu Leu
                805                 810                 815

Arg

<210>  10
<211>  816
<212>  PRT
<213>  Artificial Sequence

<220>
<221>  UNSURE
<222>  (1)..(816)
<223>  unknown

<400>  10

Ala Gln Val Ile Asn Thr Asn Ser Leu Ser Leu Leu Thr Gln Asn Asn
1               5                   10                  15

Leu Asn Lys Ser Gln Ser Ala Leu Gly Thr Ala Ile Glu Arg Leu Ser
            20                  25                  30

Ser Gly Leu Arg Ile Asn Ser Ala Lys Asp Asp Ala Ala Gly Gln Ala
        35                  40                  45

Ile Ala Asn Arg Phe Thr Ala Asn Ile Lys Gly Leu Thr Gln Ala Ser
    50                  55                  60

Arg Asn Ala Asn Asp Gly Ile Ser Ile Ala Gln Thr Thr Glu Gly Ala
65                  70                  75                  80

Leu Asn Glu Ile Asn Asn Asn Leu Gln Arg Val Arg Glu Leu Ala Val
                85                  90                  95

Gln Ser Ala Asn Ser Thr Asn Ser Gln Ser Asp Leu Asp Ser Ile Gln
        100                 105                 110

Ala Glu Ile Thr Gln Arg Leu Asn Glu Ile Asp Arg Val Ser Gly Gln
    115                 120                 125

Thr Gln Phe Asn Gly Val Lys Val Leu Ala Gln Asp Asn Thr Leu Thr
    130                 135                 140

Ile Gln Val Gly Ala Asn Asp Gly Glu Thr Ile Asp Ile Asp Leu Lys
145                 150                 155                 160

Gln Ile Asn Ser Gln Thr Leu Gly Leu Asp Thr Leu Asn Val Gln Gly
                165                 170                 175

Gly Gly Gly Ser Gly Gly Gly Gly Ser Gly Gly Gly Gly Ser Ser Val
        180                 185                 190

Leu Arg Tyr Asp Asp Phe His Ile Asp Glu Asp Lys Leu Asp Thr Asn
    195                 200                 205

Ser Val Tyr Glu Pro Tyr Tyr His Ser Asp His Ala Glu Ser Ser Trp
    210                 215                 220

Val Asn Arg Gly Glu Ser Ser Arg Lys Ala Tyr Asp His Asn Ser Pro
225                 230                 235                 240

Tyr Ile Trp Pro Arg Asn Asp Tyr Asp Gly Phe Leu Glu Asn Ala His
                245                 250                 255

Glu His His Gly Val Tyr Asn Gln Gly Arg Gly Ile Asp Ser Gly Glu
        260                 265                 270

Arg Leu Met Gln Pro Thr Gln Met Ser Ala Gln Glu Asp Leu Gly Asp
    275                 280                 285

Asp Thr Gly Ile His Val Ile Pro Thr Leu Asn Gly Asp Asp Arg His
    290                 295                 300

Lys Ile Val Asn Val Asp Gln Arg Gln Tyr Gly Asp Val Phe Lys Gly
305                 310                 315                 320

Asp Leu Asn Pro Lys Pro Gln Gly Gln Arg Leu Ile Glu Val Ser Val
                325                 330                 335

Glu Glu Asn His Pro Phe Thr Leu Arg Ala Pro Ile Gln Arg Ile Tyr
        340                 345                 350

Gly Val Arg Tyr Thr Glu Thr Trp Ser Phe Leu Pro Ser Leu Thr Cys
        355                 360                 365

Thr Gly Asp Ala Ala Pro Ala Ile Gln His Ile Cys Leu Lys His Thr
    370                 375                 380

Thr Cys Phe Gln Asp Val Val Val Asp Val Asp Cys Ala Glu Asn Thr
385                 390                 395                 400

Lys Glu Asp Gln Leu Ala Glu Ile Ser Tyr Arg Phe Gln Gly Lys Lys
                405                 410                 415

Glu Ala Asp Gln Pro Trp Ile Val Val Asn Thr Ser Thr Leu Phe Asp
            420                 425                 430

Glu Leu Glu Leu Asp Pro Pro Glu Ile Glu Pro Gly Val Leu Lys Val
        435                 440                 445

Leu Arg Thr Glu Lys Gln Tyr Leu Gly Val Tyr Ile Trp Asn Met Arg
    450                 455                 460

Gly Ser Asp Gly Thr Ser Thr Tyr Ala Thr Phe Leu Val Thr Trp Lys
465                 470                 475                 480

Gly Asp Glu Lys Thr Arg Asn Pro Thr Pro Ala Val Thr Pro Gln Pro
                485                 490                 495

Arg Gly Ala Glu Phe His Met Trp Asn Tyr His Ser His Val Phe Ser
            500                 505                 510

Val Gly Asp Thr Phe Ser Leu Ala Met His Leu Gln Tyr Lys Ile His
        515                 520                 525

Glu Ala Pro Phe Asp Leu Leu Leu Glu Trp Leu Tyr Val Pro Ile Asp
    530                 535                 540

Pro Thr Cys Gln Pro Met Arg Leu Tyr Ser Thr Cys Leu Tyr His Pro
545                 550                 555                 560

Asn Ala Pro Gln Cys Leu Ser His Met Asn Ser Gly Cys Thr Phe Thr
                565                 570                 575

Ser Pro His Leu Ala Gln Arg Val Ala Ser Thr Val Tyr Gln Asn Cys
            580                 585                 590

Glu His Ala Asp Asn Tyr Thr Ala Tyr Cys Leu Gly Ile Ser His Met
        595                 600                 605

Glu Pro Ser Phe Gly Leu Ile Leu His Asp Gly Gly Thr Thr Leu Lys
    610                 615                 620

Phe Val Asp Thr Pro Glu Ser Leu Ser Gly Leu Tyr Val Phe Val Val
625                 630                 635                 640

Tyr Phe Asn Gly His Val Glu Ala Val Ala Tyr Thr Val Val Ser Thr
                645                 650                 655

Val Asp His Phe Val Asn Ala Ile Glu Glu Arg Gly Phe Pro Pro Thr
            660                 665                 670

Ala Gly Gln Pro Pro Ala Thr Thr Lys Pro Lys Glu Ile Thr Pro Val
        675                 680                 685

Asn Pro Gly Thr Ser Pro Leu Ile Arg Gly Gly Gly Gly Ser Gly Gly
    690                 695                 700

Gly Gly Ser Gly Gly Gly Gly Ser Lys Ala Gln Pro Asp Leu Ala Glu
705                 710                 715                 720

Ala Ala Ala Thr Thr Thr Glu Asn Pro Leu Gln Lys Ile Asp Ala Ala
                725                 730                 735

Leu Ala Gln Val Asp Thr Leu Arg Ser Asp Leu Gly Ala Val Gln Asn
            740                 745                 750

Arg Phe Asn Ser Ala Ile Thr Asn Leu Gly Asn Thr Val Asn Asn Leu
        755                 760                 765

Thr Ser Ala Arg Ser Arg Ile Glu Asp Ser Asp Tyr Ala Thr Glu Val
    770                 775                 780

Ser Asn Met Ser Arg Ala Gln Ile Leu Gln Gln Ala Gly Thr Ser Val
785                 790                 795                 800

Leu Ala Gln Ala Asn Gln Val Pro Gln Asn Val Leu Ser Leu Leu Arg
                805                 810                 815

<210>  11
<211>  878
<212>  DNA
<213>  Artificial sequence

<220>
<221>  unsure
<222>  (1)..(878)
<223>  unknown

<400>  11
gcccaggtca ttaacaccaa ctccctgagc ctgctgaccc agaacaatct gaataaagcc      60

agagcgccct ggggaccgcc atcgagcggc tgagctccgg cctgcgcatc aattccgcca     120

aggatgacgc cgccggccag gctatcgcca accggttcac cgctaacatc aagggcctga     180

cccggctagc aggaatgcca atgacggcat tagcattgcc cagacaaccg agggcgccct     240

gaacgagatc aataataacc tgcagagagt gagggaactg gccgtgcagt ccgccaactc     300

tacaaactca agagcgatct ggattccatc caggccgaaa tcacccagcg cctgaatgag     360

atcgacagag tgagcggcca gacccagttt aacggcgtga aggtgctggc ccaggataat     420

accctgacca tccaggtggc gccaatgacg gcgagactat cgacattgat ctgaagcaga     480

ttaactccca gaccctgggc ctggacactc tgaacgtgca gagcccaggg atcagcgggg     540

ggggaggagg catcctggac agcagggcaa ggcccagcct gacctcgccg aggccgccgc     600

cacaaccacc gagaaccctc tgcagaagat cgacgccgcc ctggcccagg tggataccct     660

gcgcagtgat ctgggagccg tgcagaacag gttaacagcg ccatcaccaa cctgggcaat     720

accgtgaata atctgactag cgcccgcagc agaattgagg attctgatta cgccacagag     780

gtgtccaaca tgagccgcgc ccagatcctg cagcaggcgg gacctccgtg ctggcccagg     840

ccaatcaggt cccacagaac gtgctgagcc tgctgagg                             878


<210>  12
<211>  887
<212>  DNA
<213>  Artificial sequence

<220>
<221>  unsure
<222>  (1)..(887)
<223>  unknown

<400>  12
gccaagtgat caacaccaat agcctgagcc tcctgaccca gaacaacctg aacaagtccc      60

agagcgccct gggcaccgcc atcgagcggc tgagctccgg gctgcggatc aattccgcta     120

aggacgatgc gccggacagg ccatcgccaa ccggtttaca gccaatatca agggcctgac     180

ccaggcctcc cggaacgcca atgatggcat cagcatcgcc cagaccaccg agggcgccct     240

gaatgagatt aacaacatct gcagagagtg agggagctgg ccgtgcagtc cgccaacagc     300

accaactccc agtccgacct cgatagcatt caggccgaga tcacccagcg gctgaacgag     360

atcgatcgcg tgagcggcca gaccagttca acggcgtgaa ggtgctggcc caggacaaca     420

ccctgaccat ccaggtgggc gctaatgacg gcgagaccat tgatatcgac ctgaagcaga     480

ttaatagcca gacactgggc ctggacaccc gaacgtgcag agccctggga tcagcggcgg     540

gggaggcggc atcctggata gcatgggcaa agcccagcct gatctggccg aggctgccgc     600

caccacaacc gagaacccac tgcagaagat cgacgccccc tggcccaggt ggacaccctg 660

aggagcgacc tgggcgccgt gcagaatcgg ttcaattccg ccatcactaa cctggggaac 720

accgtgaaca acctgacctc tgcccgcagc cggattgaag acaggattac gccaccgagg 780

tgagcaacat gagcagggcc cagatcctgc agcaggccgg cacatccgtg ctggcccagg 840

ctaaccaggt gcctcagaac gtgctgagcc tgctgaggta ataataa 887

<210> 13
<211> 2457
<212> DNA
<213> Artificial sequence

<220>
<221> unsure
<222> (1)..(2457)
<223> unknown

<400> 13
gcccaggtca ttaacaccaa ctccctgagc ctgctgaccc agaacaatct gaataagagc 60

cagagcgccc tggggaccgc catcgagcgg ctgagctccg gcctgcgcat caattccgcc 120

aaggatgacg ccgccggcca ggctatcgcc aaccggttca ccgctaacat caagggcctg 180

acccaggcta gcaggaatgc caatgacggc attagcattg cccagacaac cgagggcgcc 240

ctgaacgaga tcaataataa cctgcagaga gtgagggaac tggccgtgca gtccgccaac 300

tctacaaact cacagagcga tctggattcc atccaggccg aaatcaccca gcgcctgaat 360

gagatcgaca gagtgagcgg ccagacccag tttaacggcg tgaaggtgct ggcccaggat 420

aataccctga ccatccaggt gggcgccaat gacggcgaga ctatcgacat tgatctgaag 480

cagattaact cccagaccct gggcctggac actctgaacg tgcagggcgg cggcgggtcc 540

ggaggcggcg gcagcggcgg cggcggctcc agcgtgctcc ggtatgacga cttccacatc 600

gatgaagata agctggacac caacagcgtg tacgagccct actaccactc cgaccacgct 660

gagagcagct gggtgaatag aggggagagc agcagaaagg cctatgatca taatagcccc 720

tacatctggc ctagaaatga ctacgatggc tttctcgaaa acgcccacga gcaccacggc 780

gtgtacaacc agggcagggg cattgacagc ggagaacggc tgatgcagcc aacccagatg 840

tccgcccagg aagacctggg agatgataca ggaatccacg tgatccccac cctgaacggc 900

gatgaccggc acaagattgt caatgtggat cagaggcagt acggagacgt gtttaagggg 960

gacctgaacc ctaaacccca ggggcagagg ctgatcgagg tgtctgtgga agaaaatcat 1020

cctttcacac tgagggcccc tatccagcga atctacggcg tgaggtacac cgagacctgg 1080

agcttcctgc ctagcctgac ttgtaccggg gacgccgccc ctgccattca gcacatttgt 1140

ctgaagcaca caacctgttt ccaggacgtg gtggtggatg tcgactgcgc cgagaataca 1200

aaggaagatc agctggccga aatcagctat agatttcagg gcaaaaaaga ggctgaccag 1260

ccctggatcg tggtgaacac cagcaccctg tttgacgagc tggagctgga ccctcccgag 1320

attgagccag gggtgctgaa ggtgctgagg acagagaagc agtacctggg cgtgtacatt 1380

tggaacatgc gggggtccga cggtactagc acctacgcca ccttcctggt gacatggaag 1440

ggcgatgaga agacccgcaa ccctaccccc gccgtgaccc cccagcccag aggggccgag 1500

tttcacatgt ggaattatca tagccacgtg ttctccgtgg gcgacacctt tagcctggcc 1560

atgcacctgc agtataagat ccacgaagcc ccattcgacc tgctgctgga gtggctgtat 1620

gtgcccatcg accccacctg ccagcccatg aggctgtaca gcacatgcct gtatcacccc 1680

aacgcccccc agtgcctgag ccacatgaac tctgggtgca ccttcacctc tccgcacctg 1740

gcccagcgcg tggcctccac agtgtaccag aattgtgagc acgccgataa ctacaccgcc 1800

tactgcctgg gcatcagcca catggaacct tccttcggcc tgatcctgca cgacggcggc 1860

acaaccctga aattcgtgga tacacctgaa tctctgtctg gactgtatgt ttttgtggtg 1920

tattttaatg gacacgtgga agctgtggcc tatacagtgg tgagcacagt ggatcacttt 1980

gtgaatgcca ttgaggagag aggcttccca ccaaccgccg gccagcctcc tgccaccact 2040

aaacctaaag aaatcacccc tgtgaatcct ggcactagcc ctctgattag aggcggcggg 2100

ggaagcggcg gcggcggcag cggcggcggc ggctccaagg cccagcctga cctcgccgag 2160

gccgccgcca caaccaccga gaaccctctg cagaagatcg acgccgccct ggcccaggtg 2220

gataccctgc gcagtgatct gggagccgtg cagaacaggt ttaacagcgc catcaccaac 2280

ctgggcaata ccgtgaataa tctgactagc gcccgcagca gaattgagga ttctgattac 2340

gccacagagg tgtccaacat gagccgcgcc cagatcctgc agcaggccgg gacctccgtg 2400

ctggcccagg ccaatcaggt cccacagaac gtgctgagcc tgctgaggta ataataa 2457

<210> 14
<211> 72
<212> DNA
<213> Artificial sequence

<220>
<221> unsure
<222> (1)..(72)
<223> unknown

<400> 14
atgggaaaac ggtccgccgg cagcatcatg tggctggcca gcctggccgt cgtgatcgcc 60

tgtgccggcg cc 72

<210> 15
<211> 63
<212> DNA
<213> Artificial sequence

<220>
<221>  unsure
<222>  (1)..(63)
<223>  unknown

<400>  15
atggagacag acacactcct gctatgggta ctgctgctct gggttccagg ttccactggt      60

gac                                                                    63


<210>  16
<211>  9
<212>  DNA
<213>  Artificial sequence

<220>
<221>  unsure
<222>  (1)..(9)
<223>  unknown

<400>  16
gccgccgcc                                                               9


<210>  17
<211>  442
<212>  DNA
<213>  Artificial Sequence

<220>
<221>  unsure
<222>  (1)..(442)
<223>  unknown

<400>  17
gaattcaagc ttgggatctt tgtgaaggaa ccttacttct gtggtgtgac ataattggac      60

aaacacctac agagatttaa agctctaagg taaatataaa atttttaagt gtataatgtg     120

ttaaactact gattctaatt gtttgtgtat tttagattca cagtcccaag gctcatttca     180

ggcccctcag tctcacagtc tgttcatgat cataatcagc cataccacat ttgtagaggt     240

tttacttgct ttaaaaaacc tcccacacct ccccctgaac ctgaaacata aaatgaatgc     300

aattgttgtt gttaactttt tattgcagct tataatggtt acaaataaag caatagcatc     360

acaaatttca caaataaagc atttttttca ctgcattcta gttgtggttt gtccaaactc     420

atcaatgtat cttatcatgt ct                                              442


<210>  18
<211>  1599
<212>  DNA
<213>  Artificial Sequence

<220>
<221>  unsure
<222>  (1)..(1599)
<223>  unknown

<400>  18
gccgccgcca tgggaaaacg gagcgccggc agcatcatgt ggctggcctc cctggccgtc      60

gtgatcgcct gcgccggcgc cagcgtgtga gatatgatga cttccacatt gatgaggata     120

aactagacac taatagcgtg tatgagcctt actaccatag tgaccatgct gaatctagct     180

gggtgaatag gggtgaatct agccgtaagg catagatcat aactcccctt atatctggcc     240

taggaatgac tatgacggct ttctggagaa tgcccacgag catcacggcg tgtacaatca     300

ggggcggggc atcgacagcg gcgagaggct gatgcagcca accagatgag cgcccaggag     360

gacctgggcg acgacaccgg catccacgtg atccccaccc tgaacggcga cgacaggcac     420

aagatcgtga acgtggacca gaggcagtac ggcgatgtgt tcaagggcac ctgaacccca     480

aaccccaggg ccagaggctg atcgaggtgt ccgtggagga gaaccaccct ttcaccctga     540

gagcccccat ccagcggatc tacggcgtga gatacaccga gacctggagc ttcttcccag     600

cctgacctgc acgggcgatg ccgcccccgc catccagcac atctgcctga agcacaccac     660

ctgtttccag gatgtggtgg ttgatgtgga ctgtgccgaa aatactaagg aggaccagct     720

ggcgagatct cctaccgctt ccagggcaaa aaggaggccg accagccctg gatcgtggtg     780

aacacctcca ccctgttcga tgagttagag ctggaccccc ctgagatcga gcctggcgtg     840

ctgaaagtgt gcgcaccgag aagcagtacc tgggcgtgta catctggaac atgaggggca     900

gcgacggcac cagcacctac gccaccttcc tggtgacctg gaagggcgac gagaagacca     960

gaaatcctac ccccgcgtga ccccccagcc taggggcgcc gagtttcaca tgtggaacta    1020

ccacagccac gtgttcagcg tgggcgacac cttcagcctg gccatgcacc tgcagtacaa    1080

gatccacgag gcccccttcg accgctgctg gagtggctgt acgtgcccat cgaccccacc    1140

tgccagccca tgagactgta cagcacttgc ctgtaccacc ccaacgcccc ccagtgcctg    1200

agccacatga atagcggctg caccttcacc gcccccacct ggctcagagg gtggccagca    1260

ccgtgtacca gaactgcgag cacgccgaca attacaccgc ctactgcctg ggcatcagcc    1320

acatggagcc cagcttcggc ctgatcctgc acgacggggc accaccctga agtttgtgga    1380

cacccccgag tccctgtctg gactgtatgt ctttgtggtg tattttaatg gacacgttga    1440

agccgtggcc tatacagtgg tgtctacagt ggaccacttt gtgaatgcca ttgaagaaag    1500

aggctttcct cctaccgccg gccagccccc agccacacca agcctaaaga aatcacacca    1560

gtgaatccag gaactagccc cctgatcaga taataataa                           1599

<210>  19
<211>  2041
<212>  DNA
<213>  Artificial Sequence

<220>

<221>   unsure
<222>   (1)..(2041)
<223>   unknown

<400>   19
gccgccgcca tgggaaaacg gagcgccggc agcatcatgt ggctggcctc cctggccgtc      60

gtgatcgcct gcgccggcgc cagcgtgtga gatatgatga cttccacatt gatgaggata     120

aactagacac taatagcgtg tatgagcctt actaccatag tgaccatgct gaatctagct     180

gggtgaatag gggtgaatct agccgtaagg catagatcat aactcccctt atatctggcc     240

taggaatgac tatgacggct ttctggagaa tgcccacgag catcacggcg tgtacaatca     300

ggggcggggc atcgacagcg gcgagaggct gatgcagcca accagatgag cgcccaggag     360

gacctgggcg acgacaccgg catccacgtg atccccaccc tgaacggcga cgacaggcac     420

aagatcgtga acgtggacca gaggcagtac ggcgatgtgt tcaagggcac ctgaacccca     480

aaccccaggg ccagaggctg atcgaggtgt ccgtggagga gaaccacccc ttcaccctga     540

gagcccccat ccagcggatc tacggcgtga gatacaccga gacctggagc ttcttcccag     600

cctgacctgc acgggcgatg ccgcccccgc catccagcac atctgcctga agcacaccac     660

ctgtttccag gatgtggtgg ttgatgtgga ctgtgccgaa aatactaagg aggaccagct     720

ggcgagatct cctaccgctt ccagggcaaa aaggaggccg accagccctg gatcgtggtg     780

aacacctcca ccctgttcga tgagttagag ctggaccccc ctgagatcga gcctggcgtg     840

ctgaaagtgt gcgcaccgag aagcagtacc tgggcgtgta catctggaac atgaggggca     900

gcgacggcac cagcacctac gccaccttcc tggtgacctg gaagggcgac gagaagacca     960

gaaatcctac ccccgcgtga ccccccagcc taggggcgcc gagtttcaca tgtggaacta    1020

ccacagccac gtgttcagcg tgggcgacac cttcagcctg gccatgcacc tgcagtacaa    1080

gatccacgag gcccccttcg accgctgctg gagtggctgt acgtgcccat cgaccccacc    1140

tgccagccca tgagactgta cagcacttgc ctgtaccacc ccaacgcccc ccagtgcctg    1200

agccacatga atagcggctg caccttcacc gcccccacct ggctcagagg gtggccagca    1260

ccgtgtacca gaactgcgag cacgccgaca attacaccgc ctactgcctg ggcatcagcc    1320

acatggagcc cagcttcggc ctgatcctgc acgacggggc accaccctga agtttgtgga    1380

cacccccgag tccctgtctg gactgtatgt ctttgtggtg tattttaatg gacacgttga    1440

agccgtggcc tatacagtgg tgtctacagt ggaccacttt gtgaatgcca ttgaagaaag    1500

aggctttcct cctaccgccg gccagccccc agccacacca agcctaaaga aatcacacca    1560

gtgaatccag gaactagccc cctgatcaga taataataag aattcaagct tgggatcttt    1620

gtgaaggaac cttacttctg tggtgtgaca taattggaca aacacctaca gagatttaaa    1680

gctctaaggt aaatataaaa tttttaagtg tataatgtgt taaactactg attctaattg    1740

tttgtgtatt ttagattcac agtcccaagg ctcatttcag gcccctcagt ctcacagtct 1800

gttcatgatc ataatcagcc ataccacatt tgtagaggtt ttacttgctt taaaaaacct 1860

cccacacctc cccctgaacc tgaaacataa aatgaatgca attgttgttg ttaacttttt 1920

attgcagctt ataatggtta caaataaagc aatagcatca caaatttcac aaataaagca 1980

tttttttcac tgcattctag ttgtggtttg tccaaactca tcaatgtatc ttatcatgtc 2040

t 2041

<210> 20
<211> 2522
<212> DNA
<213> Artificial Sequence

<220>
<221> unsure
<222> (1)..(2522)
<223> unknown

<400> 20
gccgccgcca tgggaaaacg gtccgccggc agcatcatgt ggctggccag cctggccgtc 60

gtgatcgcct gtgccggcgc cgcccaggtc attaacacca actccctgag cctgctgacc 120

cagaacaatc tgaataaagc cagagcgccc tggggaccgc catcgagcgg ctgagctccg 180

gcctgcgcat caattccgcc aaggatgacg ccgccggcca ggctatcgcc aaccggttca 240

ccgctaacat caagggcctg acccggctag caggaatgcc aatgacggca ttagcattgc 300

ccagacaacc gagggcgccc tgaacgagat caataataac ctgcagagag tgagggaact 360

ggccgtgcag tccgccaact ctacaaactc aagagcgatc tggattccat ccaggccgaa 420

atcacccagc gcctgaatga gatcgacaga gtgagcggcc agacccagtt taacggcgtg 480

aaggtgctgg cccaggataa taccctgacc atccaggtgg cgccaatgac ggcgagacta 540

tcgacattga tctgaagcag attaactccc agaccctggg cctggacact ctgaacgtgc 600

agagcccagg gatcagcggg gggggaggag gcatcctgga cagcagggca aggcccagcc 660

tgacctcgcc gaggccgccg ccacaaccac cgagaaccct ctgcagaaga tcgacgccgc 720

cctggcccag gtggataccc tgcgcagtga tctgggagcc gtgcagaaca ggttaacagc 780

gccatcacca acctgggcaa taccgtgaat aatctgacta gcgcccgcag cagaattgag 840

gattctgatt acgccacaga ggtgtccaac atgagccgcg cccagatcct gcagcaggcg 900

ggacctccgt gctggcccag gccaatcagg tcccacagaa cgtgctgagc ctgctgaggg 960

gcggcggcgg gtccggaggc ggcggcagcg gcggcggcgg ctccagcgtg ctccggtatg 1020

acgactccac atcgatgaag ataagctgga caccaacagc gtgtacgagc cctactacca 1080

ctccgaccac gctgagagca gctgggtgaa tagaggggag agcagcagaa aggcctatga 1140

tcataatagc cccacatctg gcctagaaat gactacgatg gctttctcga aaacgcccac 1200

gagcaccacg gcgtgtacaa ccagggcagg ggcattgaca gcggagaacg gctgatgcag 1260

ccaacccaga tgtccgccca gaagacctgg gagatgatac aggaatccac gtgatcccca 1320

ccctgaacgg cgatgaccgg cacaagattg tcaatgtgga tcagaggcag tacggagacg 1380

tgtttaaggg ggacctgaac cctaaaccca ggggcagagg ctgatcgagg tgtctgtgga 1440

agaaaatcat cctttcacac tgagggcccc tatccagcga atctacggcg tgaggtacac 1500

cgagacctgg agcttcctgc ctagcctgac ttgtaccggg gacgccgccc ctgccattca 1560

gcacatttgt ctgaagcaca caacctgttt ccaggacgtg gggtggatgt cgactgcgcc 1620

gagaatacaa aggaagatca gctggccgaa atcagctata gatttcaggg caaaaaagag 1680

gctgaccagc cctggatcgt ggtgaacacc agcaccctgt ttgacgagtg gagctggacc 1740

ctcccgagat tgagccaggg gtgctgaagg tgctgaggac agagaagcag tacctgggcg 1800

tgtacatttg gaacatgcgg gggtccgacg gtactagcac ctacgccacc ttcctgtgac 1860

atggaagggc gatgagaaga cccgcaaccc tacccccgcc gtgacccccc agcccagagg 1920

ggccgagttt cacatgtgga attatcatag ccacgtgttc tccgtgggcg acacctttag 1980

ccggccatgc acctgcagta taagatccac gaagccccat tcgacctgct gctggagtgg 2040

ctgtatgtgc ccatcgaccc cacctgccag cccatgaggc tgtacagcac atgcctgtat 2100

caccccaacc cccccagtgc ctgagccaca tgaactctgg gtgcaccttc acctctccgc 2160

acctggccca gcgcgtggcc tccacagtgt accagaattg tgagcacgcc gataactaca 2220

ccgcctactg cctgggatca gccacatgga accttccttc ggcctgatcc tgcacgacgg 2280

cggcacaacc ctgaaattcg tggatacacc tgaatctctg tctggactgt atgtttttgt 2340

ggtgtatttt aatggacacg tggagctgtg gcctatacag tggtgagcac agtggatcac 2400

tttgtgaatg ccattgagga gagaggcttc ccaccaaccg ccggccagcc tcctgccacc 2460

actaaaccta aagaaatcac ccctgtgaat ctggcactag ccctctgatt agataataat 2520

aa 2522

<210> 21
<211> 2967
<212> DNA
<213> Artificial Sequence

<220>
<221> unsure
<222> (1)..(2967)
<223> unknown

<400> 21
gccgccgcca tgggaaaacg gtccgccggc agcatcatgt ggctggccag cctggccgtc 60

gtgatcgcct gtgccggcgc cgcccaggtc attaacacca actccctgag cctgctgacc 120

cagaacaatc tgaataaagc cagagcgccc tggggaccgc catcgagcgg ctgagctccg 180

gcctgcgcat caattccgcc aaggatgacg ccgccggcca ggctatcgcc aaccggttca 240

ccgctaacat caagggcctg acccggctag caggaatgcc aatgacggca ttagcattgc 300

ccagacaacc gagggcgccc tgaacgagat caataataac ctgcagagag tgagggaact 360

ggccgtgcag tccgccaact ctacaaactc aagagcgatc tggattccat ccaggccgaa 420

atcacccagc gcctgaatga gatcgacaga gtgagcggcc agacccagtt taacggcgtg 480

aaggtgctgg cccaggataa taccctgacc atccaggtgg cgccaatgac ggcgagacta 540

tcgacattga tctgaagcag attaactccc agaccctggg cctggacact ctgaacgtgc 600

agagcccagg gatcagcggg gggggaggag gcatcctgga cagcagggca aggcccagcc 660

tgacctcgcc gaggccgccg ccacaaccac cgagaaccct ctgcagaaga tcgacgccgc 720

cctggcccag gtggataccc tgcgcagtga tctgggagcc gtgcagaaca ggttaacagc 780

gccatcacca acctgggcaa taccgtgaat aatctgacta gcgcccgcag cagaattgag 840

gattctgatt acgccacaga ggtgtccaac atgagccgcg cccagatcct gcagcaggcg 900

ggacctccgt gctggcccag gccaatcagg tcccacagaa cgtgctgagc ctgctgaggg 960

gcggcggcgg gtccggaggc ggcggcagcg gcggcggcgg ctccagcgtg ctccggtatg 1020

acgactccac atcgatgaag ataagctgga caccaacagc gtgtacgagc cctactacca 1080

ctccgaccac gctgagagca gctgggtgaa tagaggggag agcagcagaa aggcctatga 1140

tcataatagc cccacatctg gcctagaaat gactacgatg gctttctcga aaacgcccac 1200

gagcaccacg gcgtgtacaa ccagggcagg ggcattgaca gcggagaacg gctgatgcag 1260

ccaacccaga tgtccgccca gaagacctgg gagatgatac aggaatccac gtgatcccca 1320

ccctgaacgg cgatgaccgg cacaagattg tcaatgtgga tcagaggcag tacggagacg 1380

tgtttaaggg ggacctgaac cctaaaccca ggggcagagg ctgatcgagg tgtctgtgga 1440

agaaaatcat cctttcacac tgagggcccc tatccagcga atctacggcg tgaggtacac 1500

cgagacctgg agcttcctgc ctagcctgac ttgtaccggg gacgccgccc ctgccattca 1560

gcacatttgt ctgaagcaca caacctgttt ccaggacgtg gggtggatgt cgactgcgcc 1620

gagaatacaa aggaagatca gctggccgaa atcagctata gatttcaggg caaaaaagag 1680

gctgaccagc cctggatcgt ggtgaacacc agcaccctgt ttgacgagtg gagctggacc 1740

ctcccgagat tgagccaggg gtgctgaagg tgctgaggac agagaagcag tacctgggcg 1800

tgtacatttg gaacatgcgg gggtccgacg gtactagcac ctacgccacc ttcctgtgac 1860

atggaagggc gatgagaaga cccgcaaccc tacccccgcc gtgacccccc agcccagagg 1920

ggccgagttt cacatgtgga attatcatag ccacgtgttc tccgtgggcg acacctttag 1980

ccggccatgc acctgcagta taagatccac gaagccccat tcgacctgct gctggagtgg 2040

ctgtatgtgc ccatcgaccc cacctgccag cccatgaggc tgtacagcac atgcctgtat 2100

caccccaacc cccccagtgc ctgagccaca tgaactctgg gtgcaccttc acctctccgc 2160

acctggccca gcgcgtggcc tccacagtgt accagaattg tgagcacgcc gataactaca 2220

ccgcctactg cctgggatca gccacatgga accttccttc ggcctgatcc tgcacgacgg 2280

cggcacaacc ctgaaattcg tggatacacc tgaatctctg tctggactgt atgtttttgt 2340

ggtgtatttt aatggacacg tggagctgtg gcctatacag tggtgagcac agtggatcac 2400

tttgtgaatg ccattgagga gagaggcttc ccaccaaccg ccggccagcc tcctgccacc 2460

actaaaccta aagaaatcac ccctgtgaat ctggcactag ccctctgatt agataataat 2520

aagaattcaa gcttgggatc tttgtgaagg aaccttactt ctgtggtgtg acataattgg 2580

acaaactacc tacagagatt taaagctcta aggtaaatat aaaattttta agtgtataat 2640

gtgttaaact actgattcta attgtttgtg tattttagat tcacagtccc aaggctcatt 2700

tcaggcccct cagtcctcac agtctgttca tgatcataat cagccatacc acatttgtag 2760

aggttttact tgctttaaaa aacctcccac acctccccct gaacctgaaa cataaaatga 2820

atgcaattgt tgttgttaac ttgtttattg cagcttataa tggttacaaa taaagcaata 2880

gcatcacaaa tttcacaaat aaagcatttt tttcactgca ttctagttgt ggtttgtcca 2940

aactcatcaa tgtatcttat catgtct 2967

<210> 22
<211> 2564
<212> DNA
<213> Artificial Sequence

<220>
<221> unsure
<222> (1)..(2564)
<223> unknown

<400> 22
gccgccgcca tgggaaaacg gagcgccggc agcatcatgt ggctggctag cctggccgtc 60

gtgattgcct gtgccggagc cagcgtgctg aggtatgatg attttcatat tgatgaggac 120

aaactggaca caaatagcgt gtacgagcca tattatcata gtgatcatgc cgaaagtagc 180

tgggtgaata gaggcgagtc ttctaggaaa gcctatgatc ataattctcc ctatatttgg 240

cctaggaatg actatgatgg cttcctggaa aatgcccacg aacaccacgg agtgtataac 300

cagggaagag gcatcgactc tggggagcgg accgagacct ggagcttcct gccctgatgc 360

agccaacaca gatgagcgcc caggaggacc tgggcgacga taccggcatc cacgtgatcc 420

ccaccctgaa tggcgacgac agacacaaga tcgtgaacgt ggaccagaga cagtatggcg 480

acgtgtttaa gggcgacctg aatccaaagc cccagggcca gaggctgatt gaggtgtctg 540

tggaggagaa tcatcccttc accctgcggg cccccatcca gagaatctac ggcgtgaggt 600
ataccgagac ctggagcttc ctgccctctc tgacatgtac tggcgatgcc gcccctgcca 660
tccagcacat ctgcctgaag cacacaacat gtttccagga cgtggtggtg gacgtggact 720
gcgccgagaa cactaaagaa gaccagctgg ccgagattag ctacagattc caggggaaga 780
aggaggccga ccagccctgg atcgtggtga acaccagcac actgtttgac gagcttgagc 840
tggacccccc tgagatcgag ccaggcgtgc tcaaggtgct gcgcaccgag aagcagtatc 900
tgggcgtgta catctggaac atgcgcggct ctgacggcac cagcacctac gccacattcc 960
tggtgacatg gaagggggat gagaagaccc gcaatcctac cccagccgtg accccacagc 1020
ccaggggcgc cgagtttcat atgtggaact accatagcca cgtgtttagc gtgggcgaca 1080
ccttcagcct ggccatgcac ctgcagtaca aaatccacga agcccccttc gacctgctgc 1140
tggagtggct ctacgtgcct attgacccca catgccagcc catgagactg tacagcacct 1200
gcctgtacca ccccaatgct ccacagtgcc tgagccacat gaactccggc tgcactttca 1260
cctctcctca cctggctcag cgcgtggcct ccaccgtgta ccagaattgt gaacacgccg 1320
acaactacac cgcatactgt ctgggcatct cccacatgga gccctcattt ggcctgatcc 1380
tgcacgacgg ggggaccacc ctgaagttcg tggacacccc cgagagcctg tctggcctgt 1440
atgtgtttgt ggtgtacttc aacggccatg tggaggccgt ggcctatacc gtggtgtcca 1500
cagtggacca ctttgtgaac gccatcgagg agagaggctt cccccctacc gccggacagc 1560
cccccgccac cacaaagccc aaggagatca cccccgtgaa tcccggaacc agccccctga 1620
ttagaggcgg cgggggaagc ggcggcggcg gcagcggcgg cggcggctcc gcacaagtga 1680
tcaacaccaa tagcctgagc ctcctgaccc agaacaacct gaacaagtcc cagagcgccc 1740
tgggcaccgc catcgagcgg ctgagctccg ggctgcggat caattccgct aaggacgatg 1800
ccgccggaca ggccatcgcc aaccggttta cagccaatat caagggcctg acccaggcct 1860
cccggaacgc caatgatggc atcagcatcg cccagaccac cgaggcgccc ctgaatgaga 1920
ttaacaacaa tctgcagaga gtgagggagc tggccgtgca gtccgccaac agcaccaact 1980
cccagtccga cctcgatagc attcaggccg agatcaccca gcggctgaac gagatcgatc 2040
gcgtgagcgg ccagacccag ttcaacggcg tgaaggtgct ggcccaggac aacaccctga 2100
ccatccaggt gggcgctaat gacggcgaga ccattgatat cgacctgaag cagattaata 2160
gccagacact gggcctggac accctgaacg tgcagagccc tgggatcagc ggcgggggag 2220
gcggcatcct ggatagcatg ggcaaagccc agcctgatct ggccgaggct gccgccacca 2280
caaccgagaa cccactgcag aagatcgacg ccgccctggc ccaggtggac accctgagga 2340
gcgacctggg cgccgtgcag aatcggttca attccgccat cactaacctg gggaacaccg 2400
tgaacaacct gacctctgcc cgcagccgga ttgaagacag cgattacgcc accgaggtga 2460

gcaacatgag cagggcccag atcctgcagc aggccggcac atccgtgctg gcccaggcta 2520

accaggtgcc tcagaacgtg ctgagcctgc tgaggtaata ataa 2564

<210> 23
<211> 3009
<212> DNA
<213> Artificial Sequence

<220>
<221> unsure
<222> (1)..(3009)
<223> unknown

<400> 23
gccgccgcca tgggaaaacg gagcgccggc agcatcatgt ggctggctag cctggccgtc 60

gtgattgcct gtgccggagc cagcgtgctg aggtatgatg attttcatat tgatgaggac 120

aaactggaca caaatagcgt gtacgagcca tattatcata gtgatcatgc cgaaagtagc 180

tgggtgaata gaggcgagtc ttctaggaaa gcctatgatc ataattctcc ctatatttgg 240

cctaggaatg actatgatgg cttcctggaa aatgcccacg aacaccacgg agtgtataac 300

cagggaagag gcatcgactc tggggagcgg accgagacct ggagcttcct gccctgatgc 360

agccaacaca gatgagcgcc caggaggacc tgggcgacga taccggcatc cacgtgatcc 420

ccaccctgaa tggcgacgac agacacaaga tcgtgaacgt ggaccagaga cagtatggcg 480

acgtgtttaa gggcgacctg aatccaaagc cccagggcca gaggctgatt gaggtgtctg 540

tggaggagaa tcatcccttc accctgcggg cccccatcca gagaatctac ggcgtgaggt 600

ataccgagac ctggagcttc ctgccctctc tgacatgtac tggcgatgcc gcccctgcca 660

tccagcacat ctgcctgaag cacacaacat gtttccagga cgtggtggtg gacgtggact 720

gcgccgagaa cactaaagaa gaccagctgg ccgagattag ctacagattc caggggaaga 780

aggaggccga ccagccctgg atcgtggtga acaccagcac actgtttgac gagcttgagc 840

tggacccccc tgagatcgag ccaggcgtgc tcaaggtgct gcgcaccgag aagcagtatc 900

tgggcgtgta catctggaac atgcgcggct ctgacggcac cagcacctac gccacattcc 960

tggtgacatg gaagggggat gagaagaccc gcaatcctac cccagccgtg accccacagc 1020

ccaggggcgc cgagtttcat atgtggaact accatagcca cgtgtttagc gtgggcgaca 1080

ccttcagcct ggccatgcac ctgcagtaca aaatccacga agcccccttc gacctgctgc 1140

tggagtggct ctacgtgcct attgacccca catgccagcc catgagactg tacagcacct 1200

gcctgtacca ccccaatgct ccacagtgcc tgagccacat gaactccggc tgcactttca 1260

cctctcctca cctggctcag cgcgtggcct ccaccgtgta ccagaattgt gaacacgccg 1320

acaactacac cgcatactgt ctgggcatct cccacatgga gccctcattt ggcctgatcc 1380

tgcacgacgg ggggaccacc ctgaagttcg tggacacccc cgagagcctg tctggcctgt 1440

atgtgtttgt ggtgtacttc aacggccatg tggaggccgt ggcctatacc gtggtgtcca 1500

cagtggacca ctttgtgaac gccatcgagg agagaggctt cccccctacc gccggacagc 1560

cccccgccac cacaaagccc aaggagatca cccccgtgaa tcccggaacc agccccctga 1620

ttagaggcgg cgggggaagc ggcggcggcg gcagcggcgg cggcggctcc gcacaagtga 1680

tcaacaccaa tagcctgagc ctcctgaccc agaacaacct gaacaagtcc cagagcgccc 1740

tgggcaccgc catcgagcgg ctgagctccg ggctgcggat caattccgct aaggacgatg 1800

ccgccggaca ggccatcgcc aaccggttta cagccaatat caagggcctg acccaggcct 1860

cccggaacgc caatgatggc atcagcatcg cccagaccac cgagggcgcc ctgaatgaga 1920

ttaacaacaa tctgcagaga gtgagggagc tggccgtgca gtccgccaac agcaccaact 1980

cccagtccga cctcgatagc attcaggccg agatcaccca gcggctgaac gagatcgatc 2040

gcgtgagcgg ccagacccag ttcaacggcg tgaaggtgct ggcccaggac aacaccctga 2100

ccatccaggt gggcgctaat gacggcgaga ccattgatat cgacctgaag cagattaata 2160

gccagacact gggcctggac accctgaacg tgcagagccc tgggatcagc ggcgggggag 2220

gcggcatcct ggatagcatg ggcaaagccc agcctgatct ggccgaggct gccgccacca 2280

caaccgagaa cccactgcag aagatcgacg ccgccctggc ccaggtggac accctgagga 2340

gcgacctggg cgccgtgcag aatcggttca attccgccat cactaacctg gggaacaccg 2400

tgaacaacct gacctctgcc cgcagccgga ttgaagacag cgattacgcc accgaggtga 2460

gcaacatgag cagggcccag atcctgcagc aggccggcac atccgtgctg gcccaggcta 2520

accaggtgcc tcagaacgtg ctgagcctgc tgaggtaata ataagaattc aagcttggga 2580

tctttgtgaa ggaaccttac ttctgtggtg tgacataatt ggacaaacta cctacagaga 2640

tttaaagctc taaggtaaat ataaaatttt taagtgtata atgtgttaaa ctactgattc 2700

taattgtttg tgtattttag attcacagtc ccaaggctca tttcaggccc ctcagtcctc 2760

acagtctgtt catgatcata atcagccata ccacatttgt agaggtttta cttgctttaa 2820

aaaacctccc acacctcccc ctgaacctga aacataaaat gaatgcaatt gttgttgtta 2880

acttgtttat tgcagcttat aatggttaca aataaagcaa tagcatcaca aatttcacaa 2940

ataaagcatt tttttcactg cattctagtt gtggtttgtc caaactcatc aatgtatctt 3000

atcatgtct 3009

<210> 24
<211> 2538
<212> DNA
<213> Artificial Sequence

<220>

<221>   unsure
<222>   (1)..(2538)
<223>   unknown

<400>   24
gccgccgcca tgggaaaacg gtccgccggc agcatcatgt ggctggccag cctggccgtc      60

gtgatcgcct gtgccggcgc cgcccaggtc attaacacca actccctgag cctgctgacc     120

cagaacaatc tgaataagag ccagagcgcc ctggggaccg ccatcgagcg gctgagctcc     180

ggcctgcgca tcaattccgc caaggatgac gccgccggcc aggctatcgc caaccggttc     240

accgctaaca tcaagggcct gacccaggct agcaggaatg ccaatgacgg cattagcatt     300

gcccagacaa ccgagggcgc cctgaacgag atcaataata acctgcagag agtgagggaa     360

ctggccgtgc agtccgccaa ctctacaaac tcacagagcg atctggattc catccaggcc     420

gaaatcaccc agcgcctgaa tgagatcgac agagtgagcg gccagaccca gtttaacggc     480

gtgaaggtgc tggcccagga taataccctg accatccagg tgggcgccaa tgacggcgag     540

actatcgaca ttgatctgaa gcagattaac tcccagaccc tgggcctgga cactctgaac     600

gtgcagggcg gcggcgggtc cggaggcggc ggcagcggcg gcggcggctc cagcgtgctc     660

cggtatgacg acttccacat cgatgaagat aagctggaca ccaacagcgt gtacgagccc     720

tactaccact ccgaccacgc tgagagcagc tgggtgaata gaggggagag cagcagaaag     780

gcctatgatc ataatagccc ctacatctgg cctagaaatg actacgatgg ctttctcgaa     840

aacgcccacg agcaccacgg cgtgtacaac cagggcaggg gcattgacag cggagaacgg     900

ctgatgcagc caacccagat gtccgcccag gaagacctgg gagatgatac aggaatccac     960

gtgatcccca ccctgaacgg cgatgaccgg cacaagattg tcaatgtgga tcagaggcag    1020

tacggagacg tgtttaaggg ggacctgaac cctaaacccc aggggcagag gctgatcgag    1080

gtgtctgtgg aagaaaatca tcctttcaca ctgagggccc ctatccagcg aatctacggc    1140

gtgaggtaca ccgagacctg gagcttcctg cctagcctga cttgtaccgg ggacgccgcc    1200

cctgccattc agcacatttg tctgaagcac acaacctgtt tccaggacgt ggtggtggat    1260

gtcgactgcg ccgagaatac aaaggaagat cagctggccg aaatcagcta tagatttcag    1320

ggcaaaaaag aggctgacca gccctggatc gtggtgaaca ccagcaccct gtttgacgag    1380

ctggagctgg accctcccga gattgagcca ggggtgctga aggtgctgag gacagagaag    1440

cagtacctgg gcgtgtacat ttggaacatg cgggggtccg acggtactag cacctacgcc    1500

accttcctgg tgacatggaa gggcgatgag aagacccgca accctacccc cgccgtgacc    1560

ccccagccca gaggggccga gtttcacatg tggaattatc atagccacgt gttctccgtg    1620

ggcgacacct ttagcctggc catgcacctg cagtataaga tccacgaagc cccattcgac    1680

ctgctgctgg agtggctgta tgtgcccatc gaccccacct gccagcccat gaggctgtac    1740

agcacatgcc tgtatcaccc caacgccccc cagtgcctga gccacatgaa ctctgggtgc 1800

accttcacct ctccgcacct ggcccagcgc gtggcctcca cagtgtacca gaattgtgag 1860

cacgccgata actacaccgc ctactgcctg ggcatcagcc acatggaacc ttccttcggc 1920

ctgatcctgc acgacggcgg cacaaccctg aaattcgtgg atacacctga atctctgtct 1980

ggactgtatg tttttgtggt gtattttaat ggacacgtgg aagctgtggc ctatacagtg 2040

gtgagcacag tggatcactt tgtgaatgcc attgaggaga gaggcttccc accaaccgcc 2100

ggccagcctc ctgccaccac taaacctaaa gaaatcaccc ctgtgaatcc tggcactagc 2160

cctctgatta gaggcggcgg gggaagcggc ggcggcggca gcggcggcgg cggctccaag 2220

gcccagcctg acctcgccga ggccgccgcc acaaccaccg agaaccctct gcagaagatc 2280

gacgccgccc tggcccaggt ggataccctg cgcagtgatc tgggagccgt gcagaacagg 2340

tttaacagcg ccatcaccaa cctgggcaat accgtgaata atctgactag cgcccgcagc 2400

agaattgagg attctgatta cgccacagag gtgtccaaca tgagccgcgc ccagatcctg 2460

cagcaggccg ggacctccgt gctggcccag gccaatcagg tcccacagaa cgtgctgagc 2520

ctgctgaggt aataataa 2538

<210> 25
<211> 2983
<212> DNA
<213> Artificial Sequence

<220>
<221> unsure
<222> (1)..(2983)
<223> unknown

<400> 25
gccgccgcca tgggaaaacg gtccgccggc agcatcatgt ggctggccag cctggccgtc 60

gtgatcgcct gtgccggcgc cgcccaggtc attaacacca actccctgag cctgctgacc 120

cagaacaatc tgaataagag ccagagcgcc ctggggaccg ccatcgagcg gctgagctcc 180

ggcctgcgca tcaattccgc caaggatgac gccgccggcc aggctatcgc caaccggttc 240

accgctaaca tcaagggcct gacccaggct agcaggaatg ccaatgacgg cattagcatt 300

gcccagacaa ccgagggcgc cctgaacgag atcaataata acctgcagag agtgagggaa 360

ctggccgtgc agtccgccaa ctctacaaac tcacagagcg atctggattc catccaggcc 420

gaaatcaccc agcgcctgaa tgagatcgac agagtgagcg gccagaccca gtttaacggc 480

gtgaaggtgc tggcccagga taataccctg accatccagg tgggcgccaa tgacggcgag 540

actatcgaca ttgatctgaa gcagattaac tcccagaccc tgggcctgga cactctgaac 600

gtgcagggcg gcggcgggtc cggaggcggc ggcagcggcg gcggcggctc cagcgtgctc 660

cggtatgacg acttccacat cgatgaagat aagctggaca ccaacagcgt gtacgagccc 720

tactaccact ccgaccacgc tgagagcagc tgggtgaata gaggggagag cagcagaaag 780

gcctatgatc ataatagccc ctacatctgg cctagaaatg actacgatgg ctttctcgaa 840

aacgcccacg agcaccacgg cgtgtacaac cagggcaggg gcattgacag cggagaacgg 900

ctgatgcagc caacccagat gtccgcccag gaagacctgg gagatgatac aggaatccac 960

gtgatcccca ccctgaacgg cgatgaccgg cacaagattg tcaatgtgga tcagaggcag 1020

tacggagacg tgtttaaggg ggacctgaac cctaaacccc aggggcagag gctgatcgag 1080

gtgtctgtgg aagaaaatca tcctttcaca ctgagggccc ctatccagcg aatctacggc 1140

gtgaggtaca ccgagacctg gagcttcctg cctagcctga cttgtaccgg ggacgccgcc 1200

cctgccattc agcacatttg tctgaagcac acaacctgtt tccaggacgt ggtggtggat 1260

gtcgactgcg ccgagaatac aaaggaagat cagctggccg aaatcagcta tagatttcag 1320

ggcaaaaaag aggctgacca gccctggatc gtggtgaaca ccagcaccct gtttgacgag 1380

ctggagctgg accctcccga gattgagcca ggggtgctga aggtgctgag gacagagaag 1440

cagtacctgg gcgtgtacat ttggaacatg cgggggtccg acggtactag cacctacgcc 1500

accttcctgg tgacatggaa gggcgatgag aagacccgca accctacccc cgccgtgacc 1560

ccccagccca gaggggccga gtttcacatg tggaattatc atagccacgt gttctccgtg 1620

ggcgacacct ttagcctggc catgcacctg cagtataaga tccacgaagc cccattcgac 1680

ctgctgctgg agtggctgta tgtgcccatc gaccccacct gccagcccat gaggctgtac 1740

agcacatgcc tgtatcaccc caacgccccc cagtgcctga gccacatgaa ctctgggtgc 1800

accttcacct ctccgcacct ggcccagcgc gtggcctcca cagtgtacca gaattgtgag 1860

cacgccgata actacaccgc ctactgcctg ggcatcagcc acatggaacc ttccttcggc 1920

ctgatcctgc acgacggcgg cacaaccctg aaattcgtgg atacacctga atctctgtct 1980

ggactgtatg tttttgtggt gtattttaat ggacacgtgg aagctgtggc ctatacagtg 2040

gtgagcacag tggatcactt tgtgaatgcc attgaggaga gaggcttccc accaaccgcc 2100

ggccagcctc ctgccaccac taaacctaaa gaaatcaccc ctgtgaatcc tggcactagc 2160

cctctgatta gaggcggcgg gggaagcggc ggcggcggca gcggcggcgg cggctccaag 2220

gcccagcctg acctcgccga ggccgccgcc acaaccaccg agaaccctct gcagaagatc 2280

gacgccgccc tggcccaggt ggataccctg cgcagtgatc tgggagccgt gcagaacagg 2340

tttaacagcg ccatcaccaa cctgggcaat accgtgaata atctgactag cgcccgcagc 2400

agaattgagg attctgatta cgccacagag gtgtccaaca tgagccgcgc ccagatcctg 2460

cagcaggccg ggacctccgt gctggcccag gccaatcagg tcccacagaa cgtgctgagc 2520

ctgctgaggt aataataaga attcaagctt gggatctttg tgaaggaacc ttacttctgt 2580

ggtgtgacat aattggacaa actacctaca gagatttaaa gctctaaggt aaatataaaa 2640

tttttaagtg tataatgtgt taaactactg attctaattg tttgtgtatt ttagattcac 2700

agtcccaagg ctcatttcag gcccctcagt cctcacagtc tgttcatgat cataatcagc 2760

cataccacat ttgtagaggt tttacttgct ttaaaaaacc tcccacacct ccccctgaac 2820

ctgaaacata aaatgaatgc aattgttgtt gttaacttgt ttattgcagc ttataatggt 2880

tacaaataaa gcaatagcat cacaaatttc acaaataaag catttttttc actgcattct 2940

agttgtggtt tgtccaaact catcaatgta tcttatcatg tct 2983

<210> 26
<211> 3102
<212> DNA
<213> Artificial Sequence

<220>
<221> unsure
<222> (1)..(3102)
<223> unknown

<400> 26
ccaccatgga gacagacaca ctcctgctat gggtactgct gctctgggtt ccaggttcca 60

ctggtgacac tagtatggca caagtcatta atacaaacag cctgtcgctg ttgacccaga 120

ataacctgaa caaatcccag tccgcactgg gcactgctat cgagcgtttg tcttccggtc 180

tgcgtatcaa cagcgcgaaa gacgatgcgg caggacaggc gattgctaac cgttttaccg 240

cgaacatcaa aggtctgact caggcttccc gtaacgctaa cgacggtatc tccattgcgc 300

agaccactga aggcgcgctg aacgaaatca acaacaacct gcagcgtgtg cgtgaactgg 360

cggttcagtc tgcgaatggt actaactccc agtctgacct cgactccatc caggctgaaa 420

tcacccagcg cctgaacgaa atcgaccgtg tatccggcca gactcagttc aacggcgtga 480

aagtcctggc gcaggacaac accctgacca tccaggttgg tgccaacgac ggtgaaacta 540

tcgatattga tttaaaagaa atcagctcta aaacactggg acttgataag cttaatgtcc 600

aagatgccta caccggtggt ggtggttctg gtggtggtgg ttctgctagc ggtggtggtg 660

gttctggtgg tggtggttct atggggacag ttaataaacc tgtggtgggg gtattgatgg 720

ggttcggaat tatcacggga acgttgcgta taacgaatcc ggtcagagca tccgtcttgc 780

gatacgatga ttttcacatc gatgaagaca aactggatac aaactccgta tatgagcctt 840

actaccattc agatcatgcg gagtcttcat gggtaaatcg gggagagtct tcgcgaaaag 900

cgtacgatca taactcacct tatatatggc cacgtaatga ttatgatgga tttttagaga 960

acgcacacga acaccacggg gtgtataatc agggccgtgg tatcgatagc ggggaacggt 1020

taatgcaacc cacacaaatg tctgcacagg aggatcttgg ggacgatacg ggcatccacg 1080

ttatccctac gttaaacggc gatgacagac ataaaattgt aaatgtggac caacgtcaat 1140

acggtgacgt gtttaaagga gatcttaatc caaaacccca aggccaaaga ctcattgagg 1200

tgtcagtgga agaaaatcac ccgtttactt tacgcgcacc gattcagcgg atttatggag 1260

tccggtacac cgagacttgg agctttttgc cgtcattaac ctgtacggga gacgcagcgc 1320

ccgccatcca gcatatatgc ttaaaacata caacatgctt tcaagacgtg gtggtggatg 1380

tggattgcgc ggaaaatact aaagaggatc agttggccga aatcagttac cgttttcaag 1440

gtaagaagga agcggaccaa ccgtggattg ttgtaaacac gagcacactg tttgatgaac 1500

tcgaattaga cccccccgag attgaaccgg gtgtcttgaa agtacttcgg acagaaaaac 1560

aatacttggg tgtgtacatt tggaacatgc gcggctccga tggtacgtct acctacgcca 1620

cgtttttggt cacctggaaa ggggatgaaa aaacaagaaa ccctacgccc gcagtaactc 1680

ctcaaccaag aggggctgag tttcatatgt ggaattacca ctcgcatgta ttttcagttg 1740

gtgatacgtt tagcttggca atgcatcttc agtataagat acatgaagcg ccatttgatt 1800

tgctgttaga gtggttgtat gtccccatcg atcctacatg tcaaccaatg cggttatatt 1860

ctacgtgttt gtatcatccc aacgcacccc aatgcctctc tcatatgaat tccggttgta 1920

catttacctc gccacattta gcccagcgtg ttgcaagcac agtgtatcaa aattgtgaac 1980

atgcagataa ctacaccgca tattgtctgg gaatatctca tatggagcct agctttggtc 2040

taatcttaca cgacgggggc accacgttaa agtttgtaga tacacccgag agtttgtcgg 2100

gattatacgt ttttgtggtg tattttaacg ggcatgttga agccgtagca tacactgttg 2160

tatccacagt agatcatttt gtaaacgcaa ttgaagagcg tggatttccg ccaacggccg 2220

gtcagccacc ggcgactact aaacccaagg aaattacccc cgtaaacccc ggaacgtcac 2280

cacttatacg aggtggtggt ggttctggtg gtggtggttc tggatcccat aacttcagaa 2340

caggcggtga gcttaaagag gttaatacag ataagactga aaacccactg cagaaaattg 2400

atgctgcctt ggcacaggtt gatacacttc gttctgacct gggtgcggtt cagaaccgtt 2460

tcaactccgc tatcaccaac ctgggcaata ccgtaaataa cctgtcttct gcccgtagcc 2520

gtatcgaaga ttccgactac gcaaccgaag tctccaacat gtctcgcgcg cagattctgc 2580

agcaggccgg tacctccgtt ctggcgcagg cgaaccaggt tccgcaaaac gtcctctctt 2640

tactgcgtta actcgaggaa ttcaagcttg ggatctttgt gaaggaacct tacttctgtg 2700

gtgtgacata attggacaaa ctacctacag agatttaaag ctctaaggta aatataaaat 2760

ttttaagtgt ataatgtgtt aaactactga ttctaattgt ttgtgtattt tagattcaca 2820

gtcccaaggc tcatttcagg cccctcagtc ctcacagtct gttcatgatc ataatcagcc 2880

ataccacatt tgtagaggtt ttacttgctt taaaaaacct cccacacctc cccctgaacc 2940

tgaaacataa aatgaatgca attgttgttg ttaacttgtt tattgcagct tataatggtt 3000

acaaataaag caatagcatc acaaatttca caaataaagc atttttttca ctgcattcta 3060

gttgtggttt gtccaaactc atcaatgtat cttatcatgt ct 3102

<210> 27
<211> 295
<212> PRT
<213> Artificial Sequence

<220>
<221> UNSURE
<222> (1)..(295)
<223> unknown

<400> 27

Ala Gln Val Ile Asn Thr Asn Ser Leu Ser Leu Leu Thr Gln Asn Asn
1               5                   10                  15

Leu Asn Lys Ser Gln Ser Ala Leu Gly Thr Ala Ile Glu Arg Leu Ser
            20                  25                  30

Ser Gly Leu Arg Ile Asn Ser Ala Lys Asp Asp Ala Ala Gly Gln Ala
        35                  40                  45

Ile Ala Asn Arg Phe Thr Ala Asn Ile Lys Gly Leu Thr Gln Ala Ser
    50                  55                  60

Arg Asn Ala Asn Asp Gly Ile Ser Ile Ala Gln Thr Thr Glu Gly Ala
65                  70                  75                  80

Leu Asn Glu Ile Asn Asn Asn Leu Gln Arg Val Arg Glu Leu Ala Val
                85                  90                  95

Gln Ser Ala Asn Ser Thr Asn Ser Gln Ser Asp Leu Asp Ser Ile Gln
            100                 105                 110

Ala Glu Ile Thr Gln Arg Leu Asn Glu Ile Asp Arg Val Ser Gly Gln
        115                 120                 125

Thr Gln Phe Asn Gly Val Lys Val Leu Ala Gln Asp Asn Thr Leu Thr
    130                 135                 140

Ile Gln Val Gly Ala Asn Asp Gly Glu Thr Ile Asp Ile Asp Leu Lys
145                 150                 155                 160

Gln Ile Asn Ser Gln Thr Leu Gly Leu Asp Thr Leu Asn Val Gln Ser
                165                 170                 175

Pro Gly Ile Ser Gly Gly Gly Gly Gly Ile Leu Asp Ser Met Gly Lys
            180                 185                 190

Ala Gln Pro Asp Leu Ala Glu Ala Ala Ala Thr Thr Thr Glu Asn Pro
        195                 200                 205

Leu Gln Lys Ile Asp Ala Ala Leu Ala Gln Val Asp Thr Leu Arg Ser
    210                 215                 220

Asp Leu Gly Ala Val Gln Asn Arg Phe Asn Ser Ala Ile Thr Asn Leu
225                 230                 235                 240

Gly Asn Thr Val Asn Asn Leu Thr Ser Ala Arg Ser Arg Ile Glu Asp
                245                 250                 255

Ser Asp Tyr Ala Thr Glu Val Ser Asn Met Ser Arg Ala Gln Ile Leu
            260                 265                 270

Gln Gln Ala Gly Thr Ser Val Leu Ala Gln Ala Asn Gln Val Pro Gln
        275                 280                 285

Asn Val Leu Ser Leu Leu Arg
    290                 295

<210>  28
<211>  885
<212>  DNA
<213>  Artificial Sequence

<220>
<221>  unsure
<222>  (1)..(885)
<223>  unknown

<400>  28
gcgcaggtga ttaacaccaa cagcctgagc ctgctgaccc agaacaacct gaacaaaagc      60

cagagcgcgc tgggcaccgc gattgaacgc ctgagcagcg gcctgcgcat taacagcgcg     120

aaagatgatg cggcgggcca ggcgattgcg aaccgcttta ccgcgaacat taaaggcctg     180

acccaggcga gccgcaacgc gaacgatggc attagcattg cgcagaccac cgaaggcgcg     240

ctgaacgaaa ttaacaacaa cctgcagcgc gtgcgcgaac tggcggtgca gagcgcgaac     300

agcaccaaca gccagagcga tctggatagc attcaggcgg aaattaccca gcgcctgaac     360

gaaattgatc gcgtgagcgg ccagacccag tttaacggcg tgaaagtgct ggcgcaggat     420

aacaccctga ccattcaggt gggcgcgaac gatggcgaaa ccattgatat tgatctgaaa     480

cagattaaca gccagaccct gggcctggat accctgaacg tgcagagccc gggcattagc     540

ggcggcggcg gcggcattct ggatagcatg ggcaaagcgc agccggatct ggcggaagcg     600

gcggcgacca ccaccgaaaa cccgctgcag aaaattgatg cggcgctggc gcaggtggat     660

accctgcgca gcgatctggg cgcggtgcag aaccgcttta acagcgcgat taccaacctg 720

ggcaacaccg tgaacaacct gaccagcgcg cgcagccgca ttgaagatag cgattatgcg 780

accgaagtga gcaacatgag ccgcgcgcag attctgcagc aggcgggcac cagcgtgctg 840

gcgcaggcga accaggtgcc gcagaacgtg ctgagcctgc tgcgc 885

<210> 29
<211> 506
<212> PRT
<213> Unknown

<220>
<221> UNSURE
<222> (1)..(506)
<223> unknown

<400> 29

Met Ala Gln Val Ile Asn Thr Asn Ser Leu Ser Leu Leu Thr Gln Asn
1               5                   10                  15

Asn Leu Asn Lys Ser Gln Ser Ala Leu Gly Thr Ala Ile Glu Arg Leu
            20                  25                  30

Ser Ser Gly Leu Arg Ile Asn Ser Ala Lys Asp Asp Ala Ala Gly Gln
        35                  40                  45

Ala Ile Ala Asn Arg Phe Thr Ala Asn Ile Lys Gly Leu Thr Gln Ala
    50                  55                  60

Ser Arg Asn Ala Asn Asp Gly Ile Ser Ile Ala Gln Thr Thr Glu Gly
65                  70                  75                  80

Ala Leu Asn Glu Ile Asn Asn Asn Leu Gln Arg Val Arg Glu Leu Ala
                85                  90                  95

Val Gln Ser Ala Asn Gly Thr Asn Ser Gln Ser Asp Leu Asp Ser Ile
            100                 105                 110

Gln Ala Glu Ile Thr Gln Arg Leu Asn Glu Ile Asp Arg Val Ser Gly
        115                 120                 125

Gln Thr Gln Phe Asn Gly Val Lys Val Leu Ala Gln Asp Asn Thr Leu
    130                 135                 140

Thr Ile Gln Val Gly Ala Asn Asp Gly Glu Thr Ile Asp Ile Asp Leu
145                 150                 155                 160

Lys Glu Ile Ser Ser Lys Thr Leu Gly Leu Asp Lys Leu Asn Val Gln
                165                 170                 175

Asp Ala Tyr Thr Pro Lys Glu Thr Ala Val Thr Val Asp Lys Thr Thr
            180                 185                 190

Tyr Lys Asn Gly Thr Asp Pro Ile Thr Ala Gln Ser Asn Thr Asp Ile
        195                 200                 205

Gln Thr Ala Ile Gly Gly Gly Ala Thr Gly Val Thr Gly Ala Asp Ile
    210                 215                 220

Lys Phe Lys Asp Gly Gln Tyr Tyr Leu Asp Val Lys Gly Gly Ala Ser
225                 230                 235                 240

Ala Gly Val Tyr Lys Ala Thr Tyr Asp Glu Thr Thr Lys Lys Val Asn
                245                 250                 255

Ile Asp Thr Thr Asp Lys Thr Pro Leu Ala Thr Ala Glu Ala Thr Ala
            260                 265                 270

Ile Arg Gly Thr Ala Thr Ile Thr His Asn Gln Ile Ala Glu Val Thr
        275                 280                 285

Lys Glu Gly Val Asp Thr Thr Thr Val Ala Ala Gln Leu Ala Ala Ala
    290                 295                 300

Gly Val Thr Gly Ala Asp Lys Asp Asn Thr Ser Leu Val Lys Leu Ser
305                 310                 315                 320

Phe Glu Asp Lys Asn Gly Lys Val Ile Asp Gly Gly Tyr Ala Val Lys
                325                 330                 335

Met Gly Asp Asp Phe Tyr Ala Ala Thr Tyr Asp Glu Lys Thr Gly Ala
            340                 345                 350

Ile Thr Ala Lys Thr Thr Thr Tyr Thr Asp Gly Thr Gly Val Ala Gln
        355                 360                 365

Thr Gly Ala Val Lys Phe Gly Gly Ala Asn Gly Lys Ser Glu Val Val
    370                 375                 380

Thr Ala Thr Asp Gly Lys Thr Tyr Leu Ala Ser Asp Leu Asp Lys His
385                 390                 395                 400

Asn Phe Arg Thr Gly Gly Glu Leu Lys Glu Val Asn Thr Asp Lys Thr
                405                 410                 415

Glu Asn Pro Leu Gln Lys Ile Asp Ala Ala Leu Ala Gln Val Asp Thr

420                 425                 430

Leu Arg Ser Asp Leu Gly Ala Val Gln Asn Arg Phe Asn Ser Ala Ile
        435                 440                 445

Thr Asn Leu Gly Asn Thr Val Asn Asn Leu Ser Ser Ala Arg Ser Arg
    450                 455                 460

Ile Glu Asp Ser Asp Tyr Ala Thr Glu Val Ser Asn Met Ser Arg Ala
465                 470                 475                 480

Gln Ile Leu Gln Gln Ala Gly Thr Ser Val Leu Ala Gln Ala Asn Gln
                485                 490                 495

Val Pro Gln Asn Val Leu Ser Leu Leu Arg
            500                 505

<210>  30
<211>  179
<212>  PRT
<213>  Unknown

<220>
<221>  UNSURE
<222>  (1)..(179)
<223>  unknown

<400>  30

Ala Gln Val Ile Asn Thr Asn Ser Leu Ser Leu Leu Thr Gln Asn Asn
1               5                   10                  15

Leu Asn Lys Ser Gln Ser Ala Leu Gly Thr Ala Ile Glu Arg Leu Ser
            20                  25                  30

Ser Gly Leu Arg Ile Asn Ser Ala Lys Asp Asp Ala Ala Gly Gln Ala
        35                  40                  45

Ile Ala Asn Arg Phe Thr Ala Asn Ile Lys Gly Leu Thr Gln Ala Ser
    50                  55                  60

Arg Asn Ala Asn Asp Gly Ile Ser Ile Ala Gln Thr Thr Glu Gly Ala
65                  70                  75                  80

Leu Asn Glu Ile Asn Asn Asn Leu Gln Arg Val Arg Glu Leu Ala Val
                85                  90                  95

Gln Ser Ala Asn Gly Thr Asn Ser Gln Ser Asp Leu Asp Ser Ile Gln
            100                 105                 110

Ala Glu Ile Thr Gln Arg Leu Asn Glu Ile Asp Arg Val Ser Gly Gln
        115                 120                 125

Thr Gln Phe Asn Gly Val Lys Val Leu Ala Gln Asp Asn Thr Leu Thr
    130                 135                 140

Ile Gln Val Gly Ala Asn Asp Gly Glu Thr Ile Asp Ile Asp Leu Lys
145                 150                 155                 160

Glu Ile Ser Ser Lys Thr Leu Gly Leu Asp Lys Leu Asn Val Gln Asp
                165                 170                 175

Ala Tyr Thr

<210>  31
<211>  107
<212>  PRT
<213>  Unknown

<220>
<221>  UNSURE
<222>  (1)..(107)
<223>  unknown

<400>  31

His Asn Phe Arg Thr Gly Gly Glu Leu Lys Glu Val Asn Thr Asp Lys
1               5                   10                  15

Thr Glu Asn Pro Leu Gln Lys Ile Asp Ala Ala Leu Ala Gln Val Asp
            20                  25                  30

Thr Leu Arg Ser Asp Leu Gly Ala Val Gln Asn Arg Phe Asn Ser Ala
        35                  40                  45

Ile Thr Asn Leu Gly Asn Thr Val Asn Asn Leu Ser Ser Ala Arg Ser
    50                  55                  60

Arg Ile Glu Asp Ser Asp Tyr Ala Thr Glu Val Ser Asn Met Ser Arg
65                  70                  75                  80

Ala Gln Ile Leu Gln Gln Ala Gly Thr Ser Val Leu Ala Gln Ala Asn
                85                  90                  95

Gln Val Pro Gln Asn Val Leu Ser Leu Leu Arg
            100                 105

<210>  32
<211>  824
<212>  PRT
<213>  Unknown

<220>
<221>  UNSURE
<222>  (1)..(824)
<223>  unknown

<400>  32

Ala Gln Val Ile Asn Thr Asn Ser Leu Ser Leu Leu Thr Gln Asn Asn
1               5                   10                  15

Leu Asn Lys Ser Gln Ser Ala Leu Gly Thr Ala Ile Glu Arg Leu Ser
            20                  25                  30

Ser Gly Leu Arg Ile Asn Ser Ala Lys Asp Asp Ala Ala Gly Gln Ala
        35                  40                  45

Ile Ala Asn Arg Phe Thr Ala Asn Ile Lys Gly Leu Thr Gln Ala Ser
    50                  55                  60

Arg Asn Ala Asn Asp Gly Ile Ser Ile Ala Gln Thr Thr Glu Gly Ala
65                  70                  75                  80

Leu Asn Glu Ile Asn Asn Asn Leu Gln Arg Val Arg Glu Leu Ala Val
                85                  90                  95

Gln Ser Ala Asn Gly Thr Asn Ser Gln Ser Asp Leu Asp Ser Ile Gln
            100                 105                 110

Ala Glu Ile Thr Gln Arg Leu Asn Glu Ile Asp Arg Val Ser Gly Gln
        115                 120                 125

Thr Gln Phe Asn Gly Val Lys Val Leu Ala Gln Asp Asn Thr Leu Thr
    130                 135                 140

Ile Gln Val Gly Ala Asn Asp Gly Glu Thr Ile Asp Ile Asp Leu Lys
145                 150                 155                 160

Glu Ile Ser Ser Lys Thr Leu Gly Leu Asp Lys Leu Asn Val Gln Asp
                165                 170                 175

Ala Tyr Thr Ser Pro Gly Ile Ser Gly Gly Gly Gly Ile Leu Asp
            180                 185                 190

Ser Met Gly His Asn Phe Arg Thr Gly Gly Glu Leu Lys Glu Val Asn
        195                 200                 205

Thr Asp Lys Thr Glu Asn Pro Leu Gln Lys Ile Asp Ala Ala Leu Ala
    210                 215                 220

Gln Val Asp Thr Leu Arg Ser Asp Leu Gly Ala Val Gln Asn Arg Phe
225                 230                 235                 240

Asn Ser Ala Ile Thr Asn Leu Gly Asn Thr Val Asn Asn Leu Ser Ser
                245                 250                 255

Ala Arg Ser Arg Ile Glu Asp Ser Asp Tyr Ala Thr Glu Val Ser Asn
            260                 265                 270

Met Ser Arg Ala Gln Ile Leu Gln Gln Ala Gly Thr Ser Val Leu Ala
        275                 280                 285

Gln Ala Asn Gln Val Pro Gln Asn Val Leu Ser Leu Leu Arg Gly Gly
    290                 295                 300

Gly Gly Ser Gly Gly Gly Gly Ser Gly Gly Gly Gly Ser Ser Val Leu
305                 310                 315                 320

Arg Tyr Asp Asp Phe His Ile Asp Glu Asp Lys Leu Asp Thr Asn Ser
                325                 330                 335

Val Tyr Glu Pro Tyr Tyr His Ser Asp His Ala Glu Ser Ser Trp Val
            340                 345                 350

Asn Arg Gly Glu Ser Ser Arg Lys Ala Tyr Asp His Asn Ser Pro Tyr
        355                 360                 365

Ile Trp Pro Arg Asn Asp Tyr Asp Gly Phe Leu Glu Asn Ala His Glu
    370                 375                 380

His His Gly Val Tyr Asn Gln Gly Arg Gly Ile Asp Ser Gly Glu Arg
385                 390                 395                 400

Leu Met Gln Pro Thr Gln Met Ser Ala Gln Glu Asp Leu Gly Asp Asp
                405                 410                 415

Thr Gly Ile His Val Ile Pro Thr Leu Asn Gly Asp Asp Arg His Lys
            420                 425                 430

Ile Val Asn Val Asp Gln Arg Gln Tyr Gly Asp Val Phe Lys Gly Asp
        435                 440                 445

Leu Asn Pro Lys Pro Gln Gly Gln Arg Leu Ile Glu Val Ser Val Glu
    450                 455                 460

Glu Asn His Pro Phe Thr Leu Arg Ala Pro Ile Gln Arg Ile Tyr Gly
465                 470                 475                 480

Val Arg Tyr Thr Glu Thr Trp Ser Phe Leu Pro Ser Leu Thr Cys Thr
                485                 490                 495

Gly Asp Ala Ala Pro Ala Ile Gln His Ile Cys Leu Lys His Thr Thr
            500                 505                 510

Cys Phe Gln Asp Val Val Val Asp Val Asp Cys Ala Glu Asn Thr Lys
        515                 520                 525

Glu Asp Gln Leu Ala Glu Ile Ser Tyr Arg Phe Gln Gly Lys Lys Glu
    530                 535                 540

Ala Asp Gln Pro Trp Ile Val Val Asn Thr Ser Thr Leu Phe Asp Glu
545                 550                 555                 560

Leu Glu Leu Asp Pro Pro Glu Ile Glu Pro Gly Val Leu Lys Val Leu
                565                 570                 575

Arg Thr Glu Lys Gln Tyr Leu Gly Val Tyr Ile Trp Asn Met Arg Gly
            580                 585                 590

Ser Asp Gly Thr Ser Thr Tyr Ala Thr Phe Leu Val Thr Trp Lys Gly
        595                 600                 605

Asp Glu Lys Thr Arg Asn Pro Thr Pro Ala Val Thr Pro Gln Pro Arg
    610                 615                 620

Gly Ala Glu Phe His Met Trp Asn Tyr His Ser His Val Phe Ser Val
625                 630                 635                 640

Gly Asp Thr Phe Ser Leu Ala Met His Leu Gln Tyr Lys Ile His Glu
                645                 650                 655

Ala Pro Phe Asp Leu Leu Leu Glu Trp Leu Tyr Val Pro Ile Asp Pro
            660                 665                 670

Thr Cys Gln Pro Met Arg Leu Tyr Ser Thr Cys Leu Tyr His Pro Asn
        675                 680                 685

Ala Pro Gln Cys Leu Ser His Met Asn Ser Gly Cys Thr Phe Thr Ser
    690                 695                 700

Pro His Leu Ala Gln Arg Val Ala Ser Thr Val Tyr Gln Asn Cys Glu
705                 710                 715                 720

His Ala Asp Asn Tyr Thr Ala Tyr Cys Leu Gly Ile Ser His Met Glu

725                 730                 735

Pro Ser Phe Gly Leu Ile Leu His Asp Gly Gly Thr Thr Leu Lys Phe
            740                 745                 750

Val Asp Thr Pro Glu Ser Leu Ser Gly Leu Tyr Val Phe Val Val Tyr
        755                 760                 765

Phe Asn Gly His Val Glu Ala Val Ala Tyr Thr Val Val Ser Thr Val
    770                 775                 780

Asp His Phe Val Asn Ala Ile Glu Glu Arg Gly Phe Pro Pro Thr Ala
785                 790                 795                 800

Gly Gln Pro Pro Ala Thr Thr Lys Pro Lys Glu Ile Thr Pro Val Asn
                805                 810                 815

Pro Gly Thr Ser Pro Leu Ile Arg
            820

<210>  33
<211>  824
<212>  PRT
<213>  Unknown

<220>
<221>  UNSURE
<222>  (1)..(824)
<223>  unknown

<400>  33

Ser Val Leu Arg Tyr Asp Asp Phe His Ile Asp Glu Asp Lys Leu Asp
1               5                   10                  15

Thr Asn Ser Val Tyr Glu Pro Tyr Tyr His Ser Asp His Ala Glu Ser
            20                  25                  30

Ser Trp Val Asn Arg Gly Glu Ser Ser Arg Lys Ala Tyr Asp His Asn
        35                  40                  45

Ser Pro Tyr Ile Trp Pro Arg Asn Asp Tyr Asp Gly Phe Leu Glu Asn
    50                  55                  60

Ala His Glu His His Gly Val Tyr Asn Gln Gly Arg Gly Ile Asp Ser
65                  70                  75                  80

Gly Glu Arg Leu Met Gln Pro Thr Gln Met Ser Ala Gln Glu Asp Leu
                85                  90                  95

Gly Asp Asp Thr Gly Ile His Val Ile Pro Thr Leu Asn Gly Asp Asp
            100                 105                 110

Arg His Lys Ile Val Asn Val Asp Gln Arg Gln Tyr Gly Asp Val Phe
        115                 120                 125

Lys Gly Asp Leu Asn Pro Lys Pro Gln Gly Gln Arg Leu Ile Glu Val
    130                 135                 140

Ser Val Glu Glu Asn His Pro Phe Thr Leu Arg Ala Pro Ile Gln Arg
145                 150                 155                 160

Ile Tyr Gly Val Arg Tyr Thr Glu Thr Trp Ser Phe Leu Pro Ser Leu
                165                 170                 175

Thr Cys Thr Gly Asp Ala Ala Pro Ala Ile Gln His Ile Cys Leu Lys
            180                 185                 190

His Thr Thr Cys Phe Gln Asp Val Val Val Asp Val Asp Cys Ala Glu
        195                 200                 205

Asn Thr Lys Glu Asp Gln Leu Ala Glu Ile Ser Tyr Arg Phe Gln Gly
    210                 215                 220

Lys Lys Glu Ala Asp Gln Pro Trp Ile Val Val Asn Thr Ser Thr Leu
225                 230                 235                 240

Phe Asp Glu Leu Glu Leu Asp Pro Pro Glu Ile Glu Pro Gly Val Leu
                245                 250                 255

Lys Val Leu Arg Thr Glu Lys Gln Tyr Leu Gly Val Tyr Ile Trp Asn
            260                 265                 270

Met Arg Gly Ser Asp Gly Thr Ser Thr Tyr Ala Thr Phe Leu Val Thr
        275                 280                 285

Trp Lys Gly Asp Glu Lys Thr Arg Asn Pro Thr Pro Ala Val Thr Pro
    290                 295                 300

Gln Pro Arg Gly Ala Glu Phe His Met Trp Asn Tyr His Ser His Val
305                 310                 315                 320

Phe Ser Val Gly Asp Thr Phe Ser Leu Ala Met His Leu Gln Tyr Lys
                325                 330                 335

Ile His Glu Ala Pro Phe Asp Leu Leu Leu Glu Trp Leu Tyr Val Pro
            340                 345                 350

Ile Asp Pro Thr Cys Gln Pro Met Arg Leu Tyr Ser Thr Cys Leu Tyr
        355                 360                 365

His Pro Asn Ala Pro Gln Cys Leu Ser His Met Asn Ser Gly Cys Thr
    370                 375                 380

Phe Thr Ser Pro His Leu Ala Gln Arg Val Ala Ser Thr Val Tyr Gln
385                 390                 395                 400

Asn Cys Glu His Ala Asp Asn Tyr Thr Ala Tyr Cys Leu Gly Ile Ser
                405                 410                 415

His Met Glu Pro Ser Phe Gly Leu Ile Leu His Asp Gly Gly Thr Thr
            420                 425                 430

Leu Lys Phe Val Asp Thr Pro Glu Ser Leu Ser Gly Leu Tyr Val Phe
        435                 440                 445

Val Val Tyr Phe Asn Gly His Val Glu Ala Val Ala Tyr Thr Val Val
    450                 455                 460

Ser Thr Val Asp His Phe Val Asn Ala Ile Glu Glu Arg Gly Phe Pro
465                 470                 475                 480

Pro Thr Ala Gly Gln Pro Pro Ala Thr Thr Lys Pro Lys Glu Ile Thr
                485                 490                 495

Pro Val Asn Pro Gly Thr Ser Pro Leu Ile Arg Gly Gly Gly Gly Ser
            500                 505                 510

Gly Gly Gly Gly Ser Gly Gly Gly Gly Ser Ala Gln Val Ile Asn Thr
        515                 520                 525

Asn Ser Leu Ser Leu Leu Thr Gln Asn Asn Leu Asn Lys Ser Gln Ser
    530                 535                 540

Ala Leu Gly Thr Ala Ile Glu Arg Leu Ser Ser Gly Leu Arg Ile Asn
545                 550                 555                 560

Ser Ala Lys Asp Asp Ala Ala Gly Gln Ala Ile Ala Asn Arg Phe Thr
                565                 570                 575

Ala Asn Ile Lys Gly Leu Thr Gln Ala Ser Arg Asn Ala Asn Asp Gly
            580                 585                 590

Ile Ser Ile Ala Gln Thr Thr Glu Gly Ala Leu Asn Glu Ile Asn Asn
        595                 600                 605

Asn Leu Gln Arg Val Arg Glu Leu Ala Val Gln Ser Ala Asn Gly Thr
    610                 615                 620

Asn Ser Gln Ser Asp Leu Asp Ser Ile Gln Ala Glu Ile Thr Gln Arg
625                 630                 635                 640

Leu Asn Glu Ile Asp Arg Val Ser Gly Gln Thr Gln Phe Asn Gly Val
                645                 650                 655

Lys Val Leu Ala Gln Asp Asn Thr Leu Thr Ile Gln Val Gly Ala Asn
            660                 665                 670

Asp Gly Glu Thr Ile Asp Ile Asp Leu Lys Glu Ile Ser Ser Lys Thr
        675                 680                 685

Leu Gly Leu Asp Lys Leu Asn Val Gln Asp Ala Tyr Thr Ser Pro Gly
    690                 695                 700

Ile Ser Gly Gly Gly Gly Gly Ile Leu Asp Ser Met Gly His Asn Phe
705                 710                 715                 720

Arg Thr Gly Gly Glu Leu Lys Glu Val Asn Thr Asp Lys Thr Glu Asn
                725                 730                 735

Pro Leu Gln Lys Ile Asp Ala Ala Leu Ala Gln Val Asp Thr Leu Arg
            740                 745                 750

Ser Asp Leu Gly Ala Val Gln Asn Arg Phe Asn Ser Ala Ile Thr Asn
        755                 760                 765

Leu Gly Asn Thr Val Asn Asn Leu Ser Ser Ala Arg Ser Arg Ile Glu
    770                 775                 780

Asp Ser Asp Tyr Ala Thr Glu Val Ser Asn Met Ser Arg Ala Gln Ile
785                 790                 795                 800

Leu Gln Gln Ala Gly Thr Ser Val Leu Ala Gln Ala Asn Gln Val Pro
                805                 810                 815

Gln Asn Val Leu Ser Leu Leu Arg
            820

<210>  34
<211>  823
<212>  PRT
<213>  Unknown

<220>
<221>  UNSURE
<222>  (1)..(823)
<223>  unknown

<400>  34

Ala Gln Val Ile Asn Thr Asn Ser Leu Ser Leu Leu Thr Gln Asn Asn
1               5                   10                  15

Leu Asn Lys Ser Gln Ser Ala Leu Gly Thr Ala Ile Glu Arg Leu Ser
            20                  25                  30

Ser Gly Leu Arg Ile Asn Ser Ala Lys Asp Asp Ala Ala Gly Gln Ala
        35                  40                  45

Ile Ala Asn Arg Phe Thr Ala Asn Ile Lys Gly Leu Thr Gln Ala Ser
    50                  55                  60

Arg Asn Ala Asn Asp Gly Ile Ser Ile Ala Gln Thr Thr Glu Gly Ala
65                  70                  75                  80

Leu Asn Glu Ile Asn Asn Asn Leu Gln Arg Val Arg Glu Leu Ala Val
                85                  90                  95

Gln Ser Ala Asn Gly Thr Asn Ser Gln Ser Asp Leu Asp Ser Ile Gln
            100                 105                 110

Ala Glu Ile Thr Gln Arg Leu Asn Glu Ile Asp Arg Val Ser Gly Gln
        115                 120                 125

Thr Gln Phe Asn Gly Val Lys Val Leu Ala Gln Asp Asn Thr Leu Thr
    130                 135                 140

Ile Gln Val Gly Ala Asn Asp Gly Glu Thr Ile Asp Ile Asp Leu Lys
145                 150                 155                 160

Glu Ile Ser Ser Lys Thr Leu Gly Leu Asp Lys Leu Asn Val Gln Asp
                165                 170                 175

Ala Tyr Thr Gly Gly Gly Gly Ser Gly Gly Gly Gly Ser Gly Gly Gly
            180                 185                 190

Gly Ser Ser Val Leu Arg Tyr Asp Asp Phe His Ile Asp Glu Asp Lys
        195                 200                 205

Leu Asp Thr Asn Ser Val Tyr Glu Pro Tyr Tyr His Ser Asp His Ala
    210                 215                 220

Glu Ser Ser Trp Val Asn Arg Gly Glu Ser Ser Arg Lys Ala Tyr Asp
225                 230                 235                 240

His Asn Ser Pro Tyr Ile Trp Pro Arg Asn Asp Tyr Asp Gly Phe Leu
                245                 250                 255

Glu Asn Ala His Glu His His Gly Val Tyr Asn Gln Gly Arg Gly Ile
            260                 265                 270

Asp Ser Gly Glu Arg Leu Met Gln Pro Thr Gln Met Ser Ala Gln Glu
        275                 280                 285

Asp Leu Gly Asp Asp Thr Gly Ile His Val Ile Pro Thr Leu Asn Gly
    290                 295                 300

Asp Asp Arg His Lys Ile Val Asn Val Asp Gln Arg Gln Tyr Gly Asp
305                 310                 315                 320

Val Phe Lys Gly Asp Leu Asn Pro Lys Pro Gln Gly Gln Arg Leu Ile
                325                 330                 335

Glu Val Ser Val Glu Glu Asn His Pro Phe Thr Leu Arg Ala Pro Ile
            340                 345                 350

Gln Arg Ile Tyr Gly Val Arg Tyr Thr Glu Thr Trp Ser Phe Leu Pro
        355                 360                 365

Ser Leu Thr Cys Thr Gly Asp Ala Ala Pro Ala Ile Gln His Ile Cys
    370                 375                 380

Leu Lys His Thr Thr Cys Phe Gln Asp Val Val Val Asp Val Asp Cys
385                 390                 395                 400

Ala Glu Asn Thr Lys Glu Asp Gln Leu Ala Glu Ile Ser Tyr Arg Phe
                405                 410                 415

Gln Gly Lys Lys Glu Ala Asp Gln Pro Trp Ile Val Val Asn Thr Ser
            420                 425                 430

Thr Leu Phe Asp Glu Leu Glu Leu Asp Pro Pro Glu Ile Glu Pro Gly
        435                 440                 445

Val Leu Lys Val Leu Arg Thr Glu Lys Gln Tyr Leu Gly Val Tyr Ile
    450                 455                 460

Trp Asn Met Arg Gly Ser Asp Gly Thr Ser Thr Tyr Ala Thr Phe Leu
465                 470                 475                 480

Val Thr Trp Lys Gly Asp Glu Lys Thr Arg Asn Pro Thr Pro Ala Val
                485                 490                 495

Thr Pro Gln Pro Arg Gly Ala Glu Phe His Met Trp Asn Tyr His Ser
            500                 505                 510

His Val Phe Ser Val Gly Asp Thr Phe Ser Leu Ala Met His Leu Gln
        515                 520                 525

Tyr Lys Ile His Glu Ala Pro Phe Asp Leu Leu Leu Glu Trp Leu Tyr
    530                 535                 540

Val Pro Ile Asp Pro Thr Cys Gln Pro Met Arg Leu Tyr Ser Thr Cys
545                 550                 555                 560

Leu Tyr His Pro Asn Ala Pro Gln Cys Leu Ser His Met Asn Ser Gly
                565                 570                 575

Cys Thr Phe Thr Ser Pro His Leu Ala Gln Arg Val Ala Ser Thr Val
            580                 585                 590

Tyr Gln Asn Cys Glu His Ala Asp Asn Tyr Thr Ala Tyr Cys Leu Gly
        595                 600                 605

Ile Ser His Met Glu Pro Ser Phe Gly Leu Ile Leu His Asp Gly Gly
    610                 615                 620

Thr Thr Leu Lys Phe Val Asp Thr Pro Glu Ser Leu Ser Gly Leu Tyr
625                 630                 635                 640

Val Phe Val Val Tyr Phe Asn Gly His Val Glu Ala Val Ala Tyr Thr
                645                 650                 655

Val Val Ser Thr Val Asp His Phe Val Asn Ala Ile Glu Glu Arg Gly
            660                 665                 670

Phe Pro Pro Thr Ala Gly Gln Pro Pro Ala Thr Thr Lys Pro Lys Glu
        675                 680                 685

Ile Thr Pro Val Asn Pro Gly Thr Ser Pro Leu Ile Arg Gly Gly Gly
    690                 695                 700

Gly Ser Gly Gly Gly Gly Ser Gly Gly Gly Gly Ser His Asn Phe Arg
705                 710                 715                 720

Thr Gly Gly Glu Leu Lys Glu Val Asn Thr Asp Lys Thr Glu Asn Pro
                725                 730                 735

Leu Gln Lys Ile Asp Ala Ala Leu Ala Gln Val Asp Thr Leu Arg Ser
            740                 745                 750

Asp Leu Gly Ala Val Gln Asn Arg Phe Asn Ser Ala Ile Thr Asn Leu
        755                 760                 765

Gly Asn Thr Val Asn Asn Leu Ser Ser Ala Arg Ser Arg Ile Glu Asp
    770                 775                 780

Ser Asp Tyr Ala Thr Glu Val Ser Asn Met Ser Arg Ala Gln Ile Leu
785                 790                 795                 800

Gln Gln Ala Gly Thr Ser Val Leu Ala Gln Ala Asn Gln Val Pro Gln
                805                 810                 815

Asn Val Leu Ser Leu Leu Arg
            820

<210>  35
<211>  517
<212>  PRT
<213>  Artificial Sequence

<220>
<221>  UNSURE
<222>  (1)..(517)
<223>  unknown

<400>  35

Met Gly Ser Val Leu Arg Tyr Asp Asp Phe His Ile Asp Glu Asp Lys
1               5                   10                  15

Leu Asp Thr Asn Ser Val Tyr Glu Pro Tyr Tyr His Ser Asp His Ala
            20                  25                  30

Glu Ser Ser Trp Val Asn Arg Gly Glu Ser Ser Arg Lys Ala Tyr Asp
        35                  40                  45

His Asn Ser Pro Tyr Ile Trp Pro Arg Asn Asp Tyr Asp Gly Phe Leu
    50                  55                  60

Glu Asn Ala His Glu His His Gly Val Tyr Asn Gln Gly Arg Gly Ile
65                  70                  75                  80

Asp Ser Gly Glu Arg Leu Met Gln Pro Thr Gln Met Ser Ala Gln Glu
                85                  90                  95

Asp Leu Gly Asp Asp Thr Gly Ile His Val Ile Pro Thr Leu Asn Gly

            100                 105                 110

Asp Asp Arg His Lys Ile Val Asn Val Asp Gln Arg Gln Tyr Gly Asp
        115                 120                 125

Val Phe Lys Gly Asp Leu Asn Pro Lys Pro Gln Gly Gln Arg Leu Ile
    130                 135                 140

Glu Val Ser Val Glu Glu Asn His Pro Phe Thr Leu Arg Ala Pro Ile
145                 150                 155                 160

Gln Arg Ile Tyr Gly Val Arg Tyr Thr Glu Thr Trp Ser Phe Leu Pro
                165                 170                 175

Ser Leu Thr Cys Thr Gly Asp Ala Ala Pro Ala Ile Gln His Ile Cys
            180                 185                 190

Leu Lys His Thr Thr Cys Phe Gln Asp Val Val Val Asp Val Asp Cys
        195                 200                 205

Ala Glu Asn Thr Lys Glu Asp Gln Leu Ala Glu Ile Ser Tyr Arg Phe
    210                 215                 220

Gln Gly Lys Lys Glu Ala Asp Gln Pro Trp Ile Val Val Asn Thr Ser
225                 230                 235                 240

Thr Leu Phe Asp Glu Leu Glu Leu Asp Pro Pro Glu Ile Glu Pro Gly
                245                 250                 255

Val Leu Lys Val Leu Arg Thr Glu Lys Gln Tyr Leu Gly Val Tyr Ile
            260                 265                 270

Trp Asn Met Arg Gly Ser Asp Gly Thr Ser Thr Tyr Ala Thr Phe Leu
        275                 280                 285

Val Thr Trp Lys Gly Asp Glu Lys Thr Arg Asn Pro Thr Pro Ala Val
    290                 295                 300

Thr Pro Gln Pro Arg Gly Ala Glu Phe His Met Trp Asn Tyr His Ser
305                 310                 315                 320

His Val Phe Ser Val Gly Asp Thr Phe Ser Leu Ala Met His Leu Gln
                325                 330                 335

Tyr Lys Ile His Glu Ala Pro Phe Asp Leu Leu Leu Glu Trp Leu Tyr
            340                 345                 350

Val Pro Ile Asp Pro Thr Cys Gln Pro Met Arg Leu Tyr Ser Thr Cys
        355                 360                 365

Leu Tyr His Pro Asn Ala Pro Gln Cys Leu Ser His Met Asn Ser Gly
    370                 375                 380

Cys Thr Phe Thr Ser Pro His Leu Ala Gln Arg Val Ala Ser Thr Val
385                 390                 395                 400

Tyr Gln Asn Cys Glu His Ala Asp Asn Tyr Thr Ala Tyr Cys Leu Gly
                405                 410                 415

Ile Ser His Met Glu Pro Ser Phe Gly Leu Ile Leu His Asp Gly Gly
            420                 425                 430

Thr Thr Leu Lys Phe Val Asp Thr Pro Glu Ser Leu Ser Gly Leu Tyr
        435                 440                 445

Val Phe Val Val Tyr Phe Asn Gly His Val Glu Ala Val Ala Tyr Thr
    450                 455                 460

Val Val Ser Thr Val Asp His Phe Val Asn Ala Ile Glu Glu Arg Gly
465                 470                 475                 480

Phe Pro Pro Thr Ala Gly Gln Pro Pro Ala Thr Thr Lys Pro Lys Glu
                485                 490                 495

Ile Thr Pro Val Asn Pro Gly Thr Ser Pro Leu Ile Arg Leu Glu His
            500                 505                 510

His His His His His
        515

<210>  36
<211>  1554
<212>  DNA
<213>  Artificial Sequence

<220>
<221>  unsure
<222>  (1)..(1554)
<223>  unknown

<400>  36
atgggtagcg ttctgcgtta cgatgatttc cacatcgatg aagataaact ggacaccaac      60

agcgtttacg aaccgtatta ccactctgat cacgctgaat ctagctgggt aaaccgtggt     120

gaaagcagcc gtaaagctta cgatcataac tctccgtaca tctggccgcg taacgattat     180

gacggctttc tggaaaacgc tcacgaacac cacggtgtgt acaaccaggg ccgcggtatc     240

gattctggtg aacgtctgat gcagccgacc cagatgtctg ctcaggaaga tctgggtgat 300

gatactggta ttcacgtgat cccgaccctg aatggtgatg atcgtcataa aatcgttaac 360

gttgatcagc gtcagtatgg tgatgttttc aaaggtgatc tgaacccgaa accgcagggt 420

cagcgtctga tcgaagtgtc tgtggaagaa aaccacccgt tcactctgcg tgctccgatt 480

cagcgtattt atggcgttcg ttacaccgaa acctggtctt ttctgccgtc tctgacttgc 540

actggcgatg cggctccggc aattcagcat atttgcctga aacatactac ttgctttcag 600

gatgtagtgg ttgatgttga ttgcgcggaa aacactaaag aagatcagct ggctgaaatc 660

agctaccgtt tccagggtaa aaaagaagcc gatcagccgt ggattgttgt gaacaccagc 720

accctgtttg atgaactgga actggatccg ccggaaattg aaccgggtgt tctgaaagtt 780

ctgcgtaccg aaaaacagta tctgggcgtg tatatttgga acatgcgtgg ttctgacggt 840

accagcacct acgctacctt cctggtgacc tggaaaggtg atgaaaaaac ccgcaacccg 900

accccggcgg ttaccccgca gccgcgtggc gctgaattcc atatgtggaa ctatcacagc 960

cacgtgttca gcgttggtga caccttctct ctggcgatgc atctgcagta taaaatccac 1020

gaagcaccgt tcgatctgct gcttgaatgg ctgtacgttc cgattgatcc gacctgccag 1080

ccgatgcgtc tgtactccac ctgcctgtac catccgaacg caccacagtg cctgagccat 1140

atgaactccg gctgtacgtt taccagcccg cacctggcgc agcgtgtggc tagcaccgtg 1200

taccagaact gcgaacatgc ggacaactat accgcatact gcctgggtat ttctcatatg 1260

gaaccgtctt tcggtctgat cctgcacgat ggcggcacca ctttgaaatt cgttgatact 1320

ccggaatccc tgtctggcct gtacgttttc gtggtgtact tcaacggtca cgtggaagca 1380

gtggcgtaca ccgttgttag caccgttgat cactttgtta acgcaattga agaacgtggc 1440

ttcccgccga ccgctggtca gccgccggca accactaaac cgaaagaaat taccccggtt 1500

aacccaggca ccagcccgct gatccgtctc gagcaccacc accaccacca ctga 1554

<210> 37
<211> 826
<212> PRT
<213> Artificial Sequence

<220>
<221> UNSURE
<222> (1)..(826)
<223> unknown

<400> 37

Met Ala Gln Val Ile Asn Thr Asn Ser Leu Ser Leu Leu Thr Gln Asn
1               5                   10                  15

Asn Leu Asn Lys Ser Gln Ser Ala Leu Gly Thr Ala Ile Glu Arg Leu
            20                  25                  30

Ser Ser Gly Leu Arg Ile Asn Ser Ala Lys Asp Asp Ala Ala Gly Gln
        35                  40                  45

Ala Ile Ala Asn Arg Phe Thr Ala Asn Ile Lys Gly Leu Thr Gln Ala
    50                  55                  60

Ser Arg Asn Ala Asn Asp Gly Ile Ser Ile Ala Gln Thr Thr Glu Gly
65                  70                  75                  80

Ala Leu Asn Glu Ile Asn Asn Asn Leu Gln Arg Val Arg Glu Leu Ala
                85                  90                  95

Val Gln Ser Ala Asn Ser Thr Asn Ser Gln Ser Asp Leu Asp Ser Ile
            100                 105                 110

Gln Ala Glu Ile Thr Gln Arg Leu Asn Glu Ile Asp Arg Val Ser Gly
        115                 120                 125

Gln Thr Gln Phe Asn Gly Val Lys Val Leu Ala Gln Asp Asn Thr Leu
    130                 135                 140

Thr Ile Gln Val Gly Ala Asn Asp Gly Glu Thr Ile Asp Ile Asp Leu
145                 150                 155                 160

Lys Gln Ile Asn Ser Gln Thr Leu Gly Leu Asp Thr Leu Asn Val Gln
                165                 170                 175

Ser Pro Gly Ile Ser Gly Gly Gly Gly Gly Ile Leu Asp Ser Met Gly
            180                 185                 190

Lys Ala Gln Pro Asp Leu Ala Glu Ala Ala Ala Thr Thr Thr Glu Asn
        195                 200                 205

Pro Leu Gln Lys Ile Asp Ala Ala Leu Ala Gln Val Asp Thr Leu Arg
    210                 215                 220

Ser Asp Leu Gly Ala Val Gln Asn Arg Phe Asn Ser Ala Ile Thr Asn
225                 230                 235                 240

Leu Gly Asn Thr Val Asn Asn Leu Thr Ser Ala Arg Ser Arg Ile Glu
                245                 250                 255

Asp Ser Asp Tyr Ala Thr Glu Val Ser Asn Met Ser Arg Ala Gln Ile
            260                 265                 270

Leu Gln Gln Ala Gly Thr Ser Val Leu Ala Gln Ala Asn Gln Val Pro

275                 280                 285

Gln Asn Val Leu Ser Leu Leu Arg Gly Gly Gly Gly Ser Gly Gly Gly
    290                 295                 300

Gly Ser Gly Gly Gly Gly Ser Ser Val Leu Arg Tyr Asp Asp Phe His
305                 310                 315                 320

Ile Asp Glu Asp Lys Leu Asp Thr Asn Ser Val Tyr Glu Pro Tyr Tyr
                325                 330                 335

His Ser Asp His Ala Glu Ser Ser Trp Val Asn Arg Gly Glu Ser Ser
            340                 345                 350

Arg Lys Ala Tyr Asp His Asn Ser Pro Tyr Ile Trp Pro Arg Asn Asp
        355                 360                 365

Tyr Asp Gly Phe Leu Glu Asn Ala His Glu His His Gly Val Tyr Asn
    370                 375                 380

Gln Gly Arg Gly Ile Asp Ser Gly Glu Arg Leu Met Gln Pro Thr Gln
385                 390                 395                 400

Met Ser Ala Gln Glu Asp Leu Gly Asp Asp Thr Gly Ile His Val Ile
                405                 410                 415

Pro Thr Leu Asn Gly Asp Asp Arg His Lys Ile Val Asn Val Asp Gln
            420                 425                 430

Arg Gln Tyr Gly Asp Val Phe Lys Gly Asp Leu Asn Pro Lys Pro Gln
        435                 440                 445

Gly Gln Arg Leu Ile Glu Val Ser Val Glu Glu Asn His Pro Phe Thr
    450                 455                 460

Leu Arg Ala Pro Ile Gln Arg Ile Tyr Gly Val Arg Tyr Thr Glu Thr
465                 470                 475                 480

Trp Ser Phe Leu Pro Ser Leu Thr Cys Thr Gly Asp Ala Ala Pro Ala
                485                 490                 495

Ile Gln His Ile Cys Leu Lys His Thr Thr Cys Phe Gln Asp Val Val
            500                 505                 510

Val Asp Val Asp Cys Ala Glu Asn Thr Lys Glu Asp Gln Leu Ala Glu
        515                 520                 525

Ile Ser Tyr Arg Phe Gln Gly Lys Lys Glu Ala Asp Gln Pro Trp Ile
    530                 535                 540

Val Val Asn Thr Ser Thr Leu Phe Asp Glu Leu Glu Leu Asp Pro Pro
545                 550                 555                 560

Glu Ile Glu Pro Gly Val Leu Lys Val Leu Arg Thr Glu Lys Gln Tyr
                565                 570                 575

Leu Gly Val Tyr Ile Trp Asn Met Arg Gly Ser Asp Gly Thr Ser Thr
            580                 585                 590

Tyr Ala Thr Phe Leu Val Thr Trp Lys Gly Asp Glu Lys Thr Arg Asn
        595                 600                 605

Pro Thr Pro Ala Val Thr Pro Gln Pro Arg Gly Ala Glu Phe His Met
    610                 615                 620

Trp Asn Tyr His Ser His Val Phe Ser Val Gly Asp Thr Phe Ser Leu
625                 630                 635                 640

Ala Met His Leu Gln Tyr Lys Ile His Glu Ala Pro Phe Asp Leu Leu
                645                 650                 655

Leu Glu Trp Leu Tyr Val Pro Ile Asp Pro Thr Cys Gln Pro Met Arg
            660                 665                 670

Leu Tyr Ser Thr Cys Leu Tyr His Pro Asn Ala Pro Gln Cys Leu Ser
        675                 680                 685

His Met Asn Ser Gly Cys Thr Phe Thr Ser Pro His Leu Ala Gln Arg
    690                 695                 700

Val Ala Ser Thr Val Tyr Gln Asn Cys Glu His Ala Asp Asn Tyr Thr
705                 710                 715                 720

Ala Tyr Cys Leu Gly Ile Ser His Met Glu Pro Ser Phe Gly Leu Ile
                725                 730                 735

Leu His Asp Gly Gly Thr Thr Leu Lys Phe Val Asp Thr Pro Glu Ser
            740                 745                 750

Leu Ser Gly Leu Tyr Val Phe Val Val Tyr Phe Asn Gly His Val Glu
        755                 760                 765

Ala Val Ala Tyr Thr Val Val Ser Thr Val Asp His Phe Val Asn Ala
    770                 775                 780

Ile Glu Glu Arg Gly Phe Pro Pro Thr Ala Gly Gln Pro Pro Ala Thr
785                 790                 795                 800

Thr Lys Pro Lys Glu Ile Thr Pro Val Asn Pro Gly Thr Ser Pro Leu
                805                 810                 815

Ile Arg Leu Glu His His His His His His
            820                 825

<210>  38
<211>  827
<212>  PRT
<213>  Artificial Sequence

<220>
<221>  UNSURE
<222>  (1)..(827)
<223>  unknown

<400>  38

Met Gly Ser Val Leu Arg Tyr Asp Asp Phe His Ile Asp Glu Asp Lys
1               5                   10                  15

Leu Asp Thr Asn Ser Val Tyr Glu Pro Tyr Tyr His Ser Asp His Ala
            20                  25                  30

Glu Ser Ser Trp Val Asn Arg Gly Glu Ser Ser Arg Lys Ala Tyr Asp
        35                  40                  45

His Asn Ser Pro Tyr Ile Trp Pro Arg Asn Asp Tyr Asp Gly Phe Leu
    50                  55                  60

Glu Asn Ala His Glu His His Gly Val Tyr Asn Gln Gly Arg Gly Ile
65                  70                  75                  80

Asp Ser Gly Glu Arg Leu Met Gln Pro Thr Gln Met Ser Ala Gln Glu
                85                  90                  95

Asp Leu Gly Asp Asp Thr Gly Ile His Val Ile Pro Thr Leu Asn Gly
            100                 105                 110

Asp Asp Arg His Lys Ile Val Asn Val Asp Gln Arg Gln Tyr Gly Asp
        115                 120                 125

Val Phe Lys Gly Asp Leu Asn Pro Lys Pro Gln Gly Gln Arg Leu Ile
    130                 135                 140

Glu Val Ser Val Glu Glu Asn His Pro Phe Thr Leu Arg Ala Pro Ile
145                 150                 155                 160

Gln Arg Ile Tyr Gly Val Arg Tyr Thr Glu Thr Trp Ser Phe Leu Pro
                165                 170                 175

Ser Leu Thr Cys Thr Gly Asp Ala Ala Pro Ala Ile Gln His Ile Cys
            180                 185                 190

Leu Lys His Thr Thr Cys Phe Gln Asp Val Val Val Asp Val Asp Cys
        195                 200                 205

Ala Glu Asn Thr Lys Glu Asp Gln Leu Ala Glu Ile Ser Tyr Arg Phe
    210                 215                 220

Gln Gly Lys Lys Glu Ala Asp Gln Pro Trp Ile Val Val Asn Thr Ser
225                 230                 235                 240

Thr Leu Phe Asp Glu Leu Glu Leu Asp Pro Pro Glu Ile Glu Pro Gly
                245                 250                 255

Val Leu Lys Val Leu Arg Thr Glu Lys Gln Tyr Leu Gly Val Tyr Ile
            260                 265                 270

Trp Asn Met Arg Gly Ser Asp Gly Thr Ser Thr Tyr Ala Thr Phe Leu
        275                 280                 285

Val Thr Trp Lys Gly Asp Glu Lys Thr Arg Asn Pro Thr Pro Ala Val
    290                 295                 300

Thr Pro Gln Pro Arg Gly Ala Glu Phe His Met Trp Asn Tyr His Ser
305                 310                 315                 320

His Val Phe Ser Val Gly Asp Thr Phe Ser Leu Ala Met His Leu Gln
                325                 330                 335

Tyr Lys Ile His Glu Ala Pro Phe Asp Leu Leu Leu Glu Trp Leu Tyr
            340                 345                 350

Val Pro Ile Asp Pro Thr Cys Gln Pro Met Arg Leu Tyr Ser Thr Cys
        355                 360                 365

Leu Tyr His Pro Asn Ala Pro Gln Cys Leu Ser His Met Asn Ser Gly
    370                 375                 380

Cys Thr Phe Thr Ser Pro His Leu Ala Gln Arg Val Ala Ser Thr Val
385                 390                 395                 400

Tyr Gln Asn Cys Glu His Ala Asp Asn Tyr Thr Ala Tyr Cys Leu Gly

                405                 410                 415

Ile Ser His Met Glu Pro Ser Phe Gly Leu Ile Leu His Asp Gly Gly
            420                 425                 430

Thr Thr Leu Lys Phe Val Asp Thr Pro Glu Ser Leu Ser Gly Leu Tyr
        435                 440                 445

Val Phe Val Val Tyr Phe Asn Gly His Val Glu Ala Val Ala Tyr Thr
    450                 455                 460

Val Val Ser Thr Val Asp His Phe Val Asn Ala Ile Glu Glu Arg Gly
465                 470                 475                 480

Phe Pro Pro Thr Ala Gly Gln Pro Pro Ala Thr Thr Lys Pro Lys Glu
                485                 490                 495

Ile Thr Pro Val Asn Pro Gly Thr Ser Pro Leu Ile Arg Gly Gly Gly
            500                 505                 510

Gly Ser Gly Gly Gly Gly Ser Gly Gly Gly Gly Ser Ala Gln Val Ile
        515                 520                 525

Asn Thr Asn Ser Leu Ser Leu Leu Thr Gln Asn Asn Leu Asn Lys Ser
    530                 535                 540

Gln Ser Ala Leu Gly Thr Ala Ile Glu Arg Leu Ser Ser Gly Leu Arg
545                 550                 555                 560

Ile Asn Ser Ala Lys Asp Asp Ala Ala Gly Gln Ala Ile Ala Asn Arg
                565                 570                 575

Phe Thr Ala Asn Ile Lys Gly Leu Thr Gln Ala Ser Arg Asn Ala Asn
            580                 585                 590

Asp Gly Ile Ser Ile Ala Gln Thr Thr Glu Gly Ala Leu Asn Glu Ile
        595                 600                 605

Asn Asn Asn Leu Gln Arg Val Arg Glu Leu Ala Val Gln Ser Ala Asn
    610                 615                 620

Ser Thr Asn Ser Gln Ser Asp Leu Asp Ser Ile Gln Ala Glu Ile Thr
625                 630                 635                 640

Gln Arg Leu Asn Glu Ile Asp Arg Val Ser Gly Gln Thr Gln Phe Asn
                645                 650                 655

Gly Val Lys Val Leu Ala Gln Asp Asn Thr Leu Thr Ile Gln Val Gly
            660                 665                 670

Ala Asn Asp Gly Glu Thr Ile Asp Ile Asp Leu Lys Gln Ile Asn Ser
        675                 680                 685

Gln Thr Leu Gly Leu Asp Thr Leu Asn Val Gln Ser Pro Gly Ile Ser
    690                 695                 700

Gly Gly Gly Gly Gly Ile Leu Asp Ser Met Gly Lys Ala Gln Pro Asp
705                 710                 715                 720

Leu Ala Glu Ala Ala Ala Thr Thr Thr Glu Asn Pro Leu Gln Lys Ile
                725                 730                 735

Asp Ala Ala Leu Ala Gln Val Asp Thr Leu Arg Ser Asp Leu Gly Ala
            740                 745                 750

Val Gln Asn Arg Phe Asn Ser Ala Ile Thr Asn Leu Gly Asn Thr Val
        755                 760                 765

Asn Asn Leu Thr Ser Ala Arg Ser Arg Ile Glu Asp Ser Asp Tyr Ala
    770                 775                 780

Thr Glu Val Ser Asn Met Ser Arg Ala Gln Ile Leu Gln Gln Ala Gly
785                 790                 795                 800

Thr Ser Val Leu Ala Gln Ala Asn Gln Val Pro Gln Asn Val Leu Ser
                805                 810                 815

Leu Leu Arg Leu Glu His His His His His His
            820                 825

<210>  39
<211>  825
<212>  PRT
<213>  Artificial Sequence

<220>
<221>  UNSURE
<222>  (1)..(825)
<223>  unknown

<400>  39

Met Ala Gln Val Ile Asn Thr Asn Ser Leu Ser Leu Leu Thr Gln Asn
1               5                   10                  15

Asn Leu Asn Lys Ser Gln Ser Ala Leu Gly Thr Ala Ile Glu Arg Leu
            20                  25                  30

Ser Ser Gly Leu Arg Ile Asn Ser Ala Lys Asp Asp Ala Ala Gly Gln
        35                  40                  45

Ala Ile Ala Asn Arg Phe Thr Ala Asn Ile Lys Gly Leu Thr Gln Ala
    50                  55                  60

Ser Arg Asn Ala Asn Asp Gly Ile Ser Ile Ala Gln Thr Thr Glu Gly
65                  70                  75                  80

Ala Leu Asn Glu Ile Asn Asn Asn Leu Gln Arg Val Arg Glu Leu Ala
                85                  90                  95

Val Gln Ser Ala Asn Ser Thr Asn Ser Gln Ser Asp Leu Asp Ser Ile
            100                 105                 110

Gln Ala Glu Ile Thr Gln Arg Leu Asn Glu Ile Asp Arg Val Ser Gly
        115                 120                 125

Gln Thr Gln Phe Asn Gly Val Lys Val Leu Ala Gln Asp Asn Thr Leu
    130                 135                 140

Thr Ile Gln Val Gly Ala Asn Asp Gly Glu Thr Ile Asp Ile Asp Leu
145                 150                 155                 160

Lys Gln Ile Asn Ser Gln Thr Leu Gly Leu Asp Thr Leu Asn Val Gln
                165                 170                 175

Gly Gly Gly Gly Ser Gly Gly Gly Gly Ser Gly Gly Gly Gly Ser Ser
            180                 185                 190

Val Leu Arg Tyr Asp Asp Phe His Ile Asp Glu Asp Lys Leu Asp Thr
        195                 200                 205

Asn Ser Val Tyr Glu Pro Tyr Tyr His Ser Asp His Ala Glu Ser Ser
    210                 215                 220

Trp Val Asn Arg Gly Glu Ser Ser Arg Lys Ala Tyr Asp His Asn Ser
225                 230                 235                 240

Pro Tyr Ile Trp Pro Arg Asn Asp Tyr Asp Gly Phe Leu Glu Asn Ala
                245                 250                 255

His Glu His His Gly Val Tyr Asn Gln Gly Arg Gly Ile Asp Ser Gly
            260                 265                 270

Glu Arg Leu Met Gln Pro Thr Gln Met Ser Ala Gln Glu Asp Leu Gly
        275                 280                 285

Asp Asp Thr Gly Ile His Val Ile Pro Thr Leu Asn Gly Asp Asp Arg
    290                 295                 300

His Lys Ile Val Asn Val Asp Gln Arg Gln Tyr Gly Asp Val Phe Lys
305                 310                 315                 320

Gly Asp Leu Asn Pro Lys Pro Gln Gly Gln Arg Leu Ile Glu Val Ser
                325                 330                 335

Val Glu Glu Asn His Pro Phe Thr Leu Arg Ala Pro Ile Gln Arg Ile
            340                 345                 350

Tyr Gly Val Arg Tyr Thr Glu Thr Trp Ser Phe Leu Pro Ser Leu Thr
        355                 360                 365

Cys Thr Gly Asp Ala Ala Pro Ala Ile Gln His Ile Cys Leu Lys His
    370                 375                 380

Thr Thr Cys Phe Gln Asp Val Val Val Asp Val Asp Cys Ala Glu Asn
385                 390                 395                 400

Thr Lys Glu Asp Gln Leu Ala Glu Ile Ser Tyr Arg Phe Gln Gly Lys
                405                 410                 415

Lys Glu Ala Asp Gln Pro Trp Ile Val Val Asn Thr Ser Thr Leu Phe
            420                 425                 430

Asp Glu Leu Glu Leu Asp Pro Pro Glu Ile Glu Pro Gly Val Leu Lys
        435                 440                 445

Val Leu Arg Thr Glu Lys Gln Tyr Leu Gly Val Tyr Ile Trp Asn Met
    450                 455                 460

Arg Gly Ser Asp Gly Thr Ser Thr Tyr Ala Thr Phe Leu Val Thr Trp
465                 470                 475                 480

Lys Gly Asp Glu Lys Thr Arg Asn Pro Thr Pro Ala Val Thr Pro Gln
                485                 490                 495

Pro Arg Gly Ala Glu Phe His Met Trp Asn Tyr His Ser His Val Phe
            500                 505                 510

Ser Val Gly Asp Thr Phe Ser Leu Ala Met His Leu Gln Tyr Lys Ile
        515                 520                 525

His Glu Ala Pro Phe Asp Leu Leu Leu Glu Trp Leu Tyr Val Pro Ile

530                 535                 540

Asp Pro Thr Cys Gln Pro Met Arg Leu Tyr Ser Thr Cys Leu Tyr His
545                 550                 555                 560

Pro Asn Ala Pro Gln Cys Leu Ser His Met Asn Ser Gly Cys Thr Phe
                565                 570                 575

Thr Ser Pro His Leu Ala Gln Arg Val Ala Ser Thr Val Tyr Gln Asn
            580                 585                 590

Cys Glu His Ala Asp Asn Tyr Thr Ala Tyr Cys Leu Gly Ile Ser His
        595                 600                 605

Met Glu Pro Ser Phe Gly Leu Ile Leu His Asp Gly Gly Thr Thr Leu
    610                 615                 620

Lys Phe Val Asp Thr Pro Glu Ser Leu Ser Gly Leu Tyr Val Phe Val
625                 630                 635                 640

Val Tyr Phe Asn Gly His Val Glu Ala Val Ala Tyr Thr Val Val Ser
                645                 650                 655

Thr Val Asp His Phe Val Asn Ala Ile Glu Glu Arg Gly Phe Pro Pro
            660                 665                 670

Thr Ala Gly Gln Pro Pro Ala Thr Thr Lys Pro Lys Glu Ile Thr Pro
        675                 680                 685

Val Asn Pro Gly Thr Ser Pro Leu Ile Arg Gly Gly Gly Gly Ser Gly
    690                 695                 700

Gly Gly Gly Ser Gly Gly Gly Gly Ser Lys Ala Gln Pro Asp Leu Ala
705                 710                 715                 720

Glu Ala Ala Ala Thr Thr Thr Glu Asn Pro Leu Gln Lys Ile Asp Ala
                725                 730                 735

Ala Leu Ala Gln Val Asp Thr Leu Arg Ser Asp Leu Gly Ala Val Gln
            740                 745                 750

Asn Arg Phe Asn Ser Ala Ile Thr Asn Leu Gly Asn Thr Val Asn Asn
        755                 760                 765

Leu Thr Ser Ala Arg Ser Arg Ile Glu Asp Ser Asp Tyr Ala Thr Glu
    770                 775                 780

Val Ser Asn Met Ser Arg Ala Gln Ile Leu Gln Gln Ala Gly Thr Ser
785                 790                 795                 800

Val Leu Ala Gln Ala Asn Gln Val Pro Gln Asn Val Leu Ser Leu Leu
                805                 810                 815

Arg Leu Glu His His His His His His
            820                 825

<210>  40
<211>  2481
<212>  DNA
<213>  Artificial Sequence

<220>
<221>  unsure
<222>  (1)..(2481)
<223>  unknown

<400>  40
atggcccagg tgattaacac caacagcctg tctctgctga cccagaacaa cctgaacaaa      60

tctcagtctg ctctgggcac cgcgatcgaa cgtctgagca gcggcctgcg tatcaactct     120

gcgaaagatg atgcggcggg tcaggcaatc gctaaccgtt tcaccgctaa cattaaaggc     180

ctgacccagg cgagccgtaa cgcaaacgat ggtatctcca tcgcgcaaac caccgaaggt     240

gcgctgaacg aaatcaacaa caacctgcag cgtgtgcgcg aactggcggt gcagtctgct     300

aacagcacca acagccagtc tgatctggat agcatccaag cagaaatcac ccagcgtctg     360

aatgaaattg atcgcgttag cggtcagacc cagttcaacg gtgtgaaagt tctggctcag     420

gataacaccc tgaccatcca ggttggcgct aacgatggtg agaccattga tatcgatctg     480

aaacagatca acagccagac cctgggtctg gataccctga acgttcagtc tccgggcatc     540

agcggtggtg gcggtggcat tctggacagc atgggtaaag ctcagcctga tctggctgaa     600

gctgcggcga ccaccaccga aaacccgctg cagaaaattg atgcggcgct ggcgcaggtt     660

gacacgctgc gtagcgatct gggcgcggtg cagaaccgtt ttaacagcgc tatcaccaac     720

ctgggtaaca ccgttaacaa cctgacctct gcacgttctc gcatcgaaga ttccgactat     780

gcgaccgaag tgtccaacat gagccgtgcg cagattctgc agcaggcggg cacgtctgtg     840

ctggcccagg caaaccaggt accgcagaac gttctgtctc tgctgcgtgg tggtggcggc     900

agcggcggcg gcggtagcgg cggcggcggc tccagcgttc tgcgttacga cgactttcat     960

atcgacgaag acaaactgga tactaacagc gtctacgaac cgtattatca ttccgaccac    1020

gccgaatctt cttgggtgaa ccgcggcgaa agcagccgta aagcgtatga tcataactct    1080

ccgtacatct ggccgcgtaa cgattatgac ggcttcctgg aaaacgcaca tgaacaccac    1140

ggcgtttaca accagggtcg tggtatcgac agcggtgaac gtctgatgca gccgacccag    1200

atgtccgctc aggaagactt gggtgatgac accggcattc acgtgatccc gaccctgaac 1260

ggtgacgatc gtcacaaaat cgttaacgtg gaccagcgcc agtacggcga cgtattcaaa 1320

ggtgatctga acccgaaacc gcagggtcag cgtctgatcg aagtgtccgt tgaagaaaac 1380

cacccgttca ccctgcgtgc accgattcag cgcatctatg gtgtgcgtta caccgaaacc 1440

tggtcgttcc tgccgtcgct gacctgcacg ggtgatgcgg ccccggccat ccagcacatt 1500

tgcctgaaac acaccacttg ctttcaggat gtggttgtgg acgttgactg tgctgaaaac 1560

accaaagaag atcagctggc agaaattagc tatcgtttcc agggtaaaaa agaagcggac 1620

cagccgtgga tcgtggttaa cactagcact ctgttcgatg aactggaact ggaccctccg 1680

gaaatcgaac cgggtgttct gaaagtgctg cgtaccgaaa aacagtacct gggtgtttac 1740

atctggaaca tgcgtggctc cgacggtacc tctacctacg cgactttcct ggtcacctgg 1800

aaaggtgatg aaaaaacccg taacccgacc ccggcagtta ccccgcagcc gcgtggcgcg 1860

gaattccaca tgtggaacta tcactctcat gttttctctg ttggtgatac tttctccttg 1920

gctatgcacc tgcagtacaa aatccatgaa gcgccgttcg atctgctgct ggaatggctg 1980

tatgttccga tcgatccgac ctgtcagccg atgcgtctgt atagcacctg tctgtaccac 2040

ccgaacgcgc cgcagtgtct gtcccacatg aactccggtt gcaccttcac tagcccgcac 2100

ctggcacagc gtgtggcttc caccgtttat cagaactgcg aacatgcgga caattatacc 2160

gcttactgcc tgggcatctc tcacatggaa ccgtctttcg gtctgatcct gcacgatggc 2220

ggcaccaccc tgaaattcgt agataccccg gaaagcctgt ctggtctgta cgtgttcgtt 2280

gtttacttca atggccatgt tgaagcggtt gcgtacaccg ttgttagcac cgttgatcac 2340

ttcgttaacg cgattgaaga acgtggcttc ccgccgaccg cgggccagcc gccggctacc 2400

actaaaccga aagaaatcac cccggttaat ccgggcacct ctccgctgat ccgtctcgag 2460

caccaccacc accaccacta a 2481

<210> 41
<211> 2499
<212> DNA
<213> Artificial Sequence

<220>
<221> unsure
<222> (1)..(2499)
<223> unknown

<400> 41
atggcccagg tgattaacac caacagcctg tctctgctga cccagaacaa cctgaacaaa 60

tctcagtctg ctctgggcac cgcgatcgaa cgtctgagca gcggcctgcg tatcaactct 120

gcgaaagatg atgcggcggg tcaggcaatc gctaaccgtt tcaccgctaa cattaaaggc 180

ctgacccagg cgagccgtaa cgcaaacgat ggtatctcca tcgcgcaaac caccgaaggt 240

gcgctgaacg aaatcaacaa caacctgcag cgtgtgcgcg aactggcggt gcagtctgct 300

aacagcacca acagccagtc tgatctggat agcatccaag cagaaatcac ccagcgtctg 360

aatgaaattg atcgcgttag cggtcagacc cagttcaacg gtgtgaaagt tctggctcag 420

gataacaccc tgaccatcca ggttggcgct aacgatggtg agaccattga tatcgatctg 480

aaacagatca acagccagac cctgggtctg gataccctga acgttcagtc tccgggcatc 540

agcggtggtg gcggtggcat tctggacagc atgggtaaag ctcagcctga tctggctgaa 600

gctgcggcga ccaccaccga aaacccgctg cagaaaattg atgcggcgct ggcgcaggtt 660

gacacgctgc gtagcgatct gggcgcggtg cagaaccgtt ttaacagcgc tatcaccaac 720

ctgggtaaca ccgttaacaa cctgacctct gcacgttctc gcatcgaaga ttccgactat 780

gcgaccgaag tgtccaacat gagccgtgcg cagattctgc agcaggcggg cacgtctgtg 840

ctggcccagg caaaccaggt accgcagaac gttctgtctc tgctgcgtgg tggtggcggc 900

agcggcggcg gcggtagcgg cggcggcggc tccagcgttc tgcgttacga cgactttcat 960

atcgacgaag acaaactgga tactaacagc gtctacgaac cgtattatca ttccgaccac 1020

gccgaatctt cttgggtgaa ccgcggcgaa agcagccgta aagcgtatga tcataactct 1080

ccgtacatct ggccgcgtaa cgattatgac ggcttcctgg aaaacgcaca tgaacaccac 1140

ggcgtttaca accagggtcg tggtatcgac agcggtgaac gtctgatgca gccgacccag 1200

atgtccgctc aggaagactt gggtgatgac accggcattc acgtgatccc gaccctgaac 1260

ggtgacgatc gtcacaaaat cgttaacgtg gaccagcgcc agtacggcga cgtattcaaa 1320

ggtgatctga acccgaaacc gcagggtcag cgtctgatcg aagtgtccgt tgaagaaaac 1380

cacccgttca ccctgcgtgc accgattcag cgcatctatg gtgtgcgtta caccgaaacc 1440

tggtcgttcc tgccgtcgct gacctgcacg ggtgatgcgg ccccggccat ccagcacatt 1500

tgcctgaaac acaccacttg ctttcaggat gtggttgtgg acgttgactg tgctgaaaac 1560

accaaagaag atcagctggc agaaattagc tatcgtttcc agggtaaaaa agaagcggac 1620

cagccgtgga tcgtggttaa cactagcact ctgttcgatg aactggaact ggaccctccg 1680

gaaatcgaac cgggtgttct gaaagtgctg cgtaccgaaa aacagtacct gggtgtttac 1740

atctggaaca tgcgtggctc cgacggtacc tctacctacg cgactttcct ggtcacctgg 1800

aaaggtgatg aaaaaacccg taacccgacc ccggcagtta ccccgcagcc gcgtggcgcg 1860

gaattccaca tgtggaacta tcactctcat gttttctctg ttggtgatac tttctccttg 1920

gctatgcacc tgcagtacaa aatccatgaa gcgccgttcg atctgctgct ggaatggctg 1980

tatgttccga tcgatccgac ctgtcagccg atgcgtctgt atagcacctg tctgtaccac 2040

ccgaacgcgc cgcagtgtct gtcccacatg aactccggtt gcaccttcac tagcccgcac 2100

ctggcacagc gtgtggcttc caccgtttat cagaactgcg aacatgcgga caattatacc 2160

gcttactgcc tgggcatctc tcacatggaa ccgtctttcg gtctgatcct gcacgatggc 2220

ggcaccaccc tgaaattcgt agataccccg gaaagcctgt ctggtctgta cgtgttcgtt 2280

gtttacttca atggccatgt tgaagcggtt gcgtacaccg ttgttagcac cgttgatcac 2340

ttcgttaacg cgattgaaga acgtggcttc ccgccgaccg cgggccagcc gccggctacc 2400

actaaaccga aagaaatcac cccggttaat ccgggcacct ctccgctgat ccgtctcgag 2460

caccaccacc accaccacta acaccaccac caccactaa 2499

<210> 42
<211> 2460
<212> DNA
<213> Artificial Sequence

<220>
<221> unsure
<222> (1)..(2460)
<223> unknown

<400> 42
atggcgcagg ttatcaacac taacagcctg agcctgctga cccagaacaa cctgaacaaa 60

agccagagcg cgctgggcac ggcgatcgaa cgtctgtctt ccggcctgcg catcaacagc 120

gcgaaagatg atgcggcagg tcaggcgatt gctaaccgct tcaccgctaa tatcaaaggc 180

ctgactcagg cttcccgcaa tgcgaacgac ggtatcagca tcgcacagac caccgaaggc 240

gcgctgaacg aaatcaacaa caacctgcag cgtgtgcgtg aactggcggt gcagtctgct 300

aactcgacga acagccagtc cgacctggat tccatccagg cggaaatcac ccagcgtctg 360

aacgaaatcg atcgcgttag cggtcagacc cagttcaacg gtgttaaagt tctggcgcag 420

gataataccc tgaccattca ggtcggcgct aacgacggtg aaaccattga catcgatctg 480

aaacagatta acagccagac cctgggtctg gataccctga acgttcaggg cggtggtggc 540

tctggcggcg gtggttccgg cggtggtggt tcctctgtcc tgcgttatga tgatttccac 600

atcgatgaag acaaactgga cacgaatagc gtttacgaac cgtattatca cagcgatcat 660

gctgaaagct cttgggttaa ccgtggtgaa tcttcccgta aagcgtacga tcacaactcc 720

ccgtacattt ggccgcgtaa cgactacgat ggcttcctgg aaaacgcaca cgaacaccac 780

ggcgtgtaca accagggccg tggcattgac agcggtgaac gcctgatgca gccgacccag 840

atgagcgcgc aagaagacct gggtgatgac accggtattc acgtgatccc gaccctgaac 900

ggcgatgatc gccacaaaat tgtaaacgtc gatcagcgtc agtatggcga tgtgtttaaa 960

ggtgatctga acccgaaacc gcaaggccag cgcctgatcg aggtgagcgt tgaagaaaac 1020

cacccgttca ccctgcgcgc tccgatccag cgcatttacg gtgtccgtta taccgaaact 1080

tggtcctttc tgccgtcgct gacctgcacc ggtgatgcgg ctccggctat ccagcacatc 1140

tgcctgaaac acacgacctg tttccaggat gtagttgttg acgtggactg tgcggaaaac 1200

actaaagaag accagctggc ggaaatcagc taccgtttcc agggcaaaaa ggaagcggac 1260

cagccgtgga tcgttgtaaa cacctccacc ctgttcgacg aactggaact ggatccgccg 1320

gaaattgaac cgggtgtgct gaaagttctg cgtaccgaaa aacagtacct gggcgtttac 1380

atttggaaca tgcgcggttc tgatggtacc tccacctacg caactttcct ggttacctgg 1440

aaaggcgatg aaaaaacccg taacccgacc ccggcggtta ccccgcagcc gcgtggtgct 1500

gaattccaca tgtggaatta tcactctcac gtgtttagcg tcggtgatac ctttagcctg 1560

gcaatgcacc tgcagtacaa aatccatgaa gcgccgttcg atctgctgct ggaatggctg 1620

tatgtgccga ttgatccgac ctgtcagccg atgcgtctgt actctacttg cctgtatcac 1680

ccaaacgctc cgcagtgcct gtcccacatg aactccggct gtaccttcac tagcccgcac 1740

ctggcgcagc gtgttgcgtc caccgtttat cagaactgcg aacacgcgga taactacacc 1800

gcttattgcc tgggtatctc tcacatggaa ccatctttcg gtctgatcct gcatgacggc 1860

ggcaccaccc tcaaatttgt tgacaccccg gaatctctgt ctggcctgta cgtttttgtg 1920

gtttatttca acggtcacgt agaagcggtc gcgtataccg ttgttagcac cgttgaccac 1980

ttcgttaatg cgatcgaaga acgtggcttc ccgccgaccg cgggccagcc gccggcgacc 2040

accaaaccga aagaaattac tccggtaaat ccgggtacct ctccgctgat ccgtggcggc 2100

ggtggcagcg gcggtggcgg ctccggtggc ggtggtagca aagcgcagcc ggatctggcg 2160

gaagcagccg caactactac cgaaaacccg ctgcagaaaa tcgacgcagc cctggcgcag 2220

gtagataccc tgcgttctga cctgggcgca gttcagaacc gttttaactc cgcaatcacc 2280

aacttgggta acacggttaa caacctgacc tctgcacgca gccgtattga agatagcgac 2340

tatgcgaccg aagtttctaa catgagccgt gcgcagatcc tgcagcaggc aggtacctct 2400

gttctggcgc aggcgaacca ggttccgcag aacgtgctga gcctgctgcg tctcgagcac 2460

<210> 43
<211> 30
<212> PRT
<213> Artificial sequence

<220>
<223> unknown

<400> 43

Leu Asn Lys Ser Gln Ser Ala Leu Gly Thr Ala Ile Glu Arg Leu Ser
1               5                   10                  15

Ser Gly Leu Arg Ile Asn Ser Ala Lys Asp Asp Ala Ala Cys
            20                  25                  30

<210>  44
<211>  19
<212>  PRT
<213>  Artificial sequence

<220>
<223>  unknown

<400>  44

Asn Asn Leu Gln Arg Val Arg Glu Leu Ala Val Gln Ser Ala Asn Ser
1               5                   10                  15


Thr Asn Cys




<210>  45
<211>  20
<212>  PRT
<213>  Artificial sequence

<220>
<223>  unknown

<400>  45

Leu Thr Ser Ala Arg Ser Arg Ile Glu Asp Ser Asp Tyr Ala Thr Glu
1               5                   10                  15


Val Ser Asn Met
            20


<210>  46
<211>  55
<212>  DNA
<213>  Artificial sequence

<220>
<223>  unknown

<400>  46
ggggacaagt ttgtacaaaa aagcaggctt cgccgccgcc atgggaaaac ggtcc          55


<210>  47
<211>  55
<212>  DNA
<213>  Artificial sequence

<220>
<223>  unknown

<400>  47
ggggaccact ttgtacaaga aagctgggtc agacatgata agatacattg atgag          55


<210>  48

<211>  56
<212>  DNA
<213>  Artificial sequence

<220>
<223>  unknown

<400>  48
ggggaccact ttgtacaaga aagctgggtc ttattattat ctgatcaggg ggctag          56


<210>  49
<211>  54
<212>  DNA
<213>  Artificial sequence

<220>
<223>  unknown

<400>  49
ggggaccact ttgtacaaga aagctgggtc ttattattac ctcagcaggc tcag          54


<210>  50
<211>  56
<212>  DNA
<213>  Artificial sequence

<220>
<223>  unknown

<400>  50
ggggaccact ttgtacaaga aagctgggtc ttattattat ctaatcagag ggctag          56

## Description

### TECHNICAL FIELD

The present invention relates to a recombinant VZV gE glycoprotein, a fusion protein having self-adjuvant function, a recombinant vector, an immune composition and a preparation method therefor, and use thereof.

### BACKGROUND

Varicella-zoster virus (VZV), a member of the sub-family of herpesviridae alpha herpesvirus, is a double-stranded DNA virus with a diameter of 150-200 nm. Morphologically, it has a concentric circular structure consisting of a nucleic acid core, a protein capsid and an envelope, and has a symmetrical regular icosahedron surface consisting of 162 capsomers. VZV is a global pathogen with cutaneous and neurotropic features. Primary infection in children can lead to chickenpox. Chickenpox is a highly contagious disease transmitted by skin contact or respiratory droplets and is characterized in the presence of disseminated vesicular skin rashes on the face and body, accompanied by itching and fever. Visceral complications are occasionally incidental in VZV primary infection, such as possibly life-threatening complications like encephalitis, hepatitis, pancreatitis, or pneumonia, especially in unvaccinated young children and adults, as well as immunosuppressed populations. After primary infection, VZV can remain latent for life in the cranial nerves and dorsal root ganglia. After several decades, VZV may still be reactivated, and causes painful shingles or other serious neurological or ocular complications, as well as exhausting postherpetic neuralgia, the most common chronic complication of shingles. More than 95% of people aged 50 or more with normal immunity are VZV antibody-positive in serum and are therefore at risk of developing shingles. The risk of life-long onset of shingles is between 25% and 30%, with the risk rising to 50% after the age of 80 (Johnson RW et al., Ther Adv Vaccines, 3:109-120, 2015). In immunocompromised individuals, both incidence and mortality of VZV infection are high. For example, in organ transplant patients, chickenpox can be fatal, with severe disseminated skin damage, viscera damage, and intravascular coagulation. Shingles is also a common complication in organ transplant patients. The disease caused by VZV and its associated sequela (such as postherpetic neuralgia) have become increasingly a significant disease burden and an important public health concern, and there's an urgent need of more attention from the medical field.

For chickenpox and shingles, symptomatic treatment is mainly adopted, and no treatment method of special effect is available. Antiviral drugs such as acyclovir, valaciclovir and famciclovir are helpful for the recovery of chickenpox and HZ patients, but cannot prevent VZV infection. Administration of virus-specific immunoglobulins after the exposure to VZV also has limited effect in halting or reducing disease burden. It is found that anti-VZV membrane antigen antibody titers ≧ 1/64 are considered to be associated with protection from disease, and antibodies against the gE glycoprotein are also found to be associated with long-term protection. Although infection can produce life-long immunity to the virus, intact cellular immunity is critical for recovery from infection and recurrent diseases, as shingles occurs when T cell immunity declines or is suppressed with age. The CD4+ T cell proliferation response plays an important role in shingles prevention and/or treatment, but a correlation between CD4+ T cell proliferation response and efficacy has not been established to date (Plotkin SA., Clin Vaccine Immunol, 17:1055-1065,2010).

In view of this, vaccination remains the most effective and reliable means for preventing and controlling chickenpox and shingles.

The live attenuated varicella vaccine was first developed by the Takahashi research group in 1974. They isolated a VZV strain from a chickenpox patient named Oka aged 3, and then attenuated it by serial passages in human embryonic fibroblasts, guinea pig fibroblasts and human diploid fibroblasts. This live attenuated vaccine is called vaccine Oka (vOka). Vaccine Oka is currently incorporated into the routine immunization program in many countries. Generally, vaccine Oka is safe. It does not cause serious adverse reactions even in partially immunocompromised children and HIV-infected children, and shows excellent immune protective effects. However, the persistence of immune protection induced by vaccine Oka is not long enough, and some individuals do not reach an effective protective state after continuous vaccination. Meanwhile, the immune effect of the vaccine Oka for teenagers is lower than that for children at the age of 1-12, and therefore preschool children need a second vaccination. At present, all chickenpox vaccines on the market are live attenuated vaccines. Although serious side effects are rare, these chickenpox vaccines are reported to cause serious rash, lung or liver infection, meningitis, convulsion, pneumonia or serious systemic infection of vaccine strains after vaccination, especially in immunocompromised children.

Currently, shingles vaccines include Zostavax of Merck and Shingrix of GSK. Zostavax is a concentrated version of vaccine Oka, which was approved by US FDA in 2006. The effectiveness of Zostavax decreases with age of the vaccinees, and thus is not recommended for people over the age of 60. It has now been shown to provide 50% protection in about five years, followed by a progressive decrease in efficacy during 5-8 years after vaccination, and its protection efficacy is no longer statistically significant after 8 years after vaccination (Morrison VA, et al., Clin Infect Dis, 60:900-909, 2015). Shingrix of GSK adopts gene recombination technology to express varicella zoster virus glycoprotein E (gE) in Chinese hamster ovary cells, and it was approved by the FDA in 2017 to be used in people aged 50 or more. Shingrix, with 90% of protection rate for shingles, reduces the risk of postherpetic neuralgia and is a preferred substitute of Zostavax. However, the adjuvant used in Shingrix is AS01 from GSK which has side effects.

The live attenuated varicella vaccine brings multiple risks to the vaccinees, including rare but very serious complications, infection of immunocompromised individuals and latent infection, and more importantly, 30% of vaccinees suffer from recurrence of shingles resulting from the reactivation of latent virus. With regard to shingles vaccine, the National Medical Products Administration of China approved the imported drug registration application of Shingrix in 2019, which fills the blank of shingles vaccine in China. However, Shingrix has side effects and is sold abroad at around $ 150/dose; it is currently only for people aged 50 or more, and can not be used as a chickenpox vaccine for children because the side effects are too strong. Therefore, there is a need for further development of modified VZV vaccines that feature in higher safety, lower side effects, no risk of latency and postherpetic neuralgia complications, and lower price. However, no progress has been made yet. The novel vaccine should be capable of both eliciting a strong humoral response to neutralize the virus and inducing broad cellular immunity to control the disease.

The open reading frame (ORF) of the VZV genome encodes 8 glycoproteins in total: gE, gB, gH, gI, gC, gL, gK, and gM. The gE glycoprotein, a type I membrane protein encoded by ORF68 gene, is a glycoprotein that is necessary for generating infectious viral particles and most abundant in virus envelope and most immunogenic. The gE glycoprotein exists on the surfaces of viral particles and in cytoplasm of VZV-infected cells, and is present in different glycosylation forms at different mature stages of the virus. In serums of chickenpox and shingles patients in convalescence, VZV antibodies are mainly against gE, gB and gH. Specific anti-gE monoclonal antibodies can neutralize VZV and mediate antibody dependent cellular cytotoxicity (ADCC). gE is also a major target for cellular immunity, which can control the disease and destroy virus-infected cells. These properties make gE an ideal immunogen for the development of safe, effective broad-spectrum vaccines.

Inactive human vaccines typically consist of one or more immunogens, and an immune adjuvant is added to enhance the efficacy. Currently, only a limited number of immune adjuvants are available for human use, such as aluminium salts, mineral oils, plants or bacterial extracts. Immune adjuvants have different enhancing properties and may cause various adverse side effects. With the continuous understanding of the regulatory mechanism for immune response, Toll-like receptors (TLRs) that are expressed on the surface of sentinel cells of the immune system (e.g., dendritic cells and macrophages) and on lymphocytes and that co-regulate innate and adaptive immunity have been discovered. TLRs recognize a conserved microbial-associated molecular pattern (MAMP). Agonists trigger TLRs to produce a variety of pleiotropic immune mediators, such as cytokines and chemokines, that participate in the proinflammatory response and stimulate innate immunity, and thus act as immune adjuvants.

Toll-like receptor 5 (TLR-5) is a transmembrane receptor that specifically recognizes bacterial flagellin proteins. Flagellin protein is the major structural protein of flagella of Gram-negative bacteria. Flagellin induces and activates TLR5, initiates innate immunity, induces activation of monocyte-macrophages and epithelial cells, and releases proinflammatory cytokines such as IL-1, IL-8 and TNF-α. Thus, flagellin protein is a potent systemic and mucosal immune adjuvant. It consists of four domains D0, D1, D2 and D3, with domains D0 and D1 being highly conserved for proteobacteria. The interaction between the helix of the N-terminal D0-D1 region of the flagellin protein and the helix of the C-terminal D1-D0 region of the flagellin protein forms a stem-like core structure that is critical for the binding and activation of TLR-5. In contrast, domains D2 and D3 of the flagellin differ widely among different bacteria, and they have strong immunogenicity but are not functionally necessary. Deletion of domains D2-D3 does not impair TLR-5 activation and can minimize useless anti-flagellin protein antibody responses. Studies have shown that flagellin must bind to the target immunogen to produce optimal immune adjuvant effect. This can be achieved by constructing a self-adjuvanted fusion protein, wherein the immunogen is covalently linked to flagellin or a functional fragment thereof that retains TLR-5 binding activity, thereby retaining the innate immunostimulating properties of the flagellin.

### SUMMARY

In order to overcome the above-mentioned drawbacks of the marketed vaccines and reduce the adverse reactions, the present invention develops a novel immune composition by two different methods. One method is to produce a recombinant gE protein or gE-based fusion immunogen which is capable of inducing strong neutralizing antibodies and CD4+ T cell responses and has reduced side effects; the other method is to construct a safer replication-defective adenovirus vector to express the gE gene or gE-flagellin fusion protein gene so as to elicit neutralizing antibody responses and broader CD4+ T cell and CD8+ T cell immunity; and thus a new glycoprotein, a fusion protein, a recombinant vector, a preparation method and a composition are obtained, and can be applied to the preparation of a new vaccine against VZV infection.

The present invention provides an immune composition comprising a varicella zoster virus glycoprotein E (gE)-based antigen, which can be used for preventing or treating Varicella Zoster Virus (VZV) infection.

In some embodiments, the gE-based immunogen comprises at least: (i) a gE extracellular region or a fragment thereof, or a corresponding nucleic acid molecule encoding the same; (ii) a gE-based fusion protein, or a nucleic acid molecule encoding the same; (iii) a gE-based recombinant vector; or (iv) a combination of two or more of the above.

Further, the gE-based fusion protein comprises at least: a gE extracellular region or a fragment thereof that is covalently coupled with a self-adjuvanted bacterial flagellin protein or a fragment thereof, wherein the bacterial flagellin protein or a fragment thereof has TLR-5 agonistic activity.

The amino acid or nucleic acid sequences of VZV gE and flagellin can be found in publicly available databases such as GenBank (GB), SwissPro (sp) and EMBL, representative database entries for gE include, but are not limited to: GB AQT34120.1, AAG32558.1 and ABE03086.1, and the sequences represented by the deposit numbers are incorporated herein by reference.

The gE glycoprotein is a membrane protein comprising a signal peptide, an extracellular region, a transmembrane region and an intracellular region. The extracellular region is exposed on the surface of the bacteria and is the target recognized by the immune system. It is therefore understood that the gE referred to in the present invention comprises at least the extracellular region or a fragment thereof, and, if desired, may further comprises other structural fragments such as transmembrane and/or intracellular regions, while retaining certain antigenic activity. Based on common knowledge, those skilled in the art can determine the respective structural fragments of gE, and a fragment of the gE extracellular region can be understood as a fragment which retains certain auto-immunogenicity of gE.

It will be appreciated that certain minor modifications may be made to gE while retaining certain immunological activities, including but not limited to: mutations, substitutions (e.g., conservative substitutions of functionally similar amino acids), additions, deletions, or truncations, and the like, and these modifications are also considered to be within the scope of the present disclosure.

In some embodiments, the gE extracellular region has at least 90%, 91%, 92%, 93%, 94%, 95%, 96%, 97%, 98%, 99% or 100% homology to the amino acid sequence as set forth in SEQ ID NO: 1.

Bacterial flagellin protein is the major flagellin component of many Gram-negative bacteria (e.g., E. *coli,* salmonella, etc.), and its primary amino acid sequence varies in composition and size depending on the bacterial species. In different bacteria, the interaction between the conserved N-terminal D0-D1 region and the C-terminal D1-D0 region forms a functional stem-like structure that is essential for TLR-5 binding and signaling. The intermediate "hypervariable" D2 and D3 are domains that are not essential for TLR5 signal transduction. The "hypervariable" D2 and D3 domains in the molecule are not required for TLR-5 signal transduction, they are highly immunogenic and induce adverse effects. Therefore, these regions can be deleted without affecting TLR-5 binding activity. The bacterial flagellin protein of the present invention may be an original or modified flagellin protein. Such modifications include, but are not limited to, mutations, substitutions (e.g., conservative substitutions of functionally similar amino acids), additions, deletions, or truncations, and the like, but certain degree of TLR-5 binding capacity should be retained o activate innate immunity. It should be understood that the bacterial flagellin protein or a fragment thereof of the present invention should not cause significant proinflammatory side effects. In fact, the immunity of the flagellin protein and certain modifications thereof are described in patents US2011110962A1 and/or US2011230643A1, which are incorporated into the present invention. In the present invention, unless otherwise specified, the N-terminal of the flagellin protein refers to the N-terminal D0-D1 region, and the C-terminal of the flagellin protein refers to the C-terminal D1-D0 region.

In some embodiments, the gE-based fusion protein comprises at least: an N-terminal region of the flagellin protein, a C-terminal region of the flagellin protein, and a gE extracellular region or a fragment thereof. In other words, the gE-based fusion protein may further comprise the flagellin protein or other fragments of gE.

In some specific embodiments, the gE extracellular region or a fragment thereof is located at the N-terminal or C-terminal of the gE-based fusion protein, or is inserted between the N-terminal and C-terminal of the flagellin protein.

As a preferred embodiment, the gE-based fusion protein is selected from any one of the following fusion forms:
fusion form 1: N-terminal region of the flagellin protein - C-terminal region of the flagellin protein - gE extracellular region or a fragment thereof;
fusion form 2: gE extracellular region or a fragment thereof - N-terminal region of the flagellin protein - C-terminal region of the flagellin protein;
fusion form 3: N-terminal region of the flagellin protein - gE extracellular region or a fragment thereof - C-terminal region of the flagellin protein;
wherein, the N-terminal region or the C-terminal region of the flagellin protein may be linked to the gE extracellular region or a fragment thereof either directly or via a linker;

The N-terminal region of the flagellin protein may be linked to the C-terminal region of the flagellin protein either directly or via a linker.

Such linkers include genetically engineered peptide chains (e.g., 1-20 amino acids linked via peptide bonds) and non-peptide chemical linkers (e.g., alkyl linkers or polyethylene glycol groups, wherein the alkyl linkers may also be substituted with non-sterically hindering groups such as halogen, CN and NH₂). It should be understood that the selected linker does not interfere with the biological activity of the fusion protein.

Preferably, the linker has 1-20 amino acids linked via peptide bonds, such as linker I or linker II; linker I has the sequence as set forth in SEQ ID NO: 4; and linker II has the sequence as set forth in SEQ ID NO: 7.
SEQ ID NO: 4: SPGISGGGGGILDSMG
SEQ ID NO: 7: GGGGSGGGGSGGGGS

In some specific embodiments, the N-terminal region or the C-terminal region of the flagellin protein is linked to the gE extracellular region or a fragment thereof via linker II.

In some specific embodiments, the N-terminal region of the flagellin protein is linked to the C-terminal region of the flagellin protein via linker I.

In some embodiments, the flagellin protein is from a salmonella, such as *Salmonella enterica subsp. enterica serovar typhimurium* (*S. typhimurium*) or *Salmonella enterica subsp. enterica serovar typhi (S. typhi*), the *S. typhimurium* includes but is not limited to strain LT2, and the *S*. *typhi* includes but is not limited to strain Ty2.

In some specific embodiments, the amino acid sequence of the flagellin protein is set forth in SEQ ID NO: 3 (derived from strain LT2) or SEQ ID NO: 29 (derived from strain Ty2). Although methionine is an amino acid at position 1 of the N-terminal of a natural flagellin molecule, the N-terminal of the flagellin protein in the present invention starts at amino acid (Ala) at position 2 of the natural sequence.

The N-terminal region of the *S*. *typhimurium* LT2 flagellin protein described herein generally starts at Alanine at position 2 in SEQ ID NO: 3 and ends at any of the amino acids at positions 137-176; and the C-terminal region of the *S*. *typhimurium* LT2 flagellin protein generally starts at any of the amino acids at positions 392-406 and ends at the amino acid at position 495.

As a specific embodiment of the present invention, the N-terminal region of the flagellin protein has an amino acid sequence having at least 95% homology (e.g., 97%, 98% or 99% homology) to the amino acid region from 2 to 176 in SEQ ID NO: 3; and the C-terminal region of the flagellin protein has an amino acid sequence having at least 95% homology (e.g., 97%, 98% or 99% homology) to the amino acid region from 392 to 495 in SEQ ID NO: 3.

In a specific embodiment, the amino acid sequence of the N-terminal region of the flagellin protein is set forth in SEQ ID NO: 5 in the sequence listing; and the amino acid sequence of the C-terminal region of the flagellin protein is set forth in SEQ ID NO: 6 in the sequence listing.

The N-terminal region of the *S*. *typhi* Ty2 flagellin protein described herein generally starts at Alanine at position 2 in SEQ ID NO: 29 and ends at any of the amino acids at positions 180-200; and the C-terminal region of the S. *typhi* Ty2 flagellin protein starts at any of the amino acids at positions 278-400 and ends at the amino acid at position 506.

In some embodiments, the N-terminal region of the Ty2 flagellin protein is positions 2-180 in SEQ ID NO: 29, and the C-terminal region of the Ty2 flagellin protein is positions 400-506 in SEQ ID NO: 29; or the N-terminal region of the Ty2 flagellin protein is positions 2-220 in SEQ ID NO: 29, and the C-terminal region of the Ty2 flagellin protein is positions 320-506 in SEQ ID NO: 29; or the N-terminal region of the Ty2 flagellin protein is positions 1-190 in SEQ ID NO: 29, and the C-terminal region of the Ty2 flagellin protein is positions 278-506 in SEQ ID NO: 29.

In some specific embodiments of the present invention, the N-terminal region of the flagellin protein has an amino acid sequence having at least 95% homology (e.g., 97%, 98% or 99% homology) to the amino acid region from 2 to 180 in SEQ ID NO: 29; and the conserved C-terminal region has an amino acid sequence having at least 95% homology (e.g., 97%, 98% or 99% homology) to the amino acid region from 400 to 506 in SEQ ID NO: 29.

In a specific embodiment, the amino acid sequence of the conserved N-terminal region is set forth in SEQ ID NO: 30; and the amino acid sequence of the conserved C-terminal region is set forth in SEQ ID NO: 31 in the sequence listing.

In some specific embodiments of the present invention, the amino acid sequence of the gE-based fusion protein is set forth in any one of SEQ ID NOs: 8-10, 32-34.

The nucleic acid molecules described herein are typically optimized according to the expression system, and include, but are not limited to: DNA, RNA, mRNA, ssDNA or cDNA.

The nucleic acid molecule may be operably linked to expression control sequences, and the expression control sequences include, but are not limited to: promoters, enhancers, transcription terminators, initiation codons (e.g., ATG), splicing signals of introns, termination codons and the like, and control elements for *in vitro* and *in vivo* conditional expression may also be used. Additional gene fragments may be added by using standard molecular biology techniques, such as, but not limited to, the early enhancer of human cytomegalovirus, Kozak consensus sequences, leader sequences, Woodchuck hepatitis virus post-transcriptional regulatory elements, nucleic acid sequences encoding glycosylation receptor sequences, or unrelated proteins such as tags or cleavage sites, wherein the unrelated proteins include those used to optimize gene expression, message stability, protein production, secretion and purification, etc. Methods for cloning and constructing various genes well known to those skilled in the art, as well as expression systems for host cells, can be used. The DNA sequences encoding proteins disclosed herein may be expressed in prokaryotic and eukaryotic host cells.

In an eukaryotic expression system, the 5' terminal of the nucleic acid molecule may further include a nucleic acid leader sequence to facilitate protein secretion, and the nucleic acid leader sequence includes but is not limited to Japanese Encephalitis Virus (JEV) prM protein gene leader sequence or mouse Igκ light chain gene leader sequence; and/or the 5' terminal of the nucleic acid molecule may further include a Kozak sequence to enhance translation efficiency; and/or the 3' terminal of the nucleic acid molecule may further include a polyadelynation (polyA) sequence to increase the stability of the nucleic acid molecule, and the polyA sequence includes but is not limited to SV40 poly A.

Preferably, the JEV signal peptide gene sequence is set forth in SEQ ID NO: 14.

Preferably, the Igκ signal peptide gene sequence is set forth in SEQ ID NO: 15.

Preferably, the Kozak sequence gene sequence is set forth in SEQ ID NO: 16.

Preferably, the SV40 polyA gene sequence is set forth in SEQ ID NO: 17.

The signal peptide encoded by the nucleic acid leader sequence is cleaved hydrolytically during intracellular processing of the native protein.

In some specific embodiments, the nucleic acid molecule encoding the gE extracellular region or a fragment thereof has the sequence as set forth in any one of SEQ ID NOs: 2,18 and 19.

In some specific embodiments, the nucleic acid molecule encoding the gE-based fusion protein has the sequence as set forth in any one of SEQ ID NOs: 11-13,20-26.

The gene sequence encoding the gE-based fusion protein as set forth in SEQ ID NO: 8 is set forth in any one of SEQ ID NOs: 11, 20 and 21; the nucleic acid molecule sequence encoding the gE-based fusion protein as set forth in SEQ ID NO: 9 is set forth in any one of SEQ ID NOs: 12,22 and 23; and the nucleic acid molecule sequence encoding the gE-based fusion protein as set forth in SEQ ID NO: 10 is set forth in any one of SEQ ID NOs: 13, 33 and 34. The nucleic acid molecule sequence encoding the gE-based fusion protein as set forth in SEQ ID NO: 34 is set forth in SEQ ID NO: 26.

The gE-based recombinant vector described herein carries the nucleic acid molecules as described above. It should be understood that the gE-based recombinant vector may carry a gene encoding the gE extracellular region or a fragment thereof as described above, or carry a gene encoding the gE-based fusion protein as described above. The vector may be an expression vector, a cloning vector, or a transfer vector, including but not limited to: a virus vector, a DNA vector, an mRNA vector and the like. The virus vector includes, but is not limited to: an adenovirus vector, an adenovirus-associated virus vector, a poxvirus vector, a vesicular stomatitis virus vector, a bovine parainfluenza virus vector, a human parainfluenza virus vector, a newcastle disease virus vector, a Sendai virus vector, a measles virus vector, an attenuated RSV vector, a paramyxovirus vector, a type A virus vector (e.g., Venezuelan equine encephalitis virus vector, Semliki Forest virus vector, Sindbis virus vector), a rhabdovirus vector, a rabies virus vector, a picornavirus vector, a lentivirus vector, a herpesvirus vector, or a plant-derived virus for expression in a plant expression system.

In some specific embodiments, the adenovirus vector is a human adenovirus vector (e.g., adenovirus type-5 vector (Ad5)), a chimpanzee adenovirus vector (e.g., ChAd68), a gorilla adenovirus vector, or other human-suitable adenovirus vectors.

In some specific embodiments, the recombinant adenovirus vector is a replication-defective recombinant adenovirus vector, wherein the replication-defective recombinant adenovirus vector may be a replication-defective adenovirus formed by deletion or functional deletion of the El region of the adenovirus genome, or a further deletion or functional deletion of the E3 region, or deletions or functional deletions of both the El region and the E3 region; and all vectors with functional deletions of El are replication-defective vectors. The functional deletion generally refers to the loss of the original function of El caused by mutation, deletion or addition of sites and the like, thereby affecting the replication of adenovirus. These viruses can therefore only replicate in mammalian cells that supplementarily express El protein, such as HEK293 and PER.C6 cells, whose genomes have been modified to express the El gene.

The remaining adenovirus genomes of the replication-defective recombinant adenovirus vector of the present invention may be original adenovirus genomes (i.e., it is understood that in addition to the deletion or functional deletion of the El region, or both El and E3, the remaining genomes have not been further modified, such as the pAd5-CMV/V5-Dest vector purchased from Thermo Fisher Scientific), or may be further modified adenovirus genomes. The modification refers to substitution, mutation and the like of the original adenovirus genome. For example, in particular embodiments, the E4 region of the replication-defective chimpanzee adenovirus (e.g., ChAd68) itself is replaced by the E4 region of human adenovirus type 5 to improve the performance of the vector.

The gE-based recombinant vector is referred to as recombinant adenovirus vector A when it carries the nucleic acid molecule encoding the gE extracellular region or a fragment thereof as described above (e.g., a nucleic acid molecule as set forth in any one of SEQ ID NOs: 2,18 and 19). In other words, the gE is expressed in a non-fused form.

Preferably, the recombinant adenovirus vector A is constructed by homologous recombination.

Preferably, the backbone plasmid used to construct the recombinant adenovirus vector A is pAd5-CMV/N5-DEST.

Preferably, the shuttle plasmid used to construct the recombinant adenovirus vector A is pDONR221.

Preferably, the host cell line used to construct the recombinant adenovirus vector A includes, but is not limited to, HEK 293 cell line or PER.C6 cell line.

In some specific embodiments, the recombinant adenovirus vector A is constructed as follows: preforming homologous recombination on a correctly sequenced recombinant shuttle plasmid pDONR221-gE gene-PolyA and the virus backbone plasmid pAd5-CMV/V5-DEST, transforming the recombination mixture into *E. coli* TOP10 competent cells, screening a correctly sequenced adenovirus vector pAdS-CMV-gE gene-PolyA, linearizing it, transfecting HEK 293 or PER.C6 cells with the linearized adenovirus vector pAdS-CMV-gE gene-PolyA for packaging, and thus obtaining the recombinant adenovirus vector A. This technique is well known to those skilled in the art.

The gE-based recombinant vector is referred to as recombinant adenovirus vector B when it carries the nucleic acid molecule encoding the gE-based fusion protein as described above (e.g., a nucleic acid molecule as set forth in any one of SEQ ID NOs: 11-13,20-26).

Preferably, the recombinant adenovirus vector B is constructed by homologous recombination.

Preferably, the virus backbone plasmid used to construct the recombinant adenovirus vector B is pAdS-CMV/V5-DEST.

Preferably, the shuttle plasmid used to construct the recombinant adenovirus vector B is pDONR221.

Preferably, the host cell line used to construct the recombinant adenovirus vector B includes, but is not limited to, HEK 293 cell line or PER.C6 cell line.

In some specific embodiments, the recombinant adenovirus B is constructed as follows: performing homologous recombination on a correctly sequenced recombinant shuttle plasmid pDONR221-gE-flagellin fusion protein gene-PolyA and the virus backbone plasmid pAd5-CMV/V5-DEST, transforming the recombination mixture into *E. coli* TOP10 competent cells, screening a correctly sequenced adenovirus vector pAdS-CMV-gE-flagellin fusion protein gene-PolyA, linearizing it, then transfecting HEK 293 or PER.C6 cells with the linearized adenovirus vector pAd5-CMV-gE-flagellin fusion protein gene-PolyA for packaging, and thus obtaining the recombinant adenovirus vector B. This technique is well known to those skilled in the art.

The immune composition of the present invention as described above may further comprise one or more other components, such as a pharmaceutically acceptable carrier, and/or an adjuvant, and/or an immunostimulatory molecule. The adjuvant includes, but is not limited to: aluminum salts (such as aluminum hydroxide or aluminum phosphate), oil-in-water or water-in-oil emulsions, MF-59, TLR agonists (such as monophosphoryl lipid A (MPL) or analogs thereof, or CpG oligonucleotide), Quil A or QS21 components thereof, chitosan, or combinations of two or more thereof. The adjuvant has the function of enhancing humoral and/or cellular responses. The immunostimulatory molecule may include, but is not limited to, *E. coli* heat-stable enterotoxin (LT), cholera toxin (CT), or analogs thereof, and the like; cytokines or chemokines; antibodies or fragments thereof against specific cell surface differentiation antigens or receptors involved in immune responses and capable of enhancing both humoral and cellular immune responses.

The pharmaceutically acceptable carrier may be one conventionally used in the art and generally depends on the mode of drug administration. For example, dosage forms for parenteral administration and the like typically contain a pharmaceutically and physiologically acceptable injectable fluid as a carrier, including but not limited to water, normal saline, balanced salt solutions, glycerol or other carbohydrates. In addition, the immune composition may also comprise minor amount of nontoxic auxiliary substances such as emulsifiers, pH buffers, stabilizers or preservatives. Sterile solutions are prepared by sterile filtration or by other methods known in the art. The pH of the solutions is generally between 3.0 and 9.0, preferably between 5.0 and 7.5. Formulations may be stored in a liquid form or as a lyophilizate, and may be provided in single dose or in multiple-dose sealed containers. The immune composition of the present invention may also be delivered using carrier systems including but not limited to liposomes, microspheres, micellar systems, immunostimulatory complexes (ISCOMs) and nanoparticles, wherein the nanoparticles include ferritin, encapsulin, Sulfur Oxygenase Reductase (SOR), and lumazine synthase nanoparticles.

The immune composition of the present invention as described above may be administered by delivery systems known to those skilled in the art, including subcutaneous, intramuscular, intradermal or intranasal administration. The nucleic acid-based immune composition of the present invention may also be administered by biolistic technology, and the recombinant protein immunogen may be administered by needleless delivery systems.

The immune composition of the present invention as described above can be used for preventing and/or treating varicella zoster infection. In particular, the immune composition can be used for vaccinating infants, children, teenagers, adults or the elderly against varicella infection or vaccinating the elderly against herpes zoster infection, and on the other hand, the immune composition can be used for treating shingles and/or postherpetic neuralgia. Generally, infants are aged 0-12 months, children are 1-12 years, teenagers are 12-18 years, adults are more than 18 years, and the elderly are 50 years or more. It should be understood that the division of age groups is not in any way restricted to the above description and that the immune composition can be used to immunize the age-matched population against varicella or herpes zoster infection.

In another aspect, the present invention provides use of the immune composition as described above in the manufacture of a medicament for preventing and/or treating varicella-zoster virus infection, and further, use of the immune composition in the manufacture of chickenpox vaccine and/or shingles vaccine; or the immune composition can be used for preparing a medicament for treating shingles and/or postherpetic neuralgia.

The present invention further provides a combination vaccine comprising the immune composition as described above and one or more other vaccines. It should be understood that the antigenic components of the combination vaccine do not interfere with each other, or they may further achieve a synergistic effect. Non-interfering generally means that the immunogens are maintained stable and compatible, without competition between the antigens or risk of serious adverse reactions. In addition, each antigenic component of the combination vaccine should have the same or similar population of subjects and immunization program. In the present invention, the other combinable vaccines include, but are not limited to: mumps, measles and rubella vaccines.

The gE-based fusion protein, the corresponding nucleic acid molecule and the gE-based recombinant vector of the present invention as described above can be used for preventing and/or treating varicella zoster infection, and in particular can be used for vaccinating infants, children, teenagers, adults or the elderly against varicella infection or vaccinating the elderly against herpes zoster infection.

In one aspect, the present invention provides use of the gE-based fusion protein, the nucleic acid molecule and the gE-based fusion protein recombinant vector in the manufacture of a medicament for preventing and/or treating varicella-zoster virus infection, and further, use in the manufacture of chickenpox vaccine and/or shingles vaccine. The immune composition can be used for preparing a medicament for treating shingles and/or postherpetic neuralgia. Vaccination may involve single injection or multiple injections at intervals of one or more months, with dosage ranging from 1 µg to 100 µg of recombinant proteins or 10¹⁰ to 10¹² viral particles (VP) of adenovirus vectors. The specific dosage used will be determined in clinical trials and will depend on the administration route and the target population. If desired, boost immunizations can be given annually.

The prime-boost immunization regimen comprises: administering a first immune composition (the primary vaccine) to a subject, followed by administration of a second immune composition (the booster vaccine) to induce an optimal immune response. Those skilled in the art should appreciate the appropriate time interval between the prime immunization and boost immunization. The immune compositions administered for the prime immunization and boost immunization may be the same or different and the respective amounts may be different. In the present invention, the gE extracellular region or a fragment thereof, the gE-based fusion protein, the nucleic acid molecule and the gE-based recombinant vector can each be used for a prime immunization or a boost immunization. For example, the present invention provides the following prime-boost immunization regimen: (1) a prime immunization with the gE-based recombinant vector, and a boost immunization with the gE extracellular region or a fragment thereof or the gE-based fusion protein; or (2) a prime immunization with the gE extracellular region or a fragment thereof or the gE-based fusion protein, and a boost immunization with the gE-based recombinant vector. The combinations of prime-boost immunization regimen include, but are not limited to, those described above. For example, the prime immunization may be performed with a gE-based adenovirus vector, and then the boost immunization is performed with a different vector as described above (e.g., a poxvirus vector, etc.) expressing the same gene, or conversely, the prime immunization may be performed with a gE-based heterologous vector (it may be understood as a vector other than the adenovirus vector) and the boost immunization is performed with the gE-based adenovirus vector of the present invention. In addition, two adenovirus vectors of different types or derived from different species that express the same or different gE-based genes can also be used in combination in the prime-boost immunization regimen.

The dosage used will depend on the immune components, the administration route, the target population, and other factors. Clinical trial staff will determine the appropriate dosage of each immune component and the effective immunization regimen based on their knowledge. A single administration may be sufficient, or multiple administrations with a single and/or combined immunogen are required.

In yet another aspect, the present invention provides an isolated host cell, which comprises the gE-based gene as described above (e.g., a nucleic acid molecule encoding the gE extracellular region or a fragment thereof, or a nucleic acid molecule encoding the gE-based fusion protein). The host cell includes, but is not limited to: *E. coli, bacillus subtilis, Salmonella, Saccharomyces cerevisiae, pichia pastoris,* insect cell, HEK293 cell, PER.C6 cell, Vero cell, CHO cell, W38 cell, BHK cell, or COS cell.

In one aspect, the present invention provides a method for preparing the gE extracellular region or a fragment thereof as shown above, or the gE-based fusion protein as described above, which, in particular, can be expressed in a prokaryotic expression system or a eukaryotic expression system. The gE extracellular region or a fragment thereof is prepared into the form with or without a covalently bound protein tag that facilitates purification; the gE-based fusion protein is prepared into the form with or without a covalently bound protein tag that facilitates purification; and the covalently bound protein tag includes, but is not limited to, a polyhistidine tag (His-tag).

The prokaryotic expression system includes but is not limited to an *E. coli* expression system. In some embodiments, the *E. coli* used is BL21(DE3), and the prokaryotic expression vector may contain, but is not limited to, a T7 promoter. Preferably, the expression vector is pET28a. Preferably, the amino acid sequence of the gE extracellular region is set forth in SEQ ID NO: 35, and the gene sequence of the gE extracellular region is set forth in SEQ ID NO: 36; the amino acid sequence of the gE-based fusion protein is set forth in SEQ ID NOs: 37-39; and the gene sequence of the gE-based fusion protein is set forth in SEQ ID NOs: 37-39.

As a specific embodiment of the present invention, the prokaryotic expression may comprise the following steps: transforming the pET28a expression vector carrying the gene of the gE extracellular region or a fragment thereof or carrying the gene of the gE-based fusion protein into *E. coli* BL21(DE3) cells, coating the *E. coli* BL21(DE3) cells on an agar plate and culturing in an LB medium containing kanamycin (50 µg/ml); selecting single clones and seeding them into the LB liquid medium containing kanamycin, culturing the mixture at 37 °C until OD₆₀₀ reaches 0.6, adding 0.1-1 mM IPTG, and inducing expression at 16-37 °C; crushing the collected thalli by an ultrasound homogenizer or a high-pressure homogenizer, centrifuging to collect inclusion bodies (IBs), and washing the inclusion bodies several times with detergent-containing saline; resuspending the inclusion bodies and dissolving in a buffer solution containing 6 M guanidine hydrochloride or 8 M urea (20 mM Tris, 5 mM imidazole, 500 mM NaCl, pH 8.0); and loading the dissolved inclusion bodies onto a Ni column, washing the Ni column with 5-10 column volumes (20 mM Tris, 8 M urea, 5-50 mM imidazole, 500 mM NaCl, pH 8.0), and eluting the protein with imidazole (20 mM Tris, 8 M urea, 500 mM imidazole, 500 mM NaCl, pH 8.0) at an appropriate concentration. Protein renaturation was performed on the column or after purification.

The eukaryotic expression system includes, but is not limited to, a yeast expression system, a mammalian cell expression system, or a recombinant virus (e.g., human, animal or plant recombinant virus, or baculovirus, adenovirus, lentivirus or poxvirus) expression system, or a plant expression system. Preferably, a mammalian cell line for expression includes, but is not limited to, 293 cell line or PER.C6 cell line, Chinese hamster ovary (CHO) cell line, insect cell lines such as SF9 cell line, Vero cell line, or transgenic animal or plant cell line. The recombinant protein may be expressed by transient expression, stable expression cell line, or recombinant virus vector. Cell media are available from commercial sources, appropriate conditions for culturing cells are well known, and one skilled in the art can readily select the media and the culture conditions for the host cell to express the immunogen of interest. Suitable media may or may not contain serum.

In some specific embodiments, the eukaryotic expression comprises the following steps: infecting host cells at 90% confluence (in some embodiments, the host cells include, but are not limited to, Vero cells or CHO cells, etc.) with the gE-based recombinant vector (preferably, the recombinant adenovirus vector A encoding the gE protein and the recombinant adenovirus vector B encoding the gE-flagellin fusion protein) as described above at a certain MOI value, collecting the culture supernatant after four to five days of infection, and purifying the collected supernatant to obtain the corresponding protein. In the steps, the MOI value may be 10 to 500, and more preferably, the MOI value may be 100 to 200. The purification step comprises purifying by ion exchange chromatography and/or size exclusion chromatography after hydrophobic chromatography, wherein, the hydrophobic packing includes, but is not limited to: phenyl, octyl or butyl related packing; the ion exchange packing includes, but is not limited to: Qsephrase FF, DEAE or Source 30Q; and the size exclusion chromatography packing includes, but is not limited to: Sephadex G200, G100 or G75. As a preferred embodiment, the purification process is performed by first performing hydrophobic chromatography and then ion exchange chromatography. Preferably, the hydrophobic packing is Capto Phenyl Impress, and the ion exchange packing is Source 30Q.

The present invention further provides the recombinant adenovirus vector pAd5-CMV-gE gene-PolyA as described above, wherein the gE gene is the nucleic acid sequence as set forth in any one of SEQ ID NOs: 2,18 and 19.

The present invention further provides the recombinant adenovirus vector Ad5-CMV-gE-flagellin fusion gene-PolyA as described above, wherein the gE-flagellin fusion gene has a nucleic acid sequence as set forth in any one of SEQ ID NOs: 11-13,20-26.

In another aspect, the present invention further provides a modified flagellin protein, wherein the N-terminal region of the flagellin protein has an amino acid sequence having at least 95% homology (e.g., 96%, 97%, 98% or 99% homology) to the amino acid region from 2 to 176 in SEQ ID NO: 3; the C-terminal region of the flagellin protein has an amino acid sequence having at least 95% homology (e.g., 96%, 97%, 98% or 99% homology) to the amino acid region from 392 to 495 in SEQ ID NO: 3; and the N-terminal region of the flagellin protein is linked to the C-terminal region of the flagellin protein either directly or via a linker.

The linker may be 1-20 amino acids linked via peptide bonds, such as the amino acid sequence as set forth in SEQ ID NO: 4.

In some embodiments, the amino acid sequence of the N-terminal region of the flagellin protein is set forth in SEQ ID NO: 5 in the sequence listing; and the amino acid sequence of the C-terminal region is set forth in SEQ ID NO: 6 in the sequence listing.

In some embodiments of the present invention, the modified flagellin protein has an amino acid sequence as set forth in SEQ ID NO: 27.

The present invention further provides a nucleic acid sequence capable of encoding the amino acid sequence as set forth in SEQ ID NO: 27. Preferably, the nucleic acid sequence is set forth in SEQ ID NO: 28.

The present invention further provides use of the modified flagellin protein as an immune adjuvant. When the flagellin protein is coupled with gE or a fragment thereof to form a fusion protein, the thus formed fusion protein has intrinsic adjuvant properties. Therefore, the gE-based fusion protein or the recombinant adenovirus vector (such as the recombinant adenovirus vector B) that may express the flagellin fusion protein can be used directly to prepare a vaccine for immunizing a host (human or animal) to induce and/or enhance an immune response to VZV and thus fight against acute or potential VZV infection.

The present invention discloses a method for efficiently expressing gE or gE-flagellin fusion immunogen in a prokaryotic expression system or a recombinant adenovirus system. Experimental data shows that the prepared gE, gE-flagellin fusion protein and the recombinant adenovirus vector can stimulate an immune host to generate high-level antibody titer and good cellular immunity, and thus can be developed into a new generation of modified VZV vaccine.

### Terminology

Prevention or treatment of diseases: "prevention", "preventing", or "prevent" refers to inhibiting the overall development of infection or disease in a subject at risk of developing the disease (e.g., VZV infection). "Treatment", "treating" or "treat" refers to therapeutic intervention that ameliorates a sign or symptom of a disease or pathological state after it begins to progress. The term "amelioration", "ameliorating" or "ameliorate" refers to any observable beneficial therapeutic effect, such as delay in the onset of clinical symptoms of a disease, reduction in symptoms of a disease, slowing of disease progression, improvement in the overall health of a subject, or other specific indicators of a particular disease recognized in the art. "Prophylactic" treatment is treatment of a subject who presents no symptoms of a disease or only early symptoms, with the aim of reducing the risk of developing the condition.

Adenovirus type-5 (Ad5): the term refers to a double-stranded DNA virus that belongs to adenoviridae and causes primarily respiratory tract infection in humans. El gene products (including E1A and E1B) are involved in viral replication. Most E3 proteins are involved in regulating the immune response of infected cells. The deletion of the El region may cause the virus to lose replication capability, and then a heterologous transgene may be inserted into the deleted El region and E3 region, allowing the virus to act as a vector for immunization or gene therapy purposes.

Adjuvant: the term refers to a substance that enhances the immune response of a host to an immunogen or a vaccine.

Antibody: the term refers to a blood protein produced by specific plasma cells that plays a major role in humoral adaptive immune responses against foreign molecules or pathogens. Antibodies recognize specific sites on a homologous immunogen, thereby neutralizing or eliminating these antigens.

Antibody-dependent cellular cytotoxicity (ADCC): the term refers to an immune defense mechanism by which effector cells of the immune system actively lyse target cells whose membrane surface antigens have been bound to specific antibodies.

Cellular immunity: the term refers to an immune response that involves activation of sentinel dendritic cells and lymphocyte subsets that respond to the immunogen. The dendritic cells are responsible for the initiation of non-specific innate immunity as well as immunogen-specific adaptive immunity. The lymphocyte subsets comprise CD4+ helper T cells responsible for proinflammatory responses and helping antibody production, and cytotoxic CD8+ T cells that can kill infected targets.

Conditional gene expression: the term refers to the ability to either activate or suppress the expression of a specific gene or gene product.

CpG oligonucleotide: CpG oligodeoxynucleotide is a short single-stranded synthetic DNA molecule containing cytosine deoxynucleotide triphosphate and guanine deoxynucleotide triphosphate. CpG motifs are pathogen-associated molecular patterns, and therefore have properties of an immune adjuvant as TLR9 agonists.

Extracellular domain: the term refers to a domain formed by a membrane protein extending to the extracellular space. A membrane protein consists of an extracellular domain (ECD), a transmembrane segment and an intracytoplasmic tail.

Enhancer: the term refers to a DNA sequence capable of increasing the level of transcription of a gene located adjacent to an encoding sequence.

Flagellin: the term refers to a polymeric protein which is the major component of flagella of gram-negative bacteria and determines the specificity of flagella in eliciting an immune response. Flagellin is a potent immunomodulator.

Fusion protein: the term refers to a protein produced by the binding of two or more genes that originally encode separate proteins.

Homologous recombination: the term refers to exchange of genetic material between two strands of DNA containing long stretches of similar base sequences. Homologous recombination occurs naturally in eukaryotes, bacteria, and certain viruses, and it is a powerful tool for genetic engineering.

Host cell: the term refers to a cell containing foreign molecules, viruses or microorganisms.

Immunogen: the term refers to a substance or organism capable of eliciting immunity upon entry into a host, including humoral (antibody) and cellular responses.

Immune composition: the term refers to a composition capable of inducing immunity.

Immunostimulatory molecule: the term refers to a molecule capable of stimulating or enhancing an immune response.

Innate immunity: the term refers to a natural mechanism featuring in defense by sentinel cells (such as dendritic cells and macrophages) of the immune system. This immunity is not elicited by prior sensitization to immunogen, such as infection or vaccination. As opposed to acquired immunity which is immunogen-specific and has immunological memory, innate immunity is generally immediate, nonspecific and memoryless as it is not stimulated by specific immunogens.

Immunostimulatory complex (ISCOM): the term refers to a spherical cage-like structure that forms spontaneously when cholesterol, phospholipid and quercetin are mixed at a specific stoichiometric ratio. ISCOMs exhibit properties of an immune adjuvant and can be used in vaccines to enhance their immune response.

Kozak sequence: the term refers to a nucleic acid sequence present on mRNA of a eukaryote, typically (gcc)gccRccAUGG. The Kozak sequence plays an important role in the initiation of translation process.

Leader sequence: the term refers to a nucleotide sequence at the 5' terminal of messenger RNA (and DNA), which is located upstream of the translation initiation codon.

Liposome: the term refers to microspheres of phospholipid molecules encapsulating water droplets, particularly artificially formed liposomes for delivering vaccines, drugs or other substances to tissues.

Nanoparticle: the term refers to microparticles smaller than 100 nm, which can not only improve the stability and immunogenicity of a vaccine, but also allows for efficient delivery and sustained release.

Neutralizing: the term refers to the process the causes the loss of infectivity of a pathogen by interaction with a pathogen-specific antibody.

Packaging cell line: a recombinant vector is transfected into a packaging cell line to complement the deleted viral gene in the recombinant virus vector, thereby producing a recombinant virus containing a transgene.

Polyadenylation sequence (polyA tail): the term refers to the addition of multiple adenosine monophosphates to messenger RNA (mRNA), which is part of the maturation process of mRNA prior to translation.

Promoter: the term refers to a site in the DNA molecule where RNA polymerase and transcription factors bind to initiate transcription of mRNA by a particular gene.

Replication-defective vector: the term refers to a virus vector that cannot replicate as a critical portion of virus genome is deleted.

Shuttle plasmid: the term refers to a plasmid capable of propagating in two different host species.

Signal peptide: the term refers to a short peptide (5-30 amino acids in length) that is present at the N-terminal of most newly synthesized proteins and eventually enters the secretory pathway.

Initiation codon: the term refers to the first codon of a messenger RNA (mRNA) transcript translated by ribosomes. In eukaryotes, the initiation codon always encodes methionine, while in prokaryotes, it always encodes modified methionine (fMet). The most common initiation codon is AUG.

SV40 polyA: SV40 polyA sequence is a terminator sequence which indicates the end of a transcription unit.

Tag: protein tags are peptide sequences that are genetically grafted onto recombinant proteins, particularly to facilitate purification. For example, polyhistidine tags bind to nickel columns, thus allowing purification of proteins by affinity chromatography.

T cell subsets: the term refers to lymphocyte subsets having a specific immune function for an immune response. CD4+ T helper cells are essential for antibody production. They also participate in the proinflammatory response by releasing soluble immunostimulatory mediators, such as cytokines and chemokines. Type 1 helper T cells (Thl) are essential for the host to fight against intracellular viral and bacterial pathogens, and also produce interferon γ (IFN-γ). Type 2 helper T cells (Th2) play an important role in host resistance to extracellular pathogens and also secrete IL-4. Cytotoxic CD8+ T cell is a subset of lymphocytes responsible for killing infected cells and secreting IFN-γ.

TLR agonist: the term refers to a reagent capable of activating immune cells by interacting with cognate TLR receptors and thereby facilitating and coordinating the initiation of innate immunity and adaptive immunity.

Toll-like receptors (TLRs): the term refers to a class of proteins that play a key role in the innate immune system. They are single, transmembrane and non-catalytic receptors that are normally expressed on sentinel cells (such as macrophages and dendritic cells), and recognize structurally conserved molecules derived from microorganisms.

Transcription terminator: the term refers to a section of nucleic acid sequence that marks the end of a gene or operon in genomic DNA during transcription.

Transfection: the term refers to a process for introducing a nucleic acid into a mammalian cell. There are various methods and techniques for transfection, including lipofection and chemical and physical methods, such as electroporation.

Transformation: the term refers to the process of inserting a foreign plasmid or a ligation product into bacteria such as *E. coli.*

Virus vector: the term refers to a tool commonly used by molecular biologists to deliver genetic material into cells. This process can be carried out in a living organism (*in vivo)* or in cell medium (*in vitro*)*.* Viruses have evolved specialized molecular mechanisms to efficiently transport their genomes, and they are capable of transmitting genes and other genetic materials within the cells they infect.

### BRIEF DESCRIPTION OF THE DRAWINGS

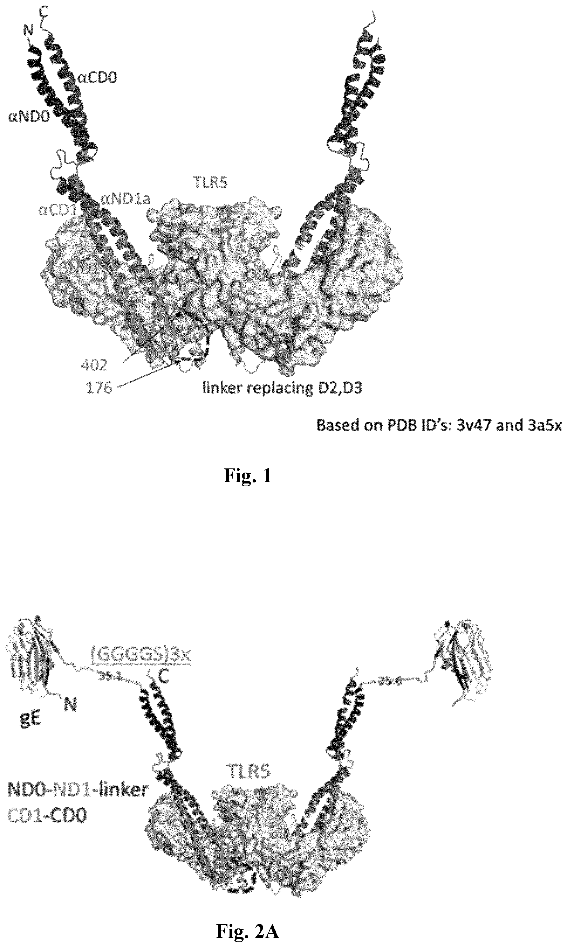

FIG. 1 shows a schematic diagram of a three-dimensional structure of the interaction between modified flagellin protein and a toll-like receptor simulated by Phyre2 software. (Reference: Phyre2 web portal for protein modeling, prediction and analysis. Kelley LA et al., Nature Protocols 10, 845-858, 2015).

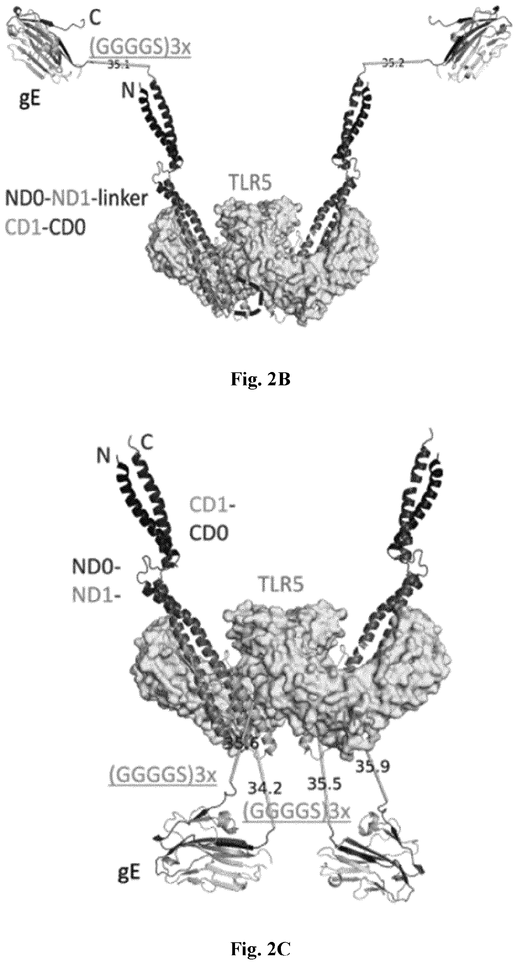

FIG. 2 shows a schematic diagram of a three-dimensional structure of the interaction between the gE-flagellin fusion protein and a toll-like receptor simulated by a computer. The method for computer-predicted immunogen design comprises the following steps: first, the envelope glycoprotein E model of varicella zoster virus (strain Dumas; UniProtKB P09259) was first generated by the Phyre2 webpage, then the signal peptide secretion sequence, transmembrane region and intracellular region of VZV gE were removed from the protein model, and then boundaries of *S. typhimurium* flagellin protein sequences (strain LT2; UniProtKB P06179) were determined based on the information in the database PDB ID's 3v47 and 3a5x (Yoon S-il et al., Science, 335:859-864, 2012). During the process of designing a fusion protein of VZV gE and flagellin protein, GGGGS linkers of different lengths were designed depending on the position of fusion (N-terminal, C-terminal, or insertion of gE protein in the middle) to minimize steric hindrance. FIG. 2A shows the fusion of modified flagellin protein to the N-terminal of gE protein (ANF); FIG. 2B shows the fusion of modified flagellin protein to the C-terminal of gE protein (ACF); and FIG. 2C shows the insertion of the gE protein, which replaces the D2 and D3 domains of the flagellin protein, into the hypervariable region of the flagellin protein (ASF).

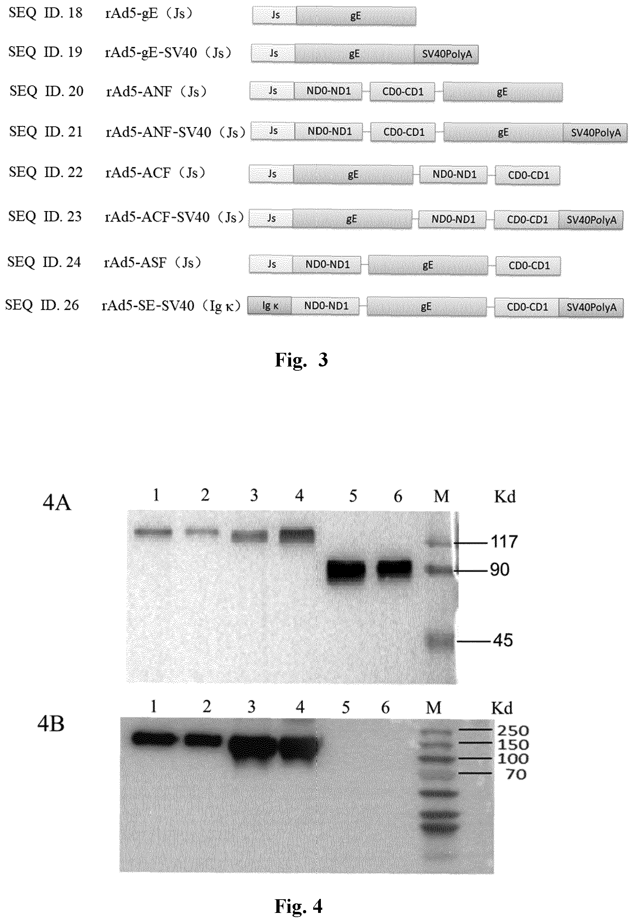

FIG. 3 shows the name abbreviations and corresponding inserted genes of the recombinant adenovirus vector carrying the gE and gE-flagellin fusion genes. "Js" represents the prM leader peptide gene sequence of Japanese Encephalitis Virus (JEV). "Igκ" refers to the leader peptide gene sequence of mouse IgG κ light chain.
FIG. 4 shows the expression of exogenous genes in the supernatant of Vero cells detected by Western Blotting (WB) after infection with recombinant adenoviruses 1: rAd5-ACF (Js); 2: rAd5-ACF-SV40 (Js); 3: rAd5-ANF (Js); 4: rAd5-ANF-SV40 (Js); 5: rAd5-gE (Js); 6: rAd5-gE-SV40 (Js). The primary antibody used in FIG. 4A is mouse anti-VZV gE monoclonal antibody, and the primary antibody used in FIG. 4B is rabbit anti-flagellin D0, D1 polyclonal antibody. M represents protein molecular weight marker.

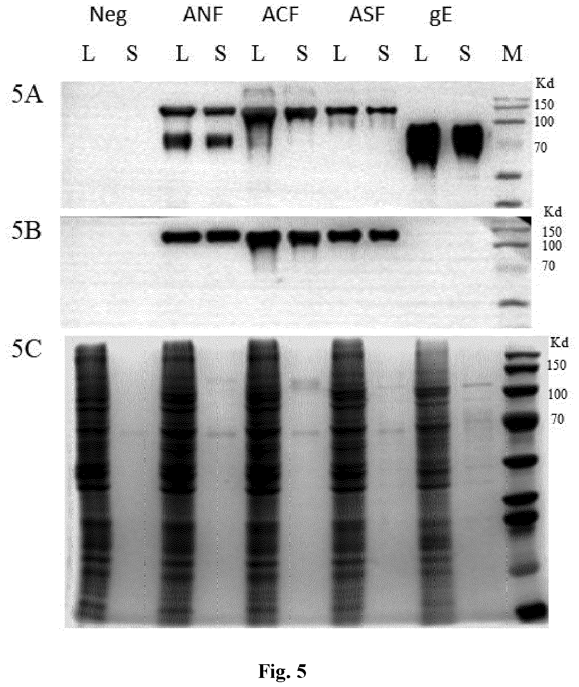

FIG. 5 shows the expression of exogenous genes in the supernatant (S) and cell lysate (L) of 293A cells analyzed by Western blotting and SDS-PAGE after infection with recombinant adenoviruses. FIG. 5A shows the results of WB detection with mouse anti-VZV gE monoclonal antibody as a primary antibody; FIG. 5B shows the results of WB detection with rabbit anti-flagellin D0, D1 antiserum as a primary antibody; and FIG. 5C shows the results of SDS-PAGE detection; in these figures, gE represents the supernatant (S) and cell lysate (L) of HEK293 cells infected with rAd5-gE-SV40 (Js), ANF represents the supernatant (S) and cell lysate (L) of HEK293 cells infected with rAd5-ANF-SV40 (Js), ACF represents the supernatant (S) and cell lysate (L) of HEK293 cells infected with rAd5-ACF-SV40 (Js), and ASF represents the supernatant (S) and cell lysate (L) of HEK293 cells infected with rAd5 ASF (Js).

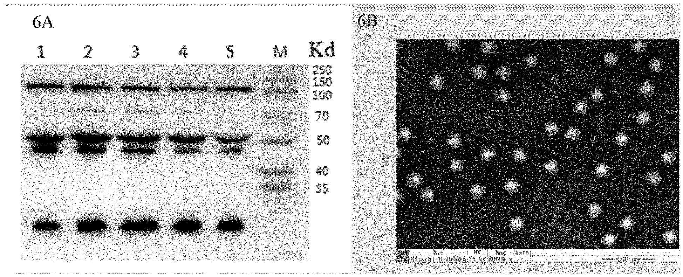

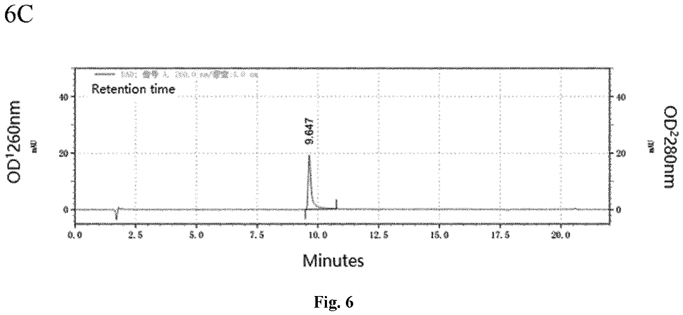

FIG. 6 shows the detection of the purified recombinant adenoviruses. FIG. 6A shows the WB detection of the purified recombinant adenovirus (rabbit anti-Ad5 polyclonal antibody as primary antibody). M: molecular weight markers; lane 1: purified rAd5-gE-SV40 (Js) virus; lane 2: purified rAd5-ANF-SV40 (Js) virus; lane 3: purified rAd5-ACF-SV40 (Js) virus; lane 4: purified rAd5-ASF (Js) virus; lane 5: purified rAd5-SE (Ig κ) virus. FIG. 6B shows Transmission Electron Microscopy (TEM) analysis of viral particles in 10¹⁰ TCID₅₀/mL sample. FIG. 6C shows anion exchange-high performance liquid chromatography (Agilent 1260) analysis of purified rAd5-gE-SV40 (Js) virus. 40 µL of the purified virus sample was loaded onto a column (4.8x250 mM Sepax SAX-NP5 anion exchange column, Sepax, China) equilibrated with 90% mobile phase A (20 mM Tris, pH 8.0) and 10% mobile phase B (20 mM Tris, 1M NaCl, pH 8.0). After loading was completed, the column was eluted in a linear gradient (10-60% mobile phase B) for 8 min, washed with 60% mobile phase B for 4 min, and then eluted in another linear gradient (60-100% mobile phase B) for 4 min. Finally, the column was washed with equilibration buffer for 4 min.

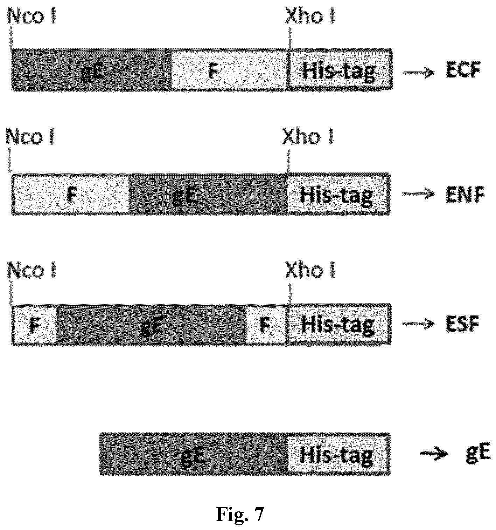

FIG. 7 shows the name abbreviations of the prokaryotically expressed recombinant gE protein and recombinant gE-flagellin fusion protein containing His-tag, and the corresponding inserted genes thereof.

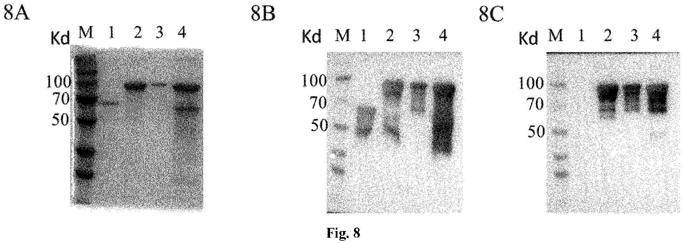

FIG. 8 shows the SDS-PAGE and Western Blotting detection of the purified *E*. *coli*-expressed recombinant gE protein and recombinant gE-flagellin fusion protein. FIG. 8A shows the results of SDS-PAGE detection; FIG. 8B shows the results of WB detection with mouse anti-VZV-gE monoclonal antibody as a primary antibody; and FIG. 8C shows the results of WB detection with rabbit anti-flagellin D0, D1 antiserum as a primary antibody. M: protein molecular weight markers; lane 1: purified gE protein; lane 2: purified ENF protein; lane 3: purified ESF protein; lane 4: purified ECF protein.

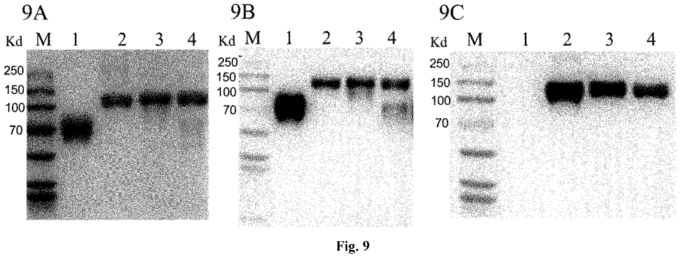

FIG. 9 shows the SDS-PAGE and Western Blotting detection of the purified Vero cell-expressed recombinant gE protein and recombinant gE-flagellin fusion protein. FIG. 9A shows the results of SDS-PAGE detection; FIG. 9B shows the results of WB detection with mouse anti-VZV-gE monoclonal antibody as a primary antibody; and FIG. 9C shows the results of WB detection with rabbit anti-flagellin D0, D1 antiserum as a primary antibody. M: protein molecular weight markers; lane 1: purified gE protein; lane 2: purified ANF protein; lane 3: purified ASF protein; lane 4: purified ACF protein.

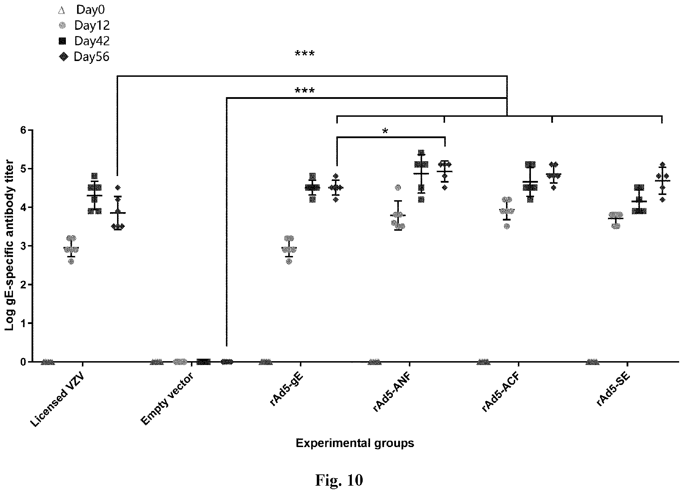

FIG. 10 shows the detection of VZV-gE specific antibodies in serum of mice immunized with recombinant adenoviruses. Each recombinant adenovirus (10⁹ TCID₅₀/dose) or a commercially available chickenpox vaccine (700 pfu/dose) was used to immunize C57BL/6 mice, by intramuscular injection, for a total of two doses with an interval of 30 days. Serum was collected at day 12, 26 and 42 after the first immunization and tested for gE-specific antibody titers by using enzyme-linked immunosorbent assay (ELISA) as described in materials and methods. Results for gE-specific antibody responses are represented by Geometric Mean Titers (GMT), with 95% upper and lower confidence interval. *** p < 0.001 (ANOVA/Bonferroni one-way analysis of variance).

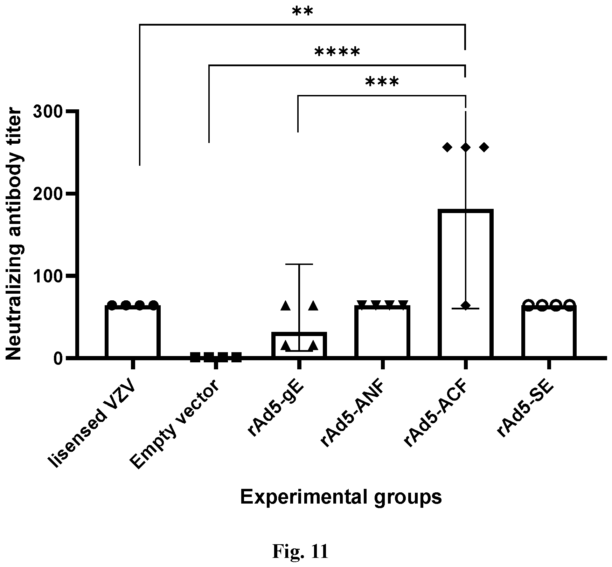

FIG. 11 shows the analysis of antibody-mediated neutralization for VZV infection in serum of mice immunized with recombinant adenovirus. Each recombinant adenovirus (10⁹ TCID₅₀/dose) or a commercially available chickenpox vaccine (700 pfu/dose) was used to immunize C57BL/6 mice, by intramuscular injection, for a total of two doses with an interval of 30 days. 30 days after the second dose of immunization, mouse serum was collected and tested for VZV-specific neutralizing antibody titers. The mean value of detection results of duplicate wells was taken to represent a neutralizing antibody titer. The dilution factor that reduced the number of plaques by 50% was calculated and the reciprocal of the dilution factor was taken to represent the neutralizing antibody titer. ** p < 0.01, *** p < 0.001 (ANOVA/Bonferroni one-way analysis of variance).

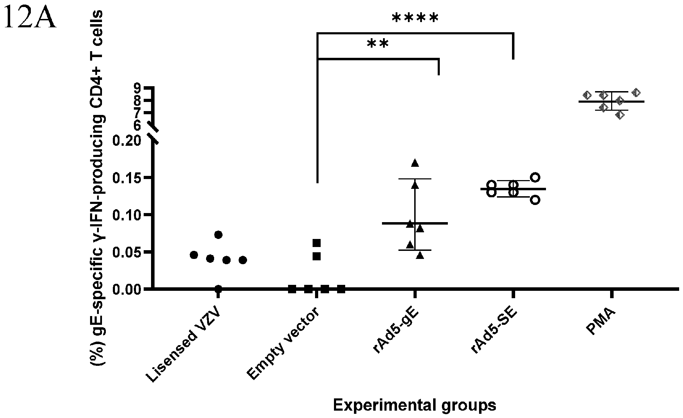

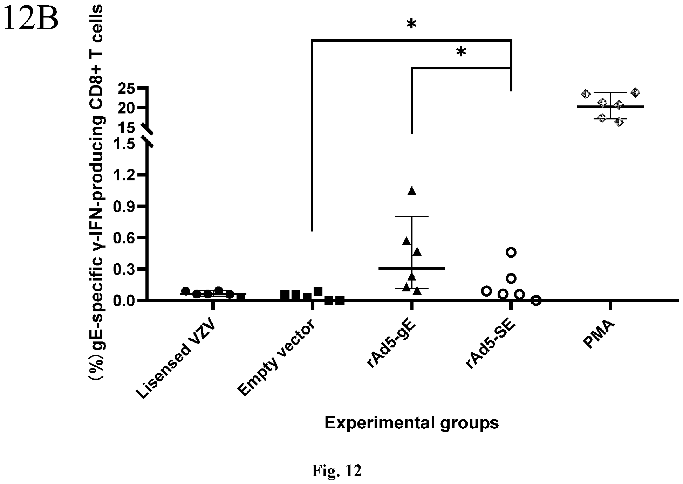

FIG. 12 shows the flow cytometry analysis of gE-specific CD4+ and CD8+ T cell responses induced by recombinant adenoviruses. Splenocytes were collected 36 days after the second dose of immunization and then stimulated with a mixture of 15 overlapping polypeptides (2 µg/peptide) covering the entire gE extracellular region. According to CD3+/CD4+ and CD3+/CD8+ T cell double-positive gates, fluorescent-labeled anti-IFN-y antibody was used for intracellular factor staining (ICS) flow cytometry analysis. The results are expressed as the percentage of IFN-γ expressing CD4+ and CD8+ T cells, with 95% upper and lower confidence interval. **p < 0.01,****p < 0.0001 (ANOVA/Bonferroni one-way analysis of variance). The negative control was unstimulated splenocytes and the positive control was PMA (50 ng).

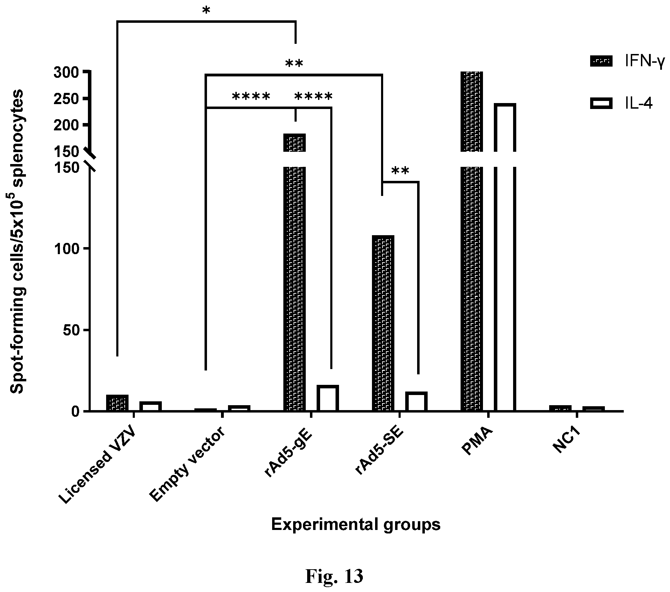

FIG. 13 shows Elispot analysis of IFN-γ- and IL-4-producing T cells induced by recombinant adenoviruses. Splenocytes were collected 36 days after the second dose of immunization, and then stimulated with a mixture of 15 overlapping polypeptides (2 µg/peptide) covering the entire gE extracellular region and analyzed for the number of IFN-γ- and IL-4-producing T cells. Results are expressed as the average number of spots/5×10⁵. * p < 0.05, ** p < 0.01, **** p < 0.0001 (ANOVA/Bonferroni one-way analysis of variance). The negative control was unstimulated splenocytes from the empty vector adenovirus group, and the positive control was PMA (50 ng).

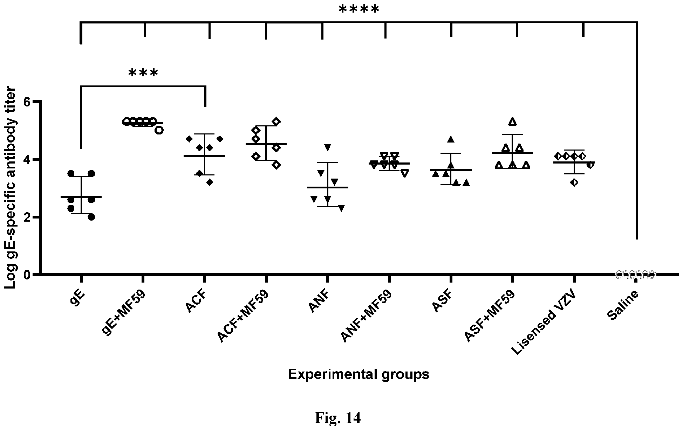

FIG. 14 shows the gE-specific antibody titers induced by gE protein and gE-flagellin fusion protein. C57BL/6 mice were immunized with gE protein (5 µg/dose) or gE-flagellin fusion protein (8 µg/dose) with or without MF59 adjuvant (50 µL/dose) for a total of two doses together with an interval of 14 days. 14 days after the second dose of immunization, immune serum was collected and the gE-specific antibody titers were detected by ELISA. *** p < 0.001, **** p < 0.0001 (ANOVA/Bonferroni one-way analysis of variance).

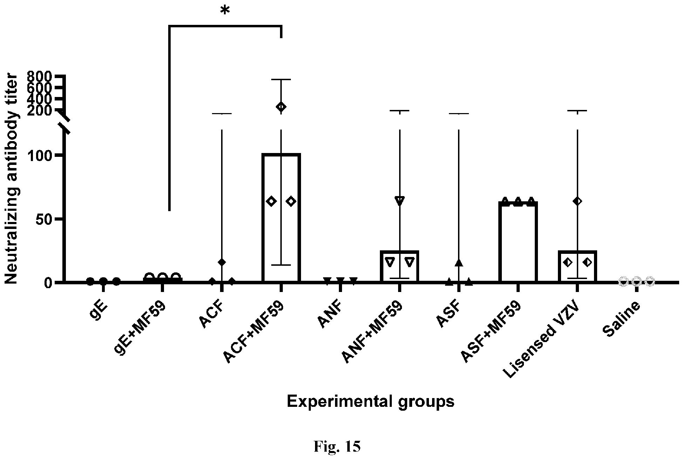

FIG. 15 shows antibody-mediated neutralizing titers for VZV infection in serum immunized with gE protein or gE-flagellin fusion protein (with or without MF59 adjuvant). C57BL/6 mice were immunized with gE protein (5 µg/dose) or gE-flagellin fusion protein (8 µg/dose) (with or without MF59 adjuvant, 50 µL/dose) or a commercially available chickenpox vaccine for a total of two doses with an interval of 14 days. 14 days after the second dose of immunization, immune serum was collected, and the mice in each group were grouped in pairs for serum combination so as to detect VZV-specific neutralizing antibody titers. The mean value of detection results of duplicate wells was taken to represent a neutralizing antibody titer. The dilution factor that reduced the number of plaques by 50% was calculated and the reciprocal of the dilution factor was taken to represent the neutralizing antibody titer. * p < 0.05 (ANOVA/Bonferroni one-way analysis of variance).

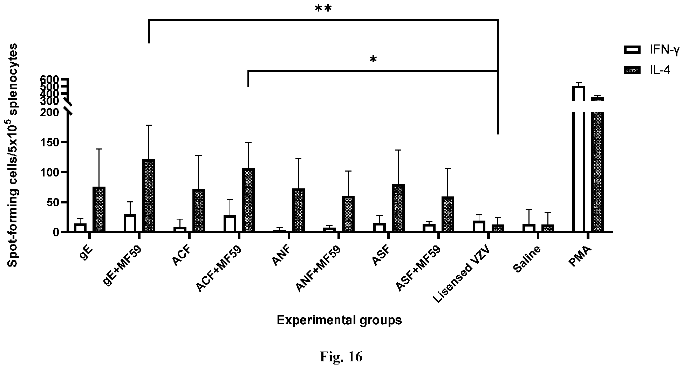

FIG. 16 shows Elispot analysis of IFN-γ- and IL-4-producing T cells induced by gE protein and gE-flagellin fusion protein (with or without MF59 adjuvant). Splenocytes were stimulated with a mixture of 15 overlapping polypeptides (2 µg/peptide) covering the entire gE extracellular region and analyzed for the number of IFN-γ- and IL-4-producing T cells. Results are expressed as the average number of spots/5×10⁵. * p < 0.05, ** p < 0.01 (ANOVA/Bonferroni one-way analysis of variance). The negative control was saline-immunized splenocytes and the positive control was PMA (50 ng).

### Emobodiments

### Materials and methods:

### Animals and cells:

Special pathogen-free (SPF grade) female C57BL/6 mice aged 6-8 weeks were purchased from Hubei Provincial Center for Disease Control and Prevention. All animal studies were performed under GLP (Good Laboratory) laboratory conditions, and animals were treated according to "Laboratory Animal-Guideline for Ethical Review of Animal Welfare". Human embryonic kidney cells HEK293 were purchased from Thermo Fisher Scientific, USA, and cultured in DMEM containing 10% fetal bovine serum (FBS). THP-1 cells were purchased from ATCC and cultured in RPMI-1640 medium containing 10% FBS and 1% penicillin/streptomycin double antibody (Gibco, USA).

### Reagents:

All gene fragments were synthesized by Sangon Biotech (Shanghai, China), and primers were synthesized by Wuhan TsingKe Biological Technology (Wuhan, China); pDONR221, pAd5-CMV/V5-Dest vector, Gateway BP recombination, LR recombinase, *E. coli* TOP10 competent cells and lip2000 transfection reagents were all purchased from Thermo Fisher Scientific (USA). The pET28a expression plasmid was purchased from Novagen (USA). Plasmid extraction kit and gel extraction kit were purchased from Axygen (USA). Mouse anti-VZV-gE monoclonal antibody was purchased from Merck (USA), and rabbit anti-flagellin D0-D1 antibody was prepared by immunization of rabbits. Three synthetic polypeptides from flagellin D0 and D1 domains (see Table 1.) were conjugated to a carrier protein (CCH, Thermo Fisher scientific, USA). Immune process: rabbits were immunized according to the following regimen: the first dose, 0.4 mg of the conjugate containing complete Freund's adjuvant, intramuscular injection; the second dose and the third dose, 0.2 mg of the conjugate containing incomplete Freund's adjuvant, intramuscular injection; finally, 0.1 mg of the conjugate, intravenous pulse. Rabbit anti-Ad5 monoclonal antibody was purchased from Abcam Inc. (UK). Cell culture flasks and pipettes were purchased from Corning Inc. (USA). Endotoxin-free flagellin protein was purchased from Alpha Diagnostic (USA); IL-8 and TNF-α ELISA kits and Elispot kits were purchased from Dakewe; guinea pig complement serum for neutralizing antibody detection was purchased from BD (USA). All antibodies used in the flow cytometry were purchased from Thermo Fisher. The commercially available live attenuated varicella vaccine was produced by Changchun Keygene (China) or Changchun Bcht (China).

**Table 1. Polypeptide sequences of the flagellin protein D0-D1**

| Serial number | Polypeptide sequence |
|---|---|
| 1 | LNKSQSALGTAIERLSSGLRINSAKDDAAC |
| 2 | NNLQRVRELAVQSANSTNC |
| 3 | LTSARSRIEDSDYATEVSNM |

### PCR and agarose electrophoresis:

25 µL of 2× PCR premixed solution and 50-100 ng of a DNA template were added into a tube containing 1 µL of upstream and downstream primers, and then ddH₂O was supplemented to 50 µL, wherein the conditions for cycling were as follows: first step, 95 °C for 2 min; second step: 95 °C for 15 s, 45-55 °C for 15s, and 72 °C for 1 min and 30 s, 30 cycles in total; and third step: 72 °C for 5 min. After completion of PCR, the PCR product was added into a sample buffer solution, and 1% agarose gel electrophoresis was performed under the electrophoresis condition of 180 V for 20-30 min, and the PCR results were detected by ultraviolet.

### SDS-PAGE and Western Blotting:

20 µL of 5x concentrated loading buffer was added into 80 µL of sample, and the mixture was boiled for 5 min. The boiled sample was subjected to 10% SDS-PAGE (100 V, 20 min, and then 160 V, 1 h and 20 min). After the completion of electrophoresis, the proteins were wet-transferred onto PVDF membrane (Merck, USA) and blocked overnight at 4 °C with PBST solution containing 5% skimmed milk powder (PBS solution containing 0.05% Tween 20); the membrane was washed twice with PBST, a mouse anti-VZV-gE protein monoclonal antibody (1:5000 dilution, Millipore) or rabbit anti-flagellin antiserum (1:10000 dilution) or rabbit anti-Ad5 polyclonal antibody (1:10000 dilution) was added, and then the mixture was incubated at 37 °C for 1 h; the membrane was washed twice with PBST, horse radish peroxidase (HRP) labeled goat anti-mouse IgG (1:5000 dilution, Beyotime) or HRP labeled goat anti-rabbit IgG (1:5000 dilution, Beyotime) was added, and then the mixture was incubated at 37 °C for 1 h; the membrane was washed twice with PBST, and Western Blotting ECL solution was used for color development by chemiluminescence.

### Adenovirus titer-TCID₅₀ method:

A flask of 293 cells at 90% confluence that were grown in DMEM medium with 10% FBS was taken (T-75 flask). One day before the assay, the cells were washed with PBS, digested with 1× TypLE for 2 min, then the digestion was terminated with DMEM medium containing 2% FBS, and cells were counted after resuspension in the same medium. The cells were adjusted to a concentration of 1.0-2.0×10⁵ cells/mL, and seeded into a 96-well plate at 100 µL per well; then the 96-well plate was incubated in an incubator at 37 °C, 5% CO₂ for 16-20 h; the virus solutions to be tested and the reference substance were subjected to serial ten-fold dilution (from 10-1 to 10-10) with DMEM+2% FBS medium, respectively. The diluted virus solutions were added into 1-10 columns respectively at 100 µL per well, and 8 replicate wells were set for each dilution degree of virus. 100 µL of DMEM+2% FBS medium was added into columns 11 and 12 as a negative control. The 96-well plate was incubated in a CO₂ incubator at 37 °C for 10 days and then observed under an inverted microscope to determine and record the cytopathic effect (CPE) status of cells in each column. The result was determined as positive as long as a small number of cells developed CPE. Finally, the virus titer was calculated according to the Karber method. (Kärber G., Archiv f experiment Pathol u Pharmakol, 162: 480-483, 1931).

### Detection of TLR-5 activity:

THP-1 cells expressing TLR-5 receptor in logarithmic growth phase and grown in RPM-1640 medium containing 10% FBS were taken and centrifuged at 125 g for 5 min, and the supernatant was discarded. The cells were resuspended in RPMI-1640 medium containing 10% FBS, adjusted to a concentration of 1×10⁷cells/mL, and then seeded into a 96-well cell culture plate at 100 µL/well. The positive control was diluted to a final concentration of 2.5 µg/mL (flagellin protein without endotoxin) with RPMI-1640 medium solution containing 10% FBS. The purified gE-flagellin fusion protein with an endotoxin content < 5EU/mL was diluted to equimolar concentration (5 µg/mL) with the same medium, and the purified gE protein served as a negative control. The diluted samples, endotoxin-free flagellin and gE were added to a 96-well plate at 100 µL per well, respectively. The 96-well plate was incubated in a CO₂ incubator at 37 °C for 12-24 h. After the incubation was completed, the cells in each well were pipetted out and centrifuged at 2000 g for 10 min, and the cell supernatant was collected. The activity of TLR-5 was detected by detecting the contents of IL-8 and TNF-α cytokines in cultural supernatant, and the contents were detected according to the manual in an IL-8 and TNF-α Elisa kit.

### Detection of anti-gE antibody titer in serum bv enzyme-linked immunosorbent assay (ELISA):

The purified prokaryotically expressed gE protein was diluted to 1 µg/mL with sterile sodium carbonate buffer (8.4 g/L NaHCO₃, 3.5 g/L Na₂CO₃, pH 9.6), added to a 96-well microplate at 100 µL per well and then coated overnight at 4 °C. The next day, the microplate was taken out, the liquid in each well was discarded, and the plate was washed 3 times with PBST (PBS solution containing 0.1% Tween 20). Blocking solution (PBST solution containing 10% skimmed milk powder) was added to each well, followed by blocking at 37 °C for 1 h. After the blocking was completed, the blocking solution was discarded. The serum of the immunized mouse was subjected to serial gradient dilution with the blocking solution, and the sealing solution was set as the blank control. The diluted serum was added to the 96-well plate at 100 µL per well, three replicate wells were set for each dilution degree of serum, and then the plate was incubated at 37 °C for 1 h. After the plate was washed three times with PBST, a 1:1000 diluted peroxidase (HRP) labeled goat anti-mouse IgG antibody was added at 100 µL per well, and then the plate was incubated at 37 °C for 1 h. After the plate was washed 3 times with PBST, TMB substrate (3,3',5,5'-tetramethylbenzidine, KPL, USA) was added. The reaction was terminated by adding 0.2 M sulfuric acid. The absorbance was measured at a wavelength of 450 nm and a reference wavelength of 620 nm using a microplate reader.

### Detection of neutralizing antibody:

The determination procedure of antibody-mediated neutralizing titers for VZV infection was as follows: VZV was diluted to 2×10³ PFU/mL with VZV diluent (phosphate buffered saline (PBS), sucrose 5%, glutamic acid 1%, fetal bovine serum (FBS) 10%, pH 7.1). 150 µL of virus was incubated with 150 µL serially diluted heat inactivated serum and 5 µL of guinea pig complement at 37 °C for 1 h. The incubated virus-serum mixture was added to a 24-well plate (100 µL/well) full of MRC-5 monolayer cells, two duplicate wells were set for each dilution degree of serum, and then the plate was incubated at 37 °C for 2 h. After 2 h, 2 mL of virus maintenance solution (MEM containing 2% FBS) was added. After 7 days, the medium was removed, the cells were fixed and stained with Coomassie blue solution (Coomassie blue 0.5%, methanol 45%, acetic acid 10%) for 10 min, and then the plate was washed with distilled water and the spots were counted. Two duplicate wells were tested for each dilution degree of serum. The reciprocal of the dilution degree of serum that reduced the number of plaques by 50% was taken as the neutralizing antibody titer.

### Isolation of mouse solenocytes:

The mouse spleen was aseptically taken out and transferred to a cell strainer placed in a single well of a 6-well plate, and then 3 mL of medium (RPMI-1640 containing 5% FBS) was added. The splenocytes were released by grinding the spleen, and then filtered through a 200 mesh cell strainer. The cells were collected, placed in a 15 mL tube and centrifuged at 350x g for 5 min at 4 °C. The supernatant was discarded, and the deposited cells were resuspended and then lysed for 10 min at room temperature by adding 2 mL of red blood cell (RBC) lysis buffer (Thermo Fisher Scientific). 6 mL of RPMI-1640 medium was then added to terminate the lysis of red blood cells, and the mixture was centrifuged (4 °C, 350 g, 5 min). The supernatant was discarded, 10 mL of RPMI-1640 medium was added to resuspend the cells, and the mixture was centrifuged (4 °C, 350 g, 5 min). The supernatant was discarded and 5 mL of RPMI-1640+10% FBS was added to resuspend the cells. The resuspended spleen cell suspension was subjected to cell counting and then preserved for later use.

### Elispot detection:

gE-specific cellular immunity was detected by Elispot detection of interferon-y (IFN-γ) and IL-4, and a mixture of 15 overlapping polypeptides covering the entire gE extracellular region was used as a stimulant. An Elispot plate (Dakewe) pre-coated with IFN-γ or IL-4 antibody was added with RPMI-1640 medium at 200 µL per well and then left to stand at room temperature for 10 min before removing the medium. The splenocytes were adjusted to a concentration of 2-8×10⁶ cells/mL. 100 µL of the splenocyte suspension was mixed with the polypeptides (concentration of each peptide was 2 µg/mL) and three replicate wells were set for each sample. The Elispot plate was incubated in an incubator at 37 °C for 36-72 h. After the incubation was completed, the color development of spots was performed according to the manual for Elispot plate (see the manufacturer's manual for the specific procedures). After the plate was air dried, the spots were counted by using an enzyme-linked spot imaging system. The number of spot-forming cells (SFC) per 5×10⁵ cells was calculated. The background level of the medium was typically < 15 SFC/5×10⁵ cells.

### Intracellular cytokine staining:

Splenocytes were stimulated *in vitro* with a mixture of polypeptides (2 µg/mL) covering the entire gE extracellular region (15 peptides containing 11 overlapping amino acids) at 37 °C for 2 h, and then Brefeldin A (3 µg/mL) and ionomycin (1 µg/mL) were added, followed by incubation overnight at 37 °C. The cells from each well were harvested, placed in an EP tube, centrifuged at 350 g for 5 min, and the supernatant was discarded. The cells were resuspended with 50 µL of PBS solution containing 2% Fc antibody and 1% FBS, and then incubated at 4 °C for 10 min. 50 µL of an antibody mixture containing anti-CD3 Alex fluor 700, anti-CD4-FITC and anti-CD8-PE-Cy7 (BD Biosciences, 1: 100 dilution) was then added, and the resulting mixture was incubated at 4 °C for 30 min in the absence of light. After the cells were washed once with FACS washing buffer, 200 µL of stationary liquid was added, followed by incubation for 25 min at room temperature in the absence of light. After the completion of fixation, 1.5 mL of the diluted permeabilization reagent was added to wash the cells. The cell suspension was centrifuged at 350 g for 5 min, and then the supernatant was discarded. IFN-γ-APC, IL-2-PerCp-Cy5.5 and IL-4-PE antibodies were diluted with the permeabilization reagent, and the diluted antibody mixture was then added to the cell suspension. The resulting mixture was incubated for 30 min at room temperature in the absence of light. CD3+/CD4+ positive T cells and CD3+/CD8+ positive T cells subsets were analyzed with CytoFLEX S flow cytometer (Beckman) and Flow Jo software.

### Example 1

### Construction, Identification, Amplification and Purification of Recombinant Adenovirus

### 1.1 Design of experiment

1.1.1 A linker sequence for gE-flagellin fusion protein was designed according to calculation and simulation by a computer. A computer-simulated diagram of the binding of the flagellin protein and TLR-5 receptor is shown in FIG. 1, and a simulated structural diagram of the binding of the designed gE-flagellin fusion protein and TLR-5 receptor is shown in FIG. 2, wherein FIG. 2A is ANF, FIG. 2B is ACF, and FIG. 2C is ASF.
1.1.2 Primers used in this study are shown in Table 2, and inserted genes and abbreviations of recombinant adenoviruses prepared accordingly are shown in FIG. 3.

**Table 2. Primers used in this study**

| Name of primer | Sequence |
|---|---|
| AttB1-JEV-F | GGGGACAAGTTTGTACAAAAAAGCAGGCTTCGCCGCCGCCATGGGAAA ACGGTCC |
| AttB2-SV40-R | GGGGACCACTTTGTACAAGAAAGCTGGGTCAGACATGATAAGATACAT TGATGAG |
| AttB2-GE-R | GGGGACCACTTTGTACAAGAAAGCTGGGTCTTATTATTATCTGATCAG GGGGCTAG |
| AttB2-hOACF-R | GGGGACCACTTTGTACAAGAAAGCTGGGTCTTATTATTACCTCAGCAG GCTCAG |
| AttB2-hOANF-R | GGGGACCACTTTGTACAAGAAAGCTGGGTCTTATTATTATCTAATCAG AGGGCTAG |

| | |
|---|---|
| Note: 1. in the name of primer, F represents a forward primer, and R represents a reverse primer; 2. forward primers used for amplifying gE and gE-flagellin genes with and without SV40 polyA are the same, namely AttBl-JEV-F; 3. reverse primers used for amplifying gE and gE-flagellin genes containing SV40 polyA are all AttB2-SV40-R. | |

### 1.2 Construction of recombinant adenovirus

### 1.2.1 Construction of pDONR221 transfer vector

The gene fragments as shown in Section 1.1 were subjected to gene synthesis, and then each target gene fragment was amplified by high fidelity DNA polymerase (the sequence of the amplification primer is shown in Table 2 in Section 1.1.2). After PCR amplification, PCR products were detected by 1% agarose gel electrophoresis and target DNA fragments were extracted by using a DNA gel extraction kit, wherein the conditions for PCR cycling were as follows: first step, 95 °C for 2 min; second step, 95 °C for 15 s, 55 °C for 15 s, and 72 °C for 1 min and 30 s, 30 cycles in total; and third step, 72 °C for 5 min. The extracted target DNA fragments were subjected to BP-recombination with pDONR221 plasmid (Thermo Fisher Scientific, Cat 11789020) according to the manufacturer's manual, respectively, and the recombination mixture was transformed into *E. coli* TOP10 competent cells, and the later were then coated on a solid Kana-resistant LB plate. The plasmids were extracted and sent for sequencing.

The prepared TOP10/pDONR221-Js-ASF-SV40plyA, TOP10/pDONR221-Js-ACF-SV40plyA and TOP10/pDONR221-Js-ANF-SV40plyA were deposited at China Center for Type Culture Collection (CCTCC) on September 10, 2019, and the deposit numbers are respectively as follows: CCTCC M 2019707, CCTCC M 2019708 and CCTCC M 2019709.

### 1.2.2 Construction of recombinant adenovirus expression vectors

The correctly sequenced recombinant pDONR221 plasmids were subjected to LR-recombination with the target plasmid pAdS-CMVNS-DEST (Thermo Fisher Scientific, Cat 11791020) according to the manufacturer's manual, respectively. The recombination mixture was transformed into *E. coli* TOP10 competent cells, and the later were then coated on a solid Ampicillin-resistant (Amp, 100 µg/mL) LB plate. The next day, different colonies were selected, which might contain different pAdS-CMV plasmids, and these colonies were made to carry a pAd5-CMV plasmid of the gE or gE-flagellin fusion gene with or without SV40 polyA (referred to as pAdS-CMV (VZV)). The selected colonies were cultured in Amp-resistant LB medium. Plasmids were extracted and sequenced.

### 1.2.3 Preparation of recombinant adenovirus plasmid

The correctly sequenced pAdS-CMV (VZV) plasmids were transformed into *E. coli* TOP10 competent cells, respectively, and the later were then coated on a solid Amp-resistant LB plate. The next day, single clones were selected and seeded into 200 mL of LB liquid medium containing Amp, and after overnight culture, a large number of pAd5-CMV (VZV) plasmids were extracted by using plasmid maxi kits.

The prepared TOP10/pAd5-Js-gE-SV40plyA was deposited at the China Center for Type Culture Collection (CCTCC) on September 10, 2019, and the deposit number was CCTCC M 2019710.

### 1.2.4 Linearization of recombinant adenovirus vectors

The plasmids obtained in 1.2.3 were digested with a PacI restriction enzyme (NEB, USA) for 3 h at 37 °C, respectively, and the digestion system was as follows: pAdS-CMV (VZV) plasmid: 10 µg; 10*NEB CutSmart buffer: 5 µL; PacI enzyme: 5 µL, with ddH₂O added to a final volume of 50 µL. After the enzyme digestion, DNA fragments were extracted by using PCR product extraction kits. The extracted DNA fragments were quantified with a micro nucleic acid quantitative analyzer.

### 1.2.5 Packaging of recombinant adenovirus

The Pac I-linearized pAdS-CMV (VZV) plasmids were respectively transfected into HEK293 cells at 60-70% confluence in a 6-well plate according to the instructions for the Lipofectamine2000 transfection reagent. 2 h before transfection, the medium was changed to an antibiotic-free medium was used and a DNA/liposome complex was added. 5 h after transfection, the medium was changed to a DMEM medium containing 10% FBS and 1% double antibody was used. The cytopathic effect was observed under an inverted microscope every other day, and the cells were collected when 60% of HEK293 cells generate plaques. The cells were subjected to three repeated freeze-thaw cycles between an ultralow temperature of -80 °C and room temperature, and then were centrifuged at 1200 g for 5min. The supernatant was collected to obtain recombinant adenoviruses rAd5-gE (Js), rAd5-gE-SV40 (Js), rAd5-ANF (Js), rAd5-ANF-SV40 (Js), rAd5-ACF (Js), rAd5-ACF-SV40 (Js), rAd5-ASF (Js) and rAd5-SE-SV40 (Ig κ), which were stored in a refrigerator at -80 °C.

### 1.3 Identification of target gene expression of recombinant adenovirus

### 1.3.1 PCR identification

Viral genomic DNA was extracted from the initial virus amplification preservation solution by using viral RNA/DNA extraction kit (Takara, Japan) according to the manual, and the extracted viral genomic DNA was subjected to PCR amplification to identify the VZV gE or gE-flagellin fusion gene that was inserted into the recombinant adenovirus vector. Primer: T7-FN5-C-R; PCR conditions: viral DNA 1 µL; forward and reverse primers: each 0.5 µL; 5 µL of 2x PrimerSTAR mix; ddH₂O: 3 µL; and conditions for cycling: first step, 95 °C for 2 min; second step, 95 °C for 15 s, 45 °C for 15 s, and 72 °C for 1 min and 30 s, 30 cycles in total; and third step, 72 °C for 5 min. After the PCR amplification was completed, the PCR products were subjected to 1% agarose gel electrophoresis, and then the gel was cut to extract target bands for sequencing by a sequencing company.

### 1.3.2 VZV gE and gE-flagellin fusion gene expression of recombinant adenovirus

HEK293 cells or Vero cells were seeded into a 6-well plate (5×10⁵/well). When the confluence in the 6-well plate was 90%, P3-generation recombinant adenoviruses were seeded into the 6-well plate at MOI 0.2 (HEK293 cells) and 20 (Vero cells), and normal cells were set as the negative control. After being cultured at 37 °C for 48 h, the cells were scraped off with a cell scraper, and then centrifuged; and the cell precipitate and supernatant were collected separately. The supernatant was labeled as cell culture supernatant. The cell precipitate was added with 100 µL of mammalian cell lysate (Beyotime, China), lysed on ice and then centrifuged at 3500 g for 5 min, and the lysed supernatant was labeled as cell lysate. 20 µL of 5x loading buffer was added to 80 µL of cell culture supernatant and cell lysate separately, and the mixtures were boiled at 100 °C for 5 min. The expression of gE protein or gE-flagellin fusion protein was detected by SDS-PAGE and WB. The results of Vero cell detection are shown in FIG. 4, and the results of HEK293 cell detection are shown in FIG. 5. As can be seen from FIGs. 4 and 5, the expression of the gE protein or the gE-flagellin fusion protein was successfully detected in the supernatants of HEK293 cells and Vero cells after adenovirus A or B infection, and the molecular weight of the expressed gE protein was about 80 Kd and the molecular weight of the expressed gE-flagellin fusion protein was about 120 Kd. The gE protein and the gE-flagellin fusion protein could be specifically recognized by a mouse anti-VZV gE monoclonal antibody, and the gE-flagellin fusion protein could be specifically recognized by an anti-flagellin polyclonal antibody.

### 1.4 Small-scale amplification of recombinant adenovirus:

HEK293 cells at 90% confluence were inoculated with different recombinant adenoviruses at MOI 0.01-1, and then placed in an incubator at 37 °C, 5% CO₂ for continuous culturing. When more than 70% of the cells became round and fell off, the cells were scraped off by a cell scraper and centrifuged at 2265 g for ten minutes. The supernatant and cell precipitate were harvested separately. The cell precipitate was resuspended with PBS, then placed in a freezer at -80 °C and subjected to three repeated freeze-thaw cycles. The cells were then centrifuged at 2265 g for ten minutes to harvest the supernatant for further purification.

### 1.5 Purification of recombinant adenovirus

The centrifuge rotor was pre-cooled to 4 °C. In a biosafety cabinet, 12 mL of 1.4 g/mL cesium chloride (53 g + 87 mL 10 mM Tris-HCl, pH 7.9) was slowly added to the centrifuge tube, and then 9 mL of 1.2 g/mL cesium chloride (26.8 g + 92 mL 10 mM Tris-HCl, pH 7.9) was added quite gently. 13 mL of virus preservation solution was then added to the top of the discontinuous gradient, the tube was equilibrated, and the mixture was centrifuged at 100,000 g (23,000 rpm on SW28 rotor) at 4 °C for 120 min. The viral bands were carefully pipetted, the virus-containing solution was transferred to a 15 mL sterile centrifuge tube, and an equal volume of 10 mM Tris HCl (pH 7.9) was added. 20 mL of 1.35 g/mL cesium chloride was added to the centrifuge tube. 15 mL of the virus suspension diluted in the previous step was added from the top very slowly. After the tube was equilibrated, the mixture was centrifuged at 100,000 g for 18 h at 4 °C. After ultracentrifugation, the blue-white viral bands were carefully collected. The viruses were dialyzed into PBS solution at 4 °C in a 10,000 Dalton cellulose ester membrane (purchased from BD, USA) to remove cesium chloride salt. The dialyzed virus solution was added with 10% glycerol, aliquoted, and cryopreserved in a refrigerator at -80 °C.

### 1.6 Assay and analysis of recombinant adenovirus

The purified recombinant adenoviruses were detected by Western blotting (WB) (see FIG. 6A), and the antibody used in the WB detection was rabbit anti-Ad5 polyclonal antibody. As can be seen from FIG. 6A, each recombinant adenovirus could be specifically recognized by the rabbit anti-rAd5 polyclonal antibody. The purified recombinant virus rAd5-gE was counterstained (1% - 2% phosphotungstic acid solution, pH 6.8), and then was subjected to electron microscope detection. FIG. 6B shows the complete virus particles that can be seen through electron microscope observation, and FIG. 6C shows that the purity of the purified virus is 95% or more according to anion-HPLC analysis. The TCID₅₀ test shows that the titer of the purified adenovirus is 10¹⁰ TCID₅₀/mL or more.

### Example 2

### Expression, Purification and Assay of gE Protein and gE-Flagellin Fusion Protein in Prokaryotic System

### 2.1 Names of genes and proteins

The names of the inserted genes and the correspondingly expressed gE protein and gE-flagellin fusion protein are shown in FIG. 7.

### 2.2 Construction of pET28a expression vector

The genes as shown in Section 2.1 were respectively digested with NcoI and XhoI and then inserted into pET28a vectors digested with the same enzymes. After ligation and transformation, single clones were selected and seeded into kanamycin-resistant (50 µg/mL) LB medium. After culturing overnight, plasmids were extracted for sequencing by a sequencing company. Expression plasmids pET28a-gE, pET28a-ENF, pET28a-ECF and pET28a-ESF were obtained.

### 2.3 Expression of gE protein and gE-flagellin fusion protein

The correctly sequenced plasmids pET28a-gE, pET28a-ENF, pET28a-ECF and pET28a-ESF, were transformed into BL21(DE3) competent cells, and single clones were selected, seeded into kanamycin-resistant LB medium and cultured at 37 °C overnight at 200 rpm. The next day, the strains were transferred to fresh kanamycin-resistant LB medium. After culturing at 37 °C for 4 h at 200 rpm, 0.1-1 mM IPTG was added for inducing expression when OD₆₀₀ reached 0.6-0.8. After inducing at an expression temperature of 16-37 °C for 4-16 h, the thalli were harvested for further purification.

### 2.3 Purification and renaturation of gE protein and gE-flagellin fusion protein

The collected thalli were crushed by a high-pressure homogenizer and centrifuged at 2265 g for 10min. The inclusion bodies were collected, washed 3-4 times with detergent-containing normal saline, and then dissolved in a buffer solution (20 mM Tris+5 mM imidazole+500 mM NaCl+6 M guanidine hydrochloride/8 M urea, pH 8.0). The washed nickel column was equilibrated with 5 column volumes (CV) of equilibration buffer A (20 mM Tris+8M urea+5 mM imidazole+500 mM NaCl, pH 8.0). The dissolved inclusion bodies were loaded onto the nickel column. After the loading, the nickel column was washed with 5 CV of the equilibrium solution and then eluted with 20 CV of eluent buffer B (linear gradient to 100%), wherein the eluent buffer B was 20 mM Tris+8 M urea+500 mM imidazole+500 mM NaCl (pH 8.0). Each elution peak was collected.

Dialysis and renaturation: the purified inclusion bodies (dissolved in 8 M urea) were gradually dialyzed into 6 M, 4 M and 2 M urea-containing PBS solutions using dialysis bags. The dialysate was changed every 2 h. Finally, the purified inclusion body proteins were slowly dialyzed into a PBS solution.

Renaturation on column: after the loading of inclusion bodies, the column was washed with 5 CV of equilibration solution A, and then renaturation on column was performed with 20 CV-40 CV of renaturation solution B (linear gradient to 100%), wherein the renaturation solution B was 20 mM Tris + 2 M urea + 5 mM imidazole + 500 mM NaCl + 0.1 mM GSSG/1 mM GSH (pH 8.0). After renaturation was completed, the column was washed with 5 CV of buffer C (20 mM Tris + 2 M urea + 5 mM imidazole + 500 mM NaCl, pH 8.0). The column was then eluted with 20 CV of eluent buffer D (linear gradient to 100%), which was 20 mM Tris + 2 M urea + 5 mM imidazole + 500 mM NaCl (pH 8.0). Each elution peak was collected. The collected elution peaks were dialyzed into PBS solution using dialysis bags.

### 2.4 Assay of gE protein and gE-flagellin fusion protein

The results of 10% SDS-PAGE electrophoresis and WB analysis of the purified proteins are shown in FIG. 8. According to the detection, the molecular weight of the purified gE protein was about 58 Kd, the molecular weight of the gE-flagellin fusion protein was about 90 Kd, and the purities of other proteins except ECF protein after purification reached 80% or more. Each protein could be specifically recognized by mouse anti-gE monoclonal antibody, and the gE-flagellin fusion protein could be specifically recognized by rabbit anti-flagellin D0-D1 antiserum. According to the detection of protein concentration by bicinchoninic acid assay (BCA), the yield of the gE protein after purification was 15 mg-20 mg/L, and the yield of the gE-flagellin fusion protein was 8-15 mg/L. As the lipopolysaccharide contamination remained after purification (LPS, an adjuvant that interferes with the flagellin activity assay) and part of the protein was degraded, the differences in immunogenicity between immunogens of proteins produced from *E. coli* and those of corresponding proteins expressed by the eukaryotic system (recombinant adenovirus vector) were not compared. However, one of ordinary skill in the art should be able to optimize the yield, prevent or minimize proteolytic hydrolysis and degradation, and significantly reduce residual LPS content. The prokaryotically expressed proteins were not further optimized in the present invention because complete, high-yield and LPS-free recombinant proteins had been obtained herein by using an adenoviral eukaryotic expression system.

### Example 3

### Expression, Purification, Assay and Activity Analysis of gE Protein and gE-Flagellin Fusion Protein in Eukaryotic Cells

### 3.1 Expression of gE protein and gE-flagellin fusion protein in Vero cells

Vero cells at 90% confluence in one flask (T-75 flask) were washed twice with PBS, and then infected separately with the recombinant adenovirus A and recombinant adenovirus B obtained through packaging at MOI 100-200. After adsorption for 1 h at 37 °C, 20 mL of DMEM medium was added into each flask. The culture flasks were placed into a CO₂ incubator and incubated at 37 °C for 4-5 days. The culture supernatant was then harvested for further purification.

### 3.2 Purification of gE protein and gE-flagellin fusion protein

The harvested gE or gE-flagellin fusion protein expression supernatant was added to an equal volume of a mixed solution of 10 mM PBS and 1 M (NH₄)₂SO₄ (pH 7.5). After filtration through a 0.2 µm filter membrane, the resulting mixture was loaded onto a well-equilibrated Capto Phenyl Impress column. The equilibration buffer was 10 mM PBS + 500 mM (NH₄)₂SO₄ (pH 7.5). After the loading, the column was washed with 5 CV of the equilibration buffer, and then eluted with 10 CV of solution B (linear gradient to 100%), wherein solution B was 10 mM PBS (pH 7.5). The elution peak at 100% B was collected.

The collected elution peak was loaded onto a Source 30Q column equilibrated with 10 mM PBS (pH 7.5). After the loading, the column was washed with 5 CV of the equilibration buffer and then subjected to gradient elution with 10 mM PB + 250 mM NaCl (pH 7.5). A purified solution was collected, which was the final purified liquid. The purified liquid (100 µg-5 mg/mL) was added with 10% glycerol and then cryopreserved in a refrigerator at -80 °C.

### 3.3 Assay and activity analysis of gE protein and gE-flagellin fusion protein

According to the SDS-PAGE analysis of the purified gE protein and gE-flagellin fusion protein (see FIG. 9A), the purity of the purified gE protein was 95% or more, and the purity of the purified gE-flagellin fusion protein was 85% or more. According to the detection of protein content after purification using BCA, the yield of the gE protein was up to 100 mg/L, and the yield of the gE-flagellin fusion protein was 50-80 mg/L. The recombinant proteins prepared herein were in a soluble state in an aqueous solution at a concentration ranging from 100 µg-5 mg/mL, such as an aqueous solution of phosphate buffer (pH 7.0-7.5) or 4 mM acetate buffer (pH 5.4). Those skilled in the art are familiar with methods for the stable storage of proteins over a long period of time.

WB analysis of the purified protein revealed that (see FIGs. 9B and 9C) both the gE protein and the gE-flagellin fusion protein could be specifically recognized by mouse anti-gE monoclonal antibody. Only the gE-flagellin fusion protein, rather than the gE protein, could be specifically recognized by rabbit anti-flagellin D0-D1 antiserum.

TLR-5 activity analysis (see Table 3) showed that three fusion proteins ANF, ACF and ASF could, by activating THP-1 TLR-5 receptor, induce THP-1 cells to secrete IL-8 and TNF-α cytokines at higher concentration in a dose-dependent manner. However, the gE protein prepared and purified according to the same method could not induce the secretion of TLR-5-active cytokines. All three gE-flagellin fusion proteins were shown to have the activity of allowing the specific functioning of the flagellin protein through TLR-5. The flagellin activity of the ASF was substantially identical to that of the commercially available flagellin protein.

**Table 3. TLR-5 activity assay**

| Sample name | Batch number | Molecular weight (Kd) | Experimental stimulation concentration (µg/mL) | IL-8 content (ng) | TNF-α content (pg) | *In vitro* relative potency (%) | |
|---|---|---|---|---|---|---|---|
| | | | | | | IL-8 | TNF-α |
| gE | MB20180731 | 80 | 5 | 0 | 0 | 0 | 0 |
| ACF | MB20181220 | 120 | 5 | 6.41 | 111.94 | 32.7 | 11.6 |
| ANF | MB20181218 | 120 | 5 | 10.83 | 552.00 | 55.3 | 57.0 |
| ASF | MB20180916 | 120 | 5 | 27.84 | 1004.00 | 142.0 | 103.6 |
| Flagellin | XA1204-L | 60 | 2.5 | 19.60 | 969.05 | 100 | 100 |

### Example 4 Immunogenicity Test

### 4.1 Immunogenicity detection of recombinant adenovirus A and recombinant adenovirus B

### 4.1.1 Animal immunization and sample collection

All animal experiments were performed according to protocols approved by Hubei Provincial Center for Food and Drug Safety Evaluation and International Animal Care and Use Committee (IACUC). 36 special pathogen-free (SPF grade) female C57BL/6 mice, weighing 12-16 g, were bred in Hubei Provincial Center for Food and Drug Safety Evaluation. After the inspection and quarantine, the mice were randomly divided into 6 groups according to their body weight, and were intramuscularly inoculated with 10⁹ TCFD₅₀/dose of recombinant adenovirus A, recombinant adenovirus B, or 700 pfu commercially available VZV vaccine (Changchun Keygene, China) on day 1 and day 28. Table 4 shows the grouping details. Blood was collected from the orbital venous plexus on day 0, day 12, day 42 and day 56, respectively.

**Table 4. Group information for immunogenicity detection of recombinant adenoviruses**

| Group | Treatment | Dosage (TCID₅₀/dose) | Administration route | Number |
|---|---|---|---|---|
| Empty vector | rAd5 vector | 10⁹ | Intramuscular injection, 0.1 mL/mouse | 6 |
| rAd5-gE | rAd5-gE (Js) | 10⁹ | Intramuscular injection, 0.1 mL/mouse | 6 |
| rAd5-ANF | rAd5-ANF (Js) | 10⁹ | Intramuscular injection, 0.1 mL/mouse | 6 |
| rAd5-ACF | rAd5-ACF (Js) | 10⁹ | Intramuscular injection, 0.1 mL/mouse | 6 |
| rAd5-SE | rAd5-SE(Igκ) | 10⁹ | Intramuscular injection, 0.1 mL/mouse | 6 |
| Commercially available VZV | Live attenuated varicella vaccine (Changchun Bcht) | 700 pfu | Intramuscular injection, 0.15 mL/mouse | 6 |

### 4.1.5 Comparison of immunogenicities of different adenovirus vector vaccines and commercially available chickenpox vaccine for mice

Serum anti-gE IgG antibody: the anti-gE IgG antibody titer in the serum after immunization was detected by ELISA (see the detection results shown in FIG. 10 and Table 5). The antibody titer increase was not detected in mice in the empty vector control group on day 12, day 42 and day 56 after immunization. For other groups, the antibody titer significantly all increased after 12 days of immunization under the dosage of 10⁹, and the antibody titer level further increased after the second booster immunization. On day 12 after immunization, the antibody levels of different recombinant adenovirus groups carrying gE-flagellin fusion protein were significantly higher than the antibody titer levels of the rAd5-gE group and the commercially available chickenpox vaccine group. On day 56 after immunization, the antibody levels of all recombinant adenovirus groups were significantly different (p < 0.001) from that of the commercially available chickenpox vaccine group. Among the adenovirus vector candidate vaccine groups, only the antibody titer of the rAd5-gE group was slightly lower than that of the rAd5-ANF group (P = 0.031), and the antibody titers of the rest of the gE-flagellin fusion adenovirus groups had no significant difference (P > 0.05).

**Table 5. gE-specific IgG antibody titers induced by recombinant adenoviruses**

| Groups | Mouse No. | Geometric Mean Titers (GMT) | | | |
|---|---|---|---|---|---|
| | | Day 0 | Day12 | Day 42 | Day 56 |
| Commercially available VZV | 1 ∼ 6 | < 100 | 898 | 20159 | 7155 |
| Empty vector | 7 ∼ 12 | < 100 | < 100 | < 100 | < 100 |
| rAd5-gE | 13 ∼ 18 | < 100 | 6142 | 32000 | 32000 |
| rAd5-ANF | 19 ∼ 24 | < 100 | 22627 | 73517 | 84449 |
| rAd5-ACF | 25 ∼ 30 | < 100 | 8652 | 45255 | 71838 |
| rAd5-SE | 31 ∼ 36 | < 100 | 5080 | 14154 | 48503 |

Serum neutralizing antibody titer: as shown in FIG. 11, all recombinant adenovirus groups induced high neutralizing antibody level on day 56 after the first dose of immunization at the dosage of 10⁹. The neutralizing antibody level induced by rAd5-ACF group had significant difference (p < 0.001) compared with the rest recombinant adenovirus groups and the commercially available VZV vaccine. The rest recombinant adenovirus groups had no significant difference, but their induced neutralizing antibody levels were comparable to that of the commercially available live attenuated vaccine. The induced neutralizing antibody levels of the rAd5-ANF group and rAd5-SE group had no statistical difference from that of the rAd 5-gE group, but were more consistent and uniform.

Detection of cellular immunity level: according to the results of intracellular cytokine staining shown in FIG. 12, 8 weeks after the C57BL/6 mice were immunized with the recombinant adenoviruses, VZV gE-specific CD4+ T cell immunity could be detected in rAd5-gE group and rAd5-SE group. The percentages of IFN- γ positive cells in CD4+ T cells and CD8+ T cells in these two groups were significantly higher than that of the adenovirus empty vector control group (P < 0.01 or P < 0.0001). As shown in FIG. 13, IFN-γ Elispot assay results (see FIG. 13) further confirmed the results of intracellular cytokine staining. The number of IFN-γ and IL-4 spots in splenocytes of the rAd5-gE group and that of the rAd5-SE group were significantly different from those of the rest experimental groups (P < 0.01 or P < 0.0001). The rAd5-gE group also had significant differences compared with the commercially available vaccine group (P < 0.05). The results showed that the rAd5-gE group and the rAd5-SE group could induce strong CD4+ Th1 and Th2 cellular immune responses, as well as relatively strong CD8+ T cytotoxic cellular immune responses.

### 4.2 Immunogenicity detection of gE-flagellin fusion proteins

### 4.2.1 Animal immunization and sample collection

All animal experiments were performed according to protocols approved by Hubei Provincial Center for Food and Drug Safety Evaluation and International Animal Care and Use Committee (IACUC). 60 special pathogen-free (SPF grade) female C57BL/6 mice, weighing 12-16 g, were bred in Hubei Provincial Center for Food and Drug Safety Evaluation. After the inspection and quarantine, the mice were randomly divided into 10 groups according to their body weight, and were intramuscularly inoculated with gE protein (5 µg/dose) (with or without MF59 (50 µL/dose)), gE-flagellin fusion protein (8 µg/dose) (with or without MF59), or 700 pfu commercially available VZV vaccine (Changchun Keygene, China) on day 1 and day 14, respectively. Table 6 shows the grouping details. Blood was collected from the orbital venous plexus on day 0 and day 28.

**Table 6. Group information of animals for gE-flagellin fusion protein experiment**

| Group | Treatment | Dosage (µg/dose) | Administration route | Number |
|---|---|---|---|---|
| Negative control group | Normal saline | / | Intramuscular injection, 0.1 mL/mouse | 6 |
| gE | gE | 5 | Intramuscular injection, 0.1 mL/mouse | 6 |
| ANF | ANF | 8 | Intramuscular injection, 0.1 mL/mouse | 6 |
| ACF | ACF | 8 | Intramuscular injection, 0.1 mL/mouse | 6 |
| ASF | ASF | 8 | Intramuscular injection, 0.1 mL/mouse | 6 |
| gE+MF59 | gE+MF59 | 5+50µl | Intramuscular injection, 0.1 mL/mouse | 6 |
| ANF+MF59 | ANF+MF59 | 8+50µl | Intramuscular injection, 0.1 mL/mouse | 6 |
| ACF+MF59 | ACF+MF59 | 8+50µl | Intramuscular injection, 0.1 mL/mouse | 6 |
| ASF+MF59 | ASF+MF59 | 8+50µl | Intramuscular injection, 0.1 mL/mouse | 6 |
| Positive vaccine | Commercially available VZV | 700PFU | Intramuscular injection, 0.15 mL/mouse | 6 |

### 4.2.2 Comparison of immunogenicities of gE protein with or without adjuvant, gE-flagellin fusion protein with or without adjuvant, and commercially available chickenpox vaccine

Serum anti-gE IgG antibody titer: the serum of mice was collected on day 28 after immunization, i.e., day 14 after the second dose of immunization, and tested for gE-specific antibody titers by ELISA (see FIG. 14 and Table 7 for the results). The gE-specific antibody titer in the ACF group significantly increased and was statistically different from that in the saline and gE groups. The gE-specific antibody titers of ANF and ASF groups also increased significantly, and were statistically different from that of the saline group and higher than that of the gE group. The results indicated that all the antibody levels induced by self-adjuvanted gE-flagellin fusion proteins without MF59 adjuvant were significantly higher than that induced by the control group (p < 0.0001) 4 weeks after immunization. With the addition of MF59 adjuvant, the antibody titer was further improved, indicating that the immune composition had the effect of enhancing humoral immunity when being combined with the adjuvant, and the combination with other adjuvants capable of inducing cellular immunity can also be considered later.

**Table 7. gE-specific IgG antibody titers induced by gE and gE-flagellin fusion proteins**

| Groups | Mouse No. | Geometric Mean Titers (GMT) | |
|---|---|---|---|
| | | Day 0 | Day 28 |
| gE | 1 ∼ 6 | < 100 | 566 |
| gE + MF59 | 7 ∼ 12 | < 100 | 182456 |
| ACF | 13 ∼ 18 | < 100 | 14368 |
| ACF + MF59 | 19 ∼ 24 | < 100 | 36204 |
| ANF | 25 ∼ 30 | < 100 | 1270 |
| ANF + MF59 | 31 ∼ 36 | < 100 | 7184 |
| ASF | 37 ∼ 42 | < 100 | 4525 |
| ASF + MF59 | 43 ∼48 | < 100 | 18102 |
| Commercially available VZV vaccine | 49 ∼ 54 | < 100 | 8063 |
| Saline | 55 ∼ 60 | < 100 | < 100 |

Serum neutralizing antibody titer: as shown in FIG. 15, on day 14 after the second dose of immunization, the neutralizing antibody titer induced in the ACF+MF59 adjuvant group was significantly higher than that induced in the gE+MF59 adjuvant group. The neutralizing antibody levels induced in other two gE-flagellin fusion protein + MF59 adjuvant groups were not significantly different from the neutralizing antibody level induced in the gE+MF59 adjuvant group, but were higher than the latter. Besides, the neutralizing antibody level induced in the gE-flagellin fusion protein + MF59 adjuvant group is comparable to that induced in the commercially available live attenuated varicella vaccine group. In addition, the neutralizing antibody levels induced in the ASF+MF59 adjuvant group and the ACF+MF59 adjuvant group were more consistent and uniform than that induced in the commercially available vaccine group.

Cellular immunity: the results of IFN-γ and IL-4 Elispot assay are shown in FIG. 16. The number of IL-4 spots in splenocytes of the gE + MF59 adjuvant and ACF + MF59 adjuvant groups increased significantly, having significant difference compared with the commercially available live attenuated varicella vaccine group.

Conclusion: there is still a need to develop a safer modified VZV vaccine. The commercially available live attenuated varicella vaccines would still put the vaccinees, especially infants and immunosuppressed populations, at the risk of rare but very serious adverse reactions. Once these reactions occur, urgent medical treatment is required. In addition, the commercially available live attenuated varicella vaccines also present the risk of infecting immunocompromised individuals. One third of the subjects vaccinated with live attenuated vaccines will be at risk of developing shingles in the future, and one fifth of them will suffer from debilitating chronic postherpetic neuralgia. Therefore, pregnant women and immunocompromised people are prohibited from using live attenuated varicella and herpes zoster vaccines at present. Although Shingrix, a subunit shingles vaccine with adjuvant, is more effective than live attenuated vaccines, it has more adverse reactions and may cause more local and systemic adverse reactions (Tricco AC et al., BMJ, 363:k4029, 2018).

The present invention discloses methods for preparing and implementing novel immune components, which can be used in vaccines for preventing VZV infection and induce extensive protective humoral and cellular immunity. The immune components select VZV-gE glycoprotein as the immunogen because the gE protein is the most abundant and most immunogenic protein in VZV virus. The immune components described herein include recombinant VZV-gE proteins with adjuvant and gE-flagellin fusion proteins having intrinsic adjuvant properties. The immune components can be prepared by the expression in a prokaryotic or eukaryotic expression system, and can also be prepared in a replication-defective adenovirus vector expressing gE or gE-flagellin protein. The moiety of the flagellin protein that is covalently linked to gE protein by genetic engineering has been shown to be able to bind to and activate TLR-5, thus triggering innate immunity. Such fusion proteins may not require further adjuvants in human vaccines, thereby reducing the risk of adverse reactions caused by adjuvants. According to the present invention, all immune components have high immunogenicity and can induce strong gE-specific antibodies and *in vitro* functional neutralizing antibodies related to the protection; meanwhile, the immune components can induce CD4+ Th1 and Th2 T cell immunity, which plays an important role in the prevention and recovery of shingles. Self-adjuvanted gE-flagellin fusion proteins are more immunogenic than the corresponding gE proteins, either directly purified or delivered via adenovirus vectors. If desired, the immunogenicity of the purified protein can be significantly improved by using a conventional adjuvant that is much less reactive than AS01 in Shingrix. The non-replicating adenovirus vector expressing gE or gE-flagellin fusion protein can not only induce good gE-specific antibodies, VZV neutralization responses and CD4+ T cell responses, but also induce the body to generate CD8+ T cell immunity, thereby further destroying cells infected by VZV.

Almost all of the immune components of the present invention are more immunogenic than the commercially available live attenuated varicella vaccine. In addition, the various immune components described herein can be used as part of a prime-boost immunization regimen to enhance and augment VZV-specific immunity. The various immune components can also be mixed with other immunogens for use in combination vaccines. These immune components are safer than the commercially available live attenuated varicella vaccines, because they are not infectious, do not cause occasional serious adverse events that may be associated with use thereof, and most importantly, they do not put the vaccinees at a significant risk of developing shingles and postherpetic neuralgia. The present invention also discloses a method for preparing the gE and gE-flagellin protein fusion protein by expression in a prokaryotic system, and the method can reduce the production cost of the vaccine. The adenovirus vector disclosed herein can also be developed into a vaccine for single immunization, so that the immunization frequency can be reduced.

In conclusion, the immune components provided herein can be used to produce a new vaccine for the prevention and control of chickenpox and shingles, which is safer, effective and possibly cheaper. It should be noted that the above examples are only used for illustrating the technical solutions of the present invention, which should not be construed as limiting the present invention. Further modification and adjustment for the above content disclosed herein made by those skilled in the art should fall within the protection scope of the present invention.

### Sequence listing

**Table 8. Sequence listing**

| Sequence number | Sequence details |
|---|---|
| SEQ ID NO: 1 | Amino acid sequence of gE extracellular region |
| SEQ ID NO: 2 | Gene sequence of gE extracellular region |
| SEQ ID NO: 3 | Amino acid sequence of flagellin protein from strain LT2 |
| SEQ ID NO: 4 | Linker I (SPGISGGGGGILDSMG) |
| SEQ ID NO: 5 | Amino acid sequence of N-terminal region of flagellin protein from strain LT2 |
| SEQ ID NO: 6 | Amino acid sequence of C-terminal region of flagellin protein from strain LT2 |
| SEQ ID NO: 7 | Linker II (GGGGSGGGGSGGGGS) |
| SEQ ID NO: 8 | Amino acid sequence of ANF fusion protein (N-terminal D0-D1-C terminal D1-D0-gE, LT2) |
| SEQ ID NO: 9 | Amino acid sequence of ACF fusion protein (gE-N-terminal DO-Dl-C-terminal D1-D0, LT2) |
| SEQ ID NO: 10 | Amino acid sequence of ASF fusion protein (N-terminal DO-Dl-gE-C-terminal D1-D0, LT2) |
| SEQ ID NO: 11 | Gene encoding ANF fusion protein (N-terminal DO-Dl-C-terminal D1-D0-gE, LT2) |
| SEQ ID NO: 12 | Gene encoding ACF fusion protein (gE-N-terminal DO-Dl-C-terminal D1-D0, LT2) |
| SEQ ID NO: 13 | Gene encoding ASF fusion protein (N-terminal DO-Dl-gE-C-terminal D1-D0, LT2) |
| SEQ ID NO: 14 | JEV prM leader sequence |
| SEQ ID NO: 15 | Mouse IgGκ light chain leader sequence |
| SEQ ID NO: 16 | Kozak sequence |
| SEQ ID NO: 17 | SV40 polyA |
| SEQ ID NO: 18 | Kozak sequence-JEV prM leader sequence-gE extracellular region gene |
| SEQ ID NO: 19 | Kozak sequence-JEV prM leader sequence-gE extracellular region gene-SV40 polyA |
| SEQ ID NO: 20 | Kozak sequence-JEV prM leader sequence-5' terminal D0-D1 gene-linker 1-3' terminal D1-D0 gene-3x(GGGGS)-gE extracellular region gene |
| SEQ ID NO: 21 | Kozak sequence-JEV prM leader sequence-5' terminal D0-D1 gene-linker 1-3' terminal D1-D0 gene-3x(GGGGS)-gE extracellular region gene-SV40 polyA |
| SEQ ID NO: 22 | Kozak sequence-JEV prM leader sequence-gE extracellular region gene-3x(GGGGS)-5' terminal D0-D1 gene-linker 1-3' terminal D1-D0 gene |
| SEQ ID NO: 23 | Kozak sequence-JEV prM leader sequence-gE extracellular region gene-3×(GGGGS)-5' terminal D0-D1 gene-linker 1-3' terminal D1-D0 gene-SV40 polyA |
| SEQ ID NO: 24 | Kozak sequence-JEV prM leader sequence-5' terminal D0-D1 gene-3x(GGGGS)-gE extracellular region gene-3x(GGGGS)-3' terminal D1-D0 gene |
| SEQ ID NO: 25 | Kozak sequence-JEV prM leader sequence-5' terminal D0-D1 gene-3x(GGGGS)-gE extracellular region gene-3x(GGGGS)-3' terminal D1-D0 gene-SV40 polyA |
| SEQ ID NO: 26 | Kozak sequence-mouse IgGκ light chain leader peptide sequence-SE-SV40 polyA (ty2) |
| SEQ ID NO: 27 | Amino acid sequence of modified flagellin protein (N-terminal D0-D1-Linker I-C-terminal D1-D0, LT2) |
| SEQ ID NO: 28 | Gene encoding modified flagellin protein (N-terminal D0-D1-Linker I-C-terminal D1-D0, LT2) |
| SEQ ID NO: 29 | Amino acid sequence of flagellin protein from strain Ty2 |
| SEQ ID NO: 30 | Amino acid sequence of N-terminal region of flagellin protein from strain Ty2 |
| SEQ ID NO: 31 | Amino acid sequence of C-terminal region of flagellin protein from strain Ty2 |
| SEQ ID NO: 32 | Amino acid sequence of ANF fusion protein (N-terminal D0-D1-C terminal D1-D0-gE, Ty2) |
| SEQ ID NO: 33 | Amino acid sequence of ACF fusion protein (gE-N-terminal D0-D1-C-terminal D1-D0, Ty2) |
| SEQ ID NO: 34 | Amino acid sequence of ASF fusion protein (N-terminal DO-Dl-gE-C-terminal D1-D0, Ty2) |
| SEQ ID NO: 35 | Amino acid sequence of *E*. coli-expressed gE extracellular region |
| SEQ ID NO: 36 | Gene sequence of *E*. coli-expressed gE extracellular region |
| SEQ ID NO: 37 | Amino acid sequence of ENF fusion protein (N-terminal D0-D1-C-terminal D1-D0-gE, LT2) |
| SEQ ID NO: 38 | Amino acid sequence of ECF fusion protein (gE-N-terminal D0-D1-C-terminal D1-D0, LT2) |
| SEQ ID NO: 39 | Amino acid sequence of ESF fusion protein (N-terminal DO-Dl-gE-C-terminal D1-D0, LT2) |
| SEQ ID NO: 40 | Gene encoding ENF fusion protein (N-terminal D0-D1-C-terminal D1-D0-gE, LT2) |
| SEQ ID NO: 41 | Gene encoding ECF fusion protein (gE-N-terminal D0-D1-C-terminal D1-D0, LT2) |
| SEQ ID NO: 42 | Gene encoding ESF fusion protein (N-terminal D0-D1-gE-C-terminal D1-D0, LT2) |

## Claims

1. An immune composition comprising at least a varicella zoster virus glycoprotein E (gE)-based antigen.

2. The immune composition according to claim 1, wherein the gE-based antigen comprises at least: (i) a gE extracellular region or a fragment thereof, or a nucleic acid molecule encoding the same; (ii) a gE-based fusion protein, or a nucleic acid molecule encoding the same; (iii) a gE-based recombinant vector; or (iv) a combination of two or more of the above.

3. The immune composition according to claim 2, wherein the gE-based fusion protein comprises: a gE extracellular region or a fragment thereof that is covalently coupled with a bacterial flagellin protein or a fragment thereof, wherein the bacterial flagellin protein or a fragment thereof acts as a TLR-5 agonist.

4. The immune composition according to claim 1, wherein the gE extracellular region has at least 90% homology to the amino acid sequence as set forth in SEQ ID No: 1.

5. The immune composition according to claim 1, wherein the gE-based fusion protein comprises at least: an N-terminal D0-D1 region of the flagellin protein, a C-terminal D0-D1 region of the flagellin protein, and the gE extracellular region or a fragment thereof.

6. The immune composition according to claim 5, wherein the gE extracellular region or a fragment thereof is located at the N-terminal or C-terminal of the fusion protein, or is inserted between the N-terminal and C-terminal of the flagellin protein.

7. The immune composition according to claim 2, wherein the fusion protein is selected from any one of the following fusion forms:
fusion form 1: N-terminal region of the flagellin protein - C-terminal region of the flagellin protein - gE extracellular region or a fragment thereof;
fusion form 2: gE extracellular region or a fragment thereof - N-terminal region of the flagellin protein - C-terminal region of the flagellin protein;
fusion form 3: N-terminal region of the flagellin protein - gE extracellular region or a fragment thereof - C-terminal region of the flagellin protein;
wherein the N-terminal region or the C-terminal region of the flagellin protein is linked to the gE extracellular region or a fragment thereof either directly or via a linker; or
the N-terminal region of the flagellin protein is linked to the C-terminal region of the flagellin protein either directly or via a linker.

8. The immune composition according to claim 7, wherein the linker has 1-20 amino acids linked via peptide bonds.

9. The immune composition according to claim 8, wherein the linker is linker I or linker II, linker I has the sequence as set forth in SEQ ID NO: 4, and linker II has the sequence as set forth in SEQ ID NO: 7.

10. The immune composition according to claim 9, wherein the N-terminal region or C-terminal region of the flagellin protein is linked to the gE extracellular region or a fragment thereof via linker II.

11. The immune composition according to claim 9, wherein the N-terminal region of the flagellin protein is linked to the C-terminal region of the flagellin protein via linker I.

12. The immune composition according to claim 3, wherein the bacterial flagellin protein is from salmonella.

13. The immune composition according to claim 12, wherein the salmonella is *S. typhimurium* or S. *typhi.*

14. The immune composition according to claim 13, wherein the amino acid sequence of the flagellin protein is set forth in SEQ ID No: 3 or SEQ ID No: 29.

15. The immune composition according to claim 13, wherein the N-terminal region of the flagellin protein has an amino acid sequence having at least 95% homology to the amino acid region from 2 to 176 in SEQ ID NO: 3, and the C-terminal region of the flagellin protein has an amino acid sequence having at least 95% homology to the amino acid region from 392 to 495 in SEQ ID NO: 3.

16. The immune composition according to claim 13, wherein the amino acid sequence of the N-terminal region of the flagellin protein is set forth in SEQ ID NO: 5, and the amino acid sequence of the C-terminal region of the flagellin protein is set forth in SEQ ID NO: 6.

17. The immune composition according to claim 13, wherein the N-terminal region of the flagellin protein has an amino acid sequence having at least 95% homology to the amino acid region from 2 to 180 in SEQ ID NO: 29, and the C-terminal region of the flagellin protein has an amino acid sequence having at least 95% homology to the amino acid region from 400 to 506 in SEQ ID NO: 29.

18. The immune composition according to claim 13, wherein the amino acid sequence of the N-terminal region of the flagellin protein is set forth in SEQ ID NO: 30, and the amino acid sequence of the C-terminal region of the flagellin protein is set forth in SEQ ID NO: 31.

19. The immune composition according to claim 2, wherein the amino acid sequence of the gE-based fusion protein is set forth in any one of SEQ ID NOs: 8-10,32-34.

20. The immune composition according to claim 2, wherein the nucleic acid molecule encoding the gE extracellular region or a fragment thereof has the sequence as set forth in any one of SEQ ID NOs: 2, 18 and 19.

21. The immune composition according to claim 2, wherein the nucleic acid molecule encoding the gE-based fusion protein has the sequence as set forth in any one of SEQ ID NOs: 11-13,20-26.

22. The immune composition according to claim 2, wherein the gE-based recombinant vector comprises the nucleic acid molecule according to any one of claims 2-21.

23. The immune composition according to claim 22, wherein the vector is an adenovirus vector, an adenovirus-associated virus vector, a poxvirus vector, a vesicular stomatitis virus vector, a bovine parainfluenza virus vector, a human parainfluenza virus vector, a newcastle disease virus vector, a Sendai virus vector, a measles virus vector, an attenuated RSV vector, a paramyxovirus vector, a type A virus vector (e.g., Venezuelan equine encephalitis virus vector, Semliki Forest virus vector, Sindbis virus vector), a rhabdovirus vector, a rabies virus vector, a picornavirus vector, a lentivirus vector, a herpesvirus vector, or a plant-derived virus for expression in a plant expression system.

24. The immune composition according to claim 23, wherein the adenovirus vector is a human adenovirus vector, a chimpanzee adenovirus vector or a gorilla adenovirus vector.

25. The immune composition according to claim 24, wherein the human adenovirus vector is an adenovirus type-5 vector (Ad5), and the chimpanzee adenovirus vector is ChAd68.

26. The immune composition according to claim 24, wherein the adenovirus vector is a replication-defective adenovirus vector.

27. The immune composition according to claim 26, wherein the El region of the adenovirus vector is deleted or functionally deleted to form a replication-defective vector; or both the El region and the E3 region are deleted or functionally deleted.

28. The immune composition according to claim 27, wherein the E4 region of the chimpanzee adenovirus vector is further replaced by the corresponding E4 region of the human adenovirus type-5 to enhance the function of the vector.

29. The immune composition according to any one of claims 22-28, wherein the gE-based recombinant vector is referred to as recombinant adenovirus vector A when it carries the nucleic acid molecule encoding the gE extracellular region or a fragment thereof.

30. The immune composition according to claim 29, wherein the recombinant adenovirus vector A carries the nucleic acid molecule as set forth in any one of SEQ ID NOs: 2,18 and 19.

31. The immune composition according to claim 29, wherein the backbone plasmid used to construct the recombinant adenovirus vector A is pAd5-CMV/V5-DEST.

32. The immune composition according to claim 29, wherein the shuttle plasmid used to construct the recombinant adenovirus vector A is pDONR221.

33. The immune composition according to claim 29, wherein the host cell line used to construct the recombinant adenovirus vector A includes, but is not limited to, HEK 293 cell line or PER.C6 cell line.

34. The immune composition according to any one of claims 29-33, wherein the recombinant adenovirus vector A is constructed as follows: performing homologous recombination on a correctly sequenced recombinant shuttle plasmid pDONR221-gE gene-PolyA and the virus backbone plasmid pAd5-CMV/V5-DEST, transforming the recombination mixture into *E. coli* TOP10 competent cells, screening a correctly sequenced adenovirus vector pAd5-CMV-gE gene-PolyA, linearizing it, then transfecting HEK 293 or PER.C6 cells with the linearized adenovirus vector pAd5-CMV-gE gene-PolyA for packaging, and thus obtaining the recombinant adenovirus vector A.

35. The immune composition according to any one of claims 22-28, wherein the gE-based recombinant vector is referred to as recombinant adenovirus vector B when it carries the nucleic acid molecule encoding the gE-based fusion protein.

36. The immune composition according to claim 35, wherein the recombinant adenovirus vector B carries the nucleic acid molecule as set forth in any one of SEQ ID NOs: 11-13,20-26.

37. The immune composition according to claim 35, wherein the backbone plasmid used to construct the recombinant adenovirus vector B is pAd5-CMV/V5-DEST.

38. The immune composition according to claim 35, wherein the shuttle plasmid used to construct the recombinant adenovirus vector B is pDONR221.

39. The immune composition according to claim 35, wherein the host cell line used to construct the recombinant adenovirus vector B includes, but is not limited to, HEK 293 cell line or PER.C6 cell line.

40. The immune composition according to any one of claims 35-39, wherein the recombinant adenovirus vector B is constructed as follows: performing homologous recombination on a correctly sequenced recombinant shuttle plasmid pDONR221-gE-flagellin fusion protein gene-PolyA and the virus backbone plasmid pAd5-CMV/V5-DEST, transforming the recombination mixture into *E. coli* TOP10 competent cells, screening a correctly sequenced adenovirus vector pAd5-CMV-gE-flagellin fusion protein gene-PolyA, linearizing it, then transfecting HEK 293 or PER.C6 cells with the linearized adenovirus vector pAd5-CMV-gE-flagellin fusion protein gene-PolyA for packaging, and thus obtaining the recombinant adenovirus vector B.

41. The immune composition according to claim 1, further comprising a pharmaceutically acceptable carrier, and/or an adjuvant, and/or an immunostimulatory molecule.

42. The immune composition according to claim 41, wherein the adjuvant includes, but is not limited to: aluminum salts, oil-in-water or water-in-oil emulsions, MF-59, Quil A or QS21 components thereof, TLR agonists, chitosan, immunostimulatory complexes (ISCOMs), or combinations of two or more of the above.

43. Use of the immune composition according to any one of claims 1-42 in the manufacture of a pharmaceutical composition for preventing and/or treating varicella zoster infection.

44. The use according to claim 43, wherein the immune composition is used to prepare a chickenpox vaccine or a shingles vaccine, or is used to prepare a medicament for treating shingles or postherpetic neuralgia.

45. A combination vaccine comprising at least the immune composition according to any one of claims 1-42 and other vaccines, wherein the other vaccines include, but are not limited to: mumps, measles and rubella vaccines.

46. The gE-based fusion protein as described in the immune composition according to any one of claims 1-21.

47. The nucleic acid molecule as described in the immune composition according to any one of claims 1-21.

48. The gE-based recombinant vector as described in the immune composition according to any one of claims 22-40.

49. An isolated host cell comprising the nucleic acid molecule according to claim 47.

50. A method for preparing the gE extracellular region or a fragment thereof, or the gE-based fusion protein according to claim 46.

51. The method according to claim 50, wherein the preparation is carried out via a prokaryotic expression system or a eukaryotic expression system.

52. The method according to claim 50, wherein the prokaryotic expression is *E. coli* expression, the *E. coli* is BL21(DE3), and the expression vector is pET28a; the amino acid sequence of the gE extracellular region is set forth in SEQ ID NO: 35, and the gene sequence of the gE extracellular region is set forth in SEQ ID NO: 36; the amino acid sequence of the gE-based fusion protein is set forth in SEQ ID NOs: 37-39; and the gene sequence of the gE-based fusion protein is set forth in SEQ ID NOs: 37-39.

53. The method according to claim 50, wherein the gE extracellular region or a fragment thereof is obtained by infecting Vero cells with the recombinant adenovirus vector A according to any one of claims 29-34 and culturing them, and the gE-based fusion protein is obtained by infecting Vero cells with the recombinant adenovirus vector B according to any one of claims 35-40 and culturing them.

54. A prime-boost immunization regimen, comprising: a prime immunization with the gE-based recombinant vector according to claim 48, and a boost immunization with the gE extracellular region or a fragment thereof or the gE-based fusion protein according to claim 46; or conversely, a prime immunization with the gE extracellular region or a fragment thereof or the gE-based fusion protein according to claim 46, and a boost immunization with the gE-based recombinant vector according to claim 48, wherein when the gE extracellular region or a fragment thereof is used for immunization, the adjuvant as described in claims 41-42 may be added.

55. A prime-boost immunization regimen, comprising: a prime immunization with the gE-based recombinant heterologous vector according to claim 23, and a boost immunization with the gE-based recombinant adenovirus vector according to any one of claims 24-40; or conversely, a prime immunization with the gE-based recombinant adenovirus vector according to any one of claims 24-40, and a boost immunization with the gE-based recombinant heterologous vector according to claim 23; wherein, the heterologous vector refers to a non-adenovirus vector.

56. A prime-boost immunization regimen, wherein the two gE-based recombinant adenovirus vectors that are of different types or derived from different species according to any one of claims 24-40 are used as a prime immunization and a boost immunization, respectively, wherein the recombinant adenovirus vector carries either a gE extracellular region gene or a gE-based fusion protein gene.

57. A recombinant adenovirus vector pAd5-CMV-gE gene-PolyA, wherein the gE gene has the nucleic acid sequence as set forth in any one of SEQ ID NOs: 2,18 and 19.

58. A recombinant adenovirus vector pAd5-CMV-gE-flagellin fusion gene-PolyA, wherein the gE-flagellin fusion gene has a nucleic acid sequence as set forth in any one of SEQ ID NOs: 11-13, 20-26.

59. A modified flagellin protein, wherein the N-terminal region of the flagellin protein has an amino acid sequence having at least 95% homology to the amino acid region from 2 to 176 in SEQ ID NO: 3, and the C-terminal region of the flagellin protein has an amino acid sequence having at least 95% homology to the amino acid region from 392 to 495 in SEQ ID NO: 3, wherein the N-terminal region of the flagellin protein is linked to the C-terminal region of the flagellin protein either directly or via a linker.

60. The modified flagellin protein according to claim 59, wherein the linker has 1-20 amino acids linked via peptide bonds.

61. The modified flagellin protein according to claim 60, wherein the linker has an amino acid sequence as set forth in SEQ ID NO: 4.

62. The modified flagellin protein according to claim 59, wherein the amino acid sequence of the N-terminal region is set forth in SEQ ID NO: 5, and the amino acid sequence of the C-terminal region is set forth in SEQ ID NO: 6.

63. The modified flagellin protein according to claim 59, wherein the modified flagellin protein has an amino acid sequence as set forth in SEQ ID NO: 27.

64. Use of the modified flagellin protein according to any one of claims 59-63 as an immune adjuvant.
